# EUROPEAN PATENT SPECIFICATION

(11) **EP 4 319 844 B1**
(45) Date of publication and mention of the grant of the patent: **24.12.2025**
(21) Application number: 22721358.4
(22) Date of filing: 07.04.2022
(51) Int. Cl.: A61M 5/24, A61M 5/315

(54) **DRUG DELIVERY DEVICE**
ARZNEIMITTELABGABEVORRICHTUNG
DISPOSITIF D'ADMINISTRATION DE MÉDICAMENTS

(30) Priority: 07.04.2021 EP 21167293; 25.06.2021 EP 21181883; 25.06.2021 EP 21181887; 08.07.2021 EP 21184545; 10.09.2021 US 202117472546; 10.09.2021 US 202117472550; 10.09.2021 US 202117472554; 10.09.2021 US 202117472561; 13.10.2021 EP 21202384
(43) Date of publication of application: 14.02.2024
(73) Proprietor: medmix Switzerland AG, 9469 Haag (Rheintal) (CH)
(72) Inventor: MÜLLER, Heiko, 70563 Stuttgart (DE); REUBZAET, Wouter, 6444 DE Brunssum (NL); HILD, André, 52064 Aachen (DE)
(74) Representative: Manitz Finsterwald Patent- und Rechtsanwaltspartnerschaft mbB
(86) International application number: PCT/EP2022/059334
(87) International publication number: WO 2022/214625

(56) References cited:
- US-A1- 2015 224 266
- US-A1- 2016 317 751

## Description

The present disclosure relates to drug delivery devices.

Drug delivery devices, such as injection devices, are used in medical applications to deliver a drug, usually a liquid, to a drug delivery site, such as an injection site. For applications in which drug delivery has to be performed, for example, in an emergency situation or multiple times within comparable short time-scales, such as weeks, days or even hours, drug delivery devices have been developed, which can be used by medically non-trained people, such as for example patients, to self-administer the respective drug. Uses of such devices may include, for example, diabetics, hormonal treatment, anticoagulant treatment, administration of adrenaline, or the like. Of the known types of drug delivery devices, which can be actuated manually, semi-automatically or automatically to eject a drug out of a drug compartment, the pen-type device has become very popular such that it is now available both in reusable and disposable designs.

Documents US 2015/224266 A1 and US 2016/317751 A1 describe injection devices.

Disposable drug delivery devices are completely discarded once the drug compartment of the device has been emptied to a degree that no further dose of medicament can be ejected from the device. With single use devices, the device is discarded after a single dose has been ejected, while multi-use devices allow the repeated ejection of several doses from the same medicament container or drug compartment.

With reusable devices, the drug delivery device includes the possibility to reset the delivery device such that the medicament container can be replaced with a new one when the last dose has been delivered from the container. Said emptying of the container may happen after one dose ejection or after several dose ejections.

Resetting may, for example, require to move the piston rod back into the housing of the device such that a new container can be attached to the device.

To define the amount of drug that is delivered, drug delivery devices usually comprise a dose definition mechanism that is actuatable by the user of the device and that defines the settable doses. To allow for a safe and fail-free use of the device, for example to prevent inadvertent over-medication, it thereby has to be ensured that the user is only allowed to set a dose within a limited range of the settable doses. Furthermore, it has to be assured that each set dose precisely amounts to a pre-defined amount of drug that is ejected from the device.

Accordingly, there is a need to provide dose delivery devices that allow for a precise and safe dose setting.

The present disclosure provides drug delivery devices according to the independent claims. Embodiments are given in the dependent claims, the description and the drawings.

In a first aspect, the present disclosure is directed at a drug delivery device having a housing with a longitudinal axis, a dose setting member that is actuatable by a user and rotatable around the longitudinal axis to set a dose to be delivered by the drug delivery device, a piston rod that is configured to be axially advanced in a proximal direction to deliver the set dose, and a dosing member for defining the axial advancement of the piston rod upon delivery of the set dose. The dosing member is thereby axially movable along the longitudinal axis and rotationally movable around the longitudinal axis during dose setting, and the dosing member is rotationally fixed to the dose setting member during dose setting. Furthermore, the dosing member comprises a maximum dose stop, wherein the maximum dose stop is configured to engage with a maximum stop feature to limit movement of the dosing member with respect to the housing when having set a maximum dose. The maximum stop feature is provided at the housing.

Engagement between the maximum dose stop and the maximum stop feature allows to reliably limit a range of the doses definable by the dosing member to at most the maximum dose. By providing the maximum stop feature at the housing, a precise and rigid reference position is established that prevents the upper limit from being exceeded even in cases in which a user exerts considerable forces during dose setting.

Axial advancement of the piston rod upon delivery of a set dose may be directly proportional to the amount of drug that is delivered. Therefore, defining the axial advancement of the piston rod also defines the set dose. For example, with the drug delivery devices according to the present disclosure, the piston rod may act upon a piston sealing a cartridge that contains the drug to be delivered and the axial advancement of the piston rod upon dose delivery may then be proportional to the amount of drug that is delivered.

During dose setting, the dosing member may be axially and/or rotationally movable with respect to the housing of the drug delivery device. Engagement between the maximum dose stop and the maximum stop feature may, for example, limit axial movement of the dosing member with respect to the housing.

The maximum dose stop and the maximum stop feature may engage with each other directly when setting the maximum dose. Alternatively, they also may engage with each other only when the dose setting member has been overturned by a predefined amount from a rotational position corresponding to the maximum dose.

According to an embodiment, the dosing member is rotationally movable with respect to the dose setting member during dose delivery. This may prevent, for example, a manipulation of the dose setting member during dose delivery to translate to the dosing member and thus to interfere with the drug delivery process. For example, the dose setting member may be prevented from rotating with respect to the housing during dose delivery, while the dosing member is configured to rotate with respect to the housing to cause the delivery of the set dose.

The drug delivery device may, for example, comprise a clutch mechanism that is provided between the dose setting member and the dosing member and that non-rotatably couples the dose setting member and the dosing member to each other during dose setting and de-couples said members during dose delivery. A transition of the clutch mechanism from its coupled or closed state to its de-coupled or open state may, for example, be affected by pushing an actuation member of the drug delivery device to initiate the dose delivery process.

According to an embodiment, the dosing member is configured to perform more than one full rotation, for example at least two full rotations, with respect to the housing during dose setting. For example, the dosing member may be configured to perform two full rotations to set a maximum settable dose.

By not limiting the movement of the dosing member to at most one full rotation, a large range of settable doses and/or a set of closely spaced doses may be provided. This allows the adaption of the dose definition mechanism of the drug delivery device to a wide range of applications.

According to an embodiment, the dosing member is threadedly connected to the housing, for example via an outer thread provided on the dosing member and a corresponding inner thread provided on the housing. The threaded connection may then define a ratio between the axial and rotational movement of the dosing member during dose setting and/or dose delivery. By providing both the threaded connection and the maximum stop feature at the housing of the drug delivery device, the movement of the dosing member may be limited in a precise and reliable manner.

According to an embodiment, the dosing member is rotatable with respect to the housing during both dose setting and dose delivery. During dose setting, movement of the dosing member from its initial position to its final position may define the set dose, whereas movement of the dosing member from the final position back to its initial position during dose delivery then effects advancement of the piston rod and ejection of the drug from the device.

According to an embodiment, the maximum dose stop comprises a stopping surface that is configured to axially abut the maximum stop feature of the housing upon setting the maximum dose. The maximum dose stop thus abuts against the maximum stop feature by moving parallel to the longitudinal axis of the device. This allows to precisely limit the axial movement of the dosing member.

According to an embodiment, the stopping surface is orientated perpendicular to the longitudinal axis. In this way, the axial position of the stopping surface does not depend on the angular position around the longitudinal axis. As a consequence, the axial position of the dosing member when hitting the maximum stop feature does not depend on the rotational position of the dosing member.

According to embodiment, the stopping surface is an annular surface surrounding the longitudinal axis. This ensures that the maximum dose stop engages with the maximum stop feature irrespective of the rotational position of the dosing member with respect to the longitudinal axis.

In general, the maximum stop feature may be configured to cover only a limited angular portion around the longitudinal axis in a cross-sectional plane perpendicular to the longitudinal axis. In cases, in which the drug delivery device comprises several maximum stop features, the set of maximum stop features may likewise be configured to cover only a limited angular portion. Configuring the stopping surface as being orientated perpendicular to the longitudinal axis and/or configuring the stopping surface as an annular surface may then prevent the axial position of the dosing member upon engagement with the maximum stop feature from being dependent on its rotational position.

According to an embodiment, the maximum dose stop protrudes from an outer surface of the dosing member. Thus, the position of the dosing member upon engagement with the maximum stop feature may be flexibly designed by choosing a position of the maximum dose stop on the outer surface of the dosing member.

According to an embodiment, the maximum stop feature protrudes from an inner surface of the housing. This allows to stop movement of the dosing member before it reaches the end of the housing. Furthermore, the position of the dosing member upon engagement with the maximum stop feature may be flexibly designed by choosing a position of the maximum stop feature on the inner surface of the housing.

According to an embodiment, the maximum dose stop is spaced apart from a distal end of the dosing member. This allows to provide additional space at the end of the dosing member, for example for engagement with other components of the drug delivery device, such as with components providing a clutch mechanism of the device and/or with components that transfer an axial force upon the dosing member.

According to an embodiment, the maximum stop feature of the housing has a limiting surface that is orientated perpendicular to the longitudinal axis and the maximum dose stop engages with the limiting surface upon setting the maximum dose. The limiting surface may, for example, be orientated perpendicular to the longitudinal axis. The limiting surface, may for example, be configured as a flat surface.

According to an embodiment, the maximum stop feature of the housing is provided on a flexible element that is configured to snap over the maximum dose stop of the dosing member upon assembly of the drug delivery device. This allows for an easy assembly of device.

The flexible element may be configured to snap over the maximum dose stop when the dosing element is moved in a proximal direction with respect to the housing and/or in a direction corresponding to a decrease of the set dose during use.

According to an embodiment, the flexible element rests against a backing element, such as an outer housing that surrounds an inner housing having the flexible element, after assembly of the drug delivery device to prevent disengagement of the maximum stop feature from the maximum dose stop. This ensures reliable stopping of the movement of the dosing member when engaging with the maximum stop feature.

According to an embodiment, the dosing member is configured as a dose indication member that provides a visual indication of the set dose to a user, for example via corresponding optical markers on an outer surface of the dosing member. Limiting the movement of the dose indication member during dose setting by equipping it with the maximum dose stop results in a precise indication of the set dose, for example in a precise indication of the setting the maximum dose.

According to an embodiment, the dose setting member is configured to axially move with respect to the housing together with the dosing member during dose setting. Such axial movement of the dose setting member provides haptic feedback to a user of the device and may therefore assist visually impaired users.

Furthermore, it allows to configure the dose setting member as an actuation member that is configured to be pushed back by the user of the device to cause the drug to be delivered.

According to an embodiment, the dosing member comprises a zero dose stop, wherein the zero dose stop is configured to engage with a zero stop feature to limit movement of the dosing member with respect to the housing upon the dosing member reaching a zero dose position, wherein the zero stop feature is provided at the housing. By defining the zero dose position via engagement between the dosing member and the housing, the zero dose position is provided as a well-defined reference position. This ensures a precise definition of the amount of drug corresponding to the individual dose settings and thus prevents inadvertent under-dosage or overdosage.

According to an embodiment, the zero dose stop engages with the zero stop feature in a contact plane that is angled with respect to a radial plane perpendicular to the longitudinal axis. The contact plane may thereby be orientated perpendicular to the radial plane. Such a contact plane provides a precise zero dose stop mechanism that is configured to receive comparably large forces. When engaging with each other, the zero stop feature and the zero dose stop may move perpendicular to the radial plane and frontally contact each other.

According to an embodiment, the zero dose stop of the dosing member comprises a stop surface that is configured to abut against a corresponding stop surface of the housing. The stop surfaces may be orientated parallel to each other. Additionally or alternatively, they may be configured as planar surfaces. Providing stop surfaces both at the zero dose stop and at the housing allows to efficiently take up forces when the dosing member is reaching the zero dose position.

According to an embodiment, the zero dose stop is provided at a proximal end of the dosing member. Such a zero dose stop is easily engageable by the zero stop feature upon proximal movement of the dosing member towards the zero dose position.

According to an embodiment, the zero stop feature is provided at a proximal end of a housing cavity of the housing. Such a zero stop feature is easily engageable by the zero dose stop upon proximal movement of the dosing member towards the zero dose position.

According to an embodiment, the zero stop feature and the maximum stop feature are provided at the same structural element of the housing, for example at an inner housing. This allows to precisely define the positions of the dosing member, in which it engages with the corresponding stop features. Consequently, also the axial distance traveled by the dosing member between the stop features and therefore the amount of drug corresponding to the individual dose settings is precisely defined.

According to an embodiment, the structural element comprises a dose thread that threadedly engages with the dosing member. The structural element may therefore also define the axial and rotational positions of the dosing member that correspond to the individual dose settings. This further enhances the reliability and repeatability of dose setting and dose delivery.

According to an embodiment, the structural element comprises a drive thread that threadedly engages with a driver of the drug delivery device, wherein the driver is coupled to the piston rod during dose delivery to axially advance the piston rod upon axial movement of the driver. Providing both the stop features and the drive thread at the same structural element, precisely couples the axial distance traveled by the piston rod during dose delivery to the axial position of the dosing member and thus provides high dose accuracy.

The drug delivery device comprises a dose definition mechanism for defining rotational dose positions of the dose setting member with respect to the housing, wherein the dose setting member is connected to the housing via a dose selector member. The dose selector member is thereby rotationally fixed and axially movable with respect to the housing and the dose definition mechanism acts between the dose selector member and the dose setting member. Such a dose selector member allows to define the rotational positions of the dosing member that correspond to the individual settable doses directly at the dosing member itself and the drug delivery device may therefore be constructed in a simple, yet sturdy way. Due to axial movability of the dose selector member, the individual doses may be defined irrespective of the axial position of the dose setting member during dose setting.

The dose selector member may be provided axially between the dosing member and the dose setting member. Furthermore, the dose selector member may be configured to transfer axial forces from an actuation member of the drug delivery device to the dosing member during dose delivery. The actuation member may be configured to be pushed upon by a user of the device to effect dose delivery. In general, an actuation member may, for example, be the dose selector member or a separate member, such as a pushbutton.

According to an embodiment, the dose selector member is axially fixed with respect to the dosing member. This allows to efficiently transfer axial forces from the dose selector member to the dosing member.

According to an embodiment, the dose selector member is connected to the housing via a connection that allows the dose selector member to be mounted to the housing only in rotational orientations that ensure that the dose setting member is set to a dose position upon engagement of the maximum dose stop with the maximum stop feature, wherein, for example, the connection allows a single rotational orientation only. This facilitates assembly of the drug delivery device.

According to an embodiment, the connection comprises a splined connection that allows axial movement and prevents rotational movement of the dose selector member with respect to the housing and the splined connection comprises a set of coding splines, wherein the coding splines have respective dimensions that differ from each other, for example in width and/or height. Such a connection integrates both the coding function and the restriction of relative mobility between the dose selector member and the housing in the same structural elements. This allows for a simple and cost-efficient construction of the drug delivery device.

According to an embodiment, the splined connection comprises a single coding spline that differs from the remaining splines of the connection. Such a connection is simple in construction, but nevertheless allows a reliable coding function.

According to an embodiment, the dosing member is coupled to the piston rod via an advancement mechanism that translates axial movement of the dosing member into the axial advancement of the piston rod during dose delivery so that the axial movement of the dosing member during dose delivery causes the piston rod to axially advance in the proximal direction. Such an advancement mechanism may, for example, comprise one or several additional members of the drug delivery device.

With the drug delivery devices according to the present disclosure, the advancement mechanism may, for example, comprise a driver and a nut, whereby the driver is coupled between the dosing member and the nut, whereas the nut is coupled between the driver and the piston rod.

According to an embodiment, the advancement mechanism is configured as a gearing mechanism that reduces the axial movement of the dosing member to a smaller axial advancement of the piston rod. Consequently, axial forces that are applied to the dosing member are converted to larger axial forces exerted by the piston rod, for example on a plunger sealing a cartridge containing the drug to be delivered. This allows for easy delivery of the drug irrespective of its viscosity.

According to an embodiment, the piston rod is rotationally fixed to the housing and the advancement mechanism comprises a nut that is coupled between the piston rod and the dosing member. The nut is thereby threadedly connected to the piston rod. During dose setting, the nut is rotationally fixed with respect to the dosing member and rotatable with respect to the housing. Furthermore, the nut is rotatable with respect to the dosing member and rotationally fixed with respect to the housing during dose delivery.

Such a construction of the advancement mechanism allows to rotate the nut and the dosing member simultaneously during dose setting and thus to axially move both the nut and the dosing member with respect to the housing and the piston rod. Rotationally fixing the nut with respect to the piston rod during dose delivery ensures that both the nut and the piston rod, which are threadedly engaged with each other but not allowed to rotate with respect to each other, move in unison along the longitudinal axis. Therefore, the nut may return to the same position with respect to the housing after each dose delivery while the piston rod is incrementally moved along the longitudinal axis with each dose delivery.

According to an embodiment, the advancement mechanism comprises a driver that is coupled between the nut and the dosing member, wherein the driver is rotationally fixed to the dosing member and axially movable with respect to the dosing member during both dose setting and dose delivery. The driver is further threadedly coupled to the housing and configured to engage with the nut during dose delivery to axially advance the nut and the piston rod upon being rotated by the dosing member. Such a driver allows the implementation of a gearing mechanism with a gearing ratio that is determined by the pitches of the threaded connection between the driver and the housing and of a further threaded connection between the dosing member and the housing.

The drug delivery devices according to the present disclosure do not necessarily have to have a maximum dose stop with a maximum stop feature provided at the housing and a maximum dose stop provided at the dosing member to reliably define the axial travel of the dosing member during dose delivery and to thus allow for precise and safe dose setting and dose delivery. According to the present disclosure, reliable definition of the axial travel of the dosing member is also achieved by providing a zero dose stop that acts directly between the housing and the dosing member.

In a second aspect, the present disclosure is therefore directed at a drug delivery device having a housing with a longitudinal axis, a dose setting member that is actuatable by a user and rotatable around the longitudinal axis to set a dose to be delivered by the drug delivery device, a piston rod that is configured to be axially advanced in a proximal direction to deliver the set dose, and a dosing member for defining the axial advancement of the piston rod upon delivery of the set dose. The dosing member is axially movable along the longitudinal axis and rotationally movable around the longitudinal axis during dose setting. Furthermore, the dosing member is rotationally fixed to the dose setting member during dose setting. The dosing member comprises a zero dose stop that is configured to engage with a zero stop feature to limit movement of the dosing member with respect to the housing upon setting a zero dose, wherein the zero stop feature is provided at the housing.

The drug delivery devices according to the second aspect of the present disclosure may further be configured as it is disclosed in connection with the drug delivery devices according to the first aspect of the present disclosure and vice versa. Therefore, all embodiments and technical effects that are disclosed in connection with the drug delivery devices according to the first aspect of the present disclosure also apply to the drug delivery devices according to the second aspect and vice versa.

In a third aspect, the present disclosure is directed at a drug delivery device having a housing with a longitudinal axis, a dose setting member that is actuatable by a user and rotatable around the longitudinal axis to set a dose to be delivered by the drug delivery device, a piston rod that is configured to be axially advanced in a proximal direction to deliver the set dose, and a dosing member for defining the axial advancement of the piston rod upon delivery of the set dose. The dosing member is thereby axially movable along the longitudinal axis and rotationally movable around the longitudinal axis during dose setting, and rotationally fixed to the dose setting member during dose setting. The dose setting member is connected to the housing via a dose selector member, whereby the dose selector member is rotationally fixed and axially movable with respect to the housing. The drug delivery device further comprises a dose definition mechanism for defining rotational dose positions of the dose setting member with respect to the housing, whereby the dose definition mechanism acts between the dose selector member and the dose setting member.

A dose definition mechanism having such a dose selector member allows to define the rotational positions of the dosing member that correspond to the individual settable doses directly at the dosing member itself and the drug delivery device may therefore be constructed in a simple, yet sturdy way. Due to axial movability of the dose selector member, the individual doses may be defined irrespective of the axial position of the dose setting member during dose setting.

The dose setting member may be axially movable with respect to the dose selector member and/or the dose selector member may be axially fixed with respect to the dosing member.

The drug delivery devices according to the third aspect of the present disclosure may further be configured as it is disclosed in connection with the drug delivery devices according to the first and/or second aspect of the present disclosure and vice versa. Therefore, all embodiments and technical effects that are disclosed in connection with the drug delivery devices according to the first and/or second aspect of the present disclosure also apply to the drug delivery devices according to the third aspect and vice versa.

With all drug delivery devices according to the present disclosure, a medication stored in a cartridge of the device may be selected from the group of members consisting of diabetes medication, such as insulin, growth hormones, fertility hormones, osteoporosis medication, blood thinners, such as heparin, and drugs against migraine, **HIV** associated lipodystrophy, non-alcoholic fatty liver diseases or obesity.

Exemplary embodiments and functions of the present disclosure are described herein in conjunction with the following drawings, showing schematically:
- Fig. 1: a perspective view of a drug delivery device according to the present disclosure with an attached cap;
- Fig. 2: a perspective view of the drug delivery device with the cap removed and an attached dispensing unit;
- Fig. 3: a perspective view of the drug delivery device, the cap and the dispensing unit;
- Fig. 4: a side view of the dispensing unit comprising a cartridge holder and a cartridge and a needle attachable to the dispensing unit;
- Fig. 5: a longitudinal cut of the drug delivery device, the dispensing unit and the cap through a first cutting plane with the drug delivery device being in a dose setting state;
- Fig. 6: a longitudinal cut of the drug delivery device, the first dispensing unit and the cap through a second cutting plane perpendicular to the first cutting plane with the drug delivery device being in the dose setting state;
- Fig. 7: an exploded partial view of a dosing mechanism of the drug delivery device;
- Fig. 8: a longitudinal cut of the dosing mechanism of the drug delivery device through the first cutting plane prior to setting a dose;
- Fig. 9: a longitudinal cut of the dosing mechanism through the first cutting plane after setting a dose, the dosing mechanism being in a dose setting state;
- Fig. 10: a longitudinal cut of the dosing mechanism through the first cutting plane after setting the dose, the dosing mechanism being in a dose delivery state;
- Fig. 11: a longitudinal cut of the dosing mechanism through the first cutting plane after delivering the dose, the dosing mechanism being in the dose setting state;
- Fig. 12: a clutch mechanism of the dosing mechanism in a dose setting state;
- Fig. 13: the clutch mechanism in a dose delivery state;
- Fig. 14: a radial cut through a dose definition mechanism of the drug delivery device;
- Fig. 15: a perspective view of a proximal side of a dose setting member of the drug delivery device;
- Fig. 16: a perspective view of a distal side of a clutch member of the drug delivery device;
- Fig. 17: a perspective view of a proximal side of the clutch member of the drug delivery device;
- Fig. 18: a longitudinal cut through a dosing member and a dose selector member of the drug delivery device with a first friction reduction mechanism;
- Fig. 19: a perspective view of a connection between a nut and a driver of the drug delivery device with a second friction reduction mechanism;
- Fig. 20: a perspective view of a dosing member of the drug delivery device;
- Fig. 21: a longitudinal cut through an inner housing of the drug delivery device;
- Fig. 22: a perspective view of the inner housing with the dosing member in a zero-dose position;
- Fig. 23: a perspective view of the inner housing with the dosing member in a maximum dose position;
- Fig. 24: a longitudinal cut through an outer housing of the drug delivery device;
- Fig. 25: a longitudinal cut through the inner housing mounted within the outer housing of the drug delivery device;
- Fig. 26: a radial cut through the outer and inner housing of the drug delivery device;
- Fig. 27: an exploded partial view of a resetting mechanism of the drug delivery device;
- Fig. 28: a longitudinal cut through the resetting mechanism of the drug delivery device with a resetting element in a proximal position;
- Fig. 29: a distal perspective view of the resetting element of the resetting mechanism;
- Fig. 30: a proximal perspective view of the resetting element;
- Fig. 31: a proximal perspective view of a coupling part of the resetting mechanism;
- Fig. 32: a perspective view of the coupling part and the inner housing;
- Fig. 33: a longitudinal cut through the resetting mechanism with the dispensing unit attached to the drug delivery device and the resetting element located in a distal position;
- Fig. 34: a longitudinal cut through a proximal end of a cartridge holder attachable to the drug delivery device;
- Fig. 35: a perspective distal view of a radial cut through a proximal part of the cartridge holder;
- Fig. 36: a longitudinal cut through a first dispensing unit attachable to a first drug delivery device, a longitudinal cut through a second dispensing unit attachable to a second drug delivery device, and a longitudinal cut through a third dispensing unit attachable to a third drug delivery device;
- Fig. 37: a longitudinal cut through a first connection means of the first drug delivery device and a perspective view of the first connection means, a longitudinal cut through a second connection means of the second drug delivery device and a perspective view of the second connection means, and a longitudinal cut through a third connection means of the third drug delivery device and a perspective view of the third connection means;
- Fig. 38: a perspective view of a further drug delivery device according to the present disclosure;
- Fig. 39: the further drug delivery device with a removed cap;
- Fig. 40: an exploded view of the further drug delivery device;
- Fig. 41: a clutch mechanism of the further drug delivery device;
- Fig. 42: a dose setting member of the further drug delivery device;
- Fig. 43: a dose selector member of the further drug delivery device;
- Fig. 44: an alternative embodiment of the resetting element of the drug delivery device;
- Fig. 45: a longitudinal cut through the alternative embodiment of the resetting element;
- Fig. 46: an alternative embodiment of the coupling part of the drug delivery device;
- Fig. 47: the alternative embodiment of the resetting element and the alternative embodiment of the coupling part mounted to an alternative embodiment of the inner housing of the drug delivery device;
- Fig. 48: a perspective view of an alternative connection between a further alternative embodiment of the inner housing and an alternative embodiment of the dose selector member;
- Fig. 49: a longitudinal cut through the further alternative embodiment of the inner housing and the alternative embodiment of the dose selector member;
- Fig. 50: the alternative embodiments of the inner housing, the dose selector member and the dosing member with the dosing member in a zero-dose position;
- Fig. 51: the alternative embodiments of the inner housing, the dose selector member and the dosing member with the dosing member in a maximum-dose position;
- Fig. 52: an alternative embodiment of the clutch member;
- Fig. 53: the drug delivery device with a further alternative embodiment of the inner housing with a balancing weight located on an outer surface of the inner housing;
- Fig. 54: a radial cut perpendicular to the longitudinal axis through the drug delivery device with the balancing weight;
- Fig. 55: the alternative embodiment of the inner housing;
- Fig. 56: the balancing weight;
- Fig. 57: a radial cut perpendicular to the longitudinal axis through an alternative embodiment of the drug delivery device with the balancing weight;
- Fig. 58: longitudinal cuts through the first, second and third dispensing unit showing additional dimensions;
- Fig. 59: longitudinal cuts through the first, second and third connections means of the first, second and third drug delivery device showing additional dimensions and perspective views of the first, second and third connection means.

In the present disclosure, the term "distal part/end" refers to the part/end of the device, or the parts/ends of the components or members thereof, which in accordance with the use of the device, is located the furthest away from a delivery/injection site of a patient. Correspondingly, the term "proximal part/end" refers to the part/end of the device, or the parts/ends of the components or members thereof, which in accordance with the use of the device is located closest to the delivery/injection site of the patient. A proximal direction is directed towards the delivery/injection site and a distal direction is directed away from the delivery/injection site.

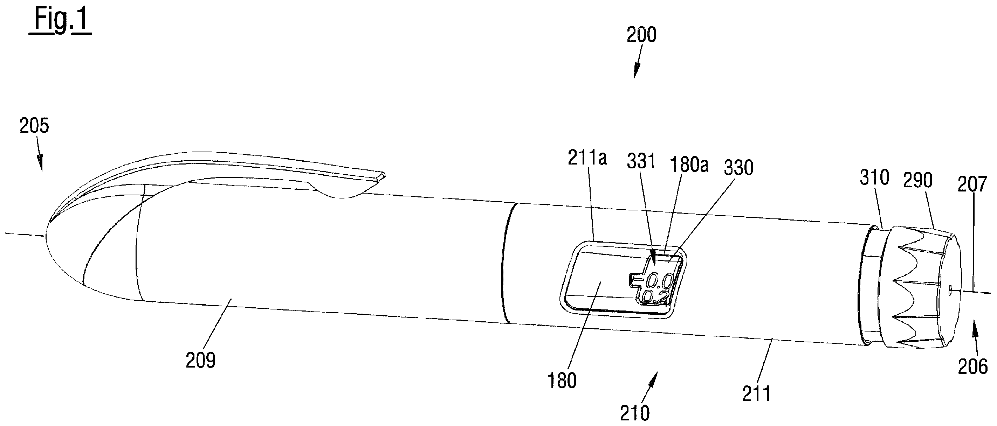

The present disclosure of resetting mechanisms is applicable with a number of medicament delivery devices, for example, injection devices. One possible injection device is the pen-type design illustrated in Fig. 1.

**Fig. 1** shows a drug delivery device 200 that comprises connection means for attaching a dispensing unit according to the present disclosure. The drug delivery device 200 has a generally tubular housing 210, which is elongated along a longitudinal axis 207. A generally tubular cap 209 is attached to a proximal end 205 of the housing 210. At a distal end 206 of the housing 210, which distal end 206 is located opposite to the proximal end 205 along the longitudinal axis 207, the drug delivery device 200 comprises a dose setting member 290.

The dose setting member 290 is rotatable around the longitudinal axis 207 and is configured to be gripped and rotated by a user of the device 200 to set a dose to be delivered by the device 200. In this way the dose setting member 290 can also be considered a knob or the like. In the embodiment shown in Fig. 1, the dose setting member 290 is configured as a knob that terminates the drug delivery device 200 at its distal end 206. With other embodiments, the dose setting member 290 may also be, for example, configured as a rotatable sleeve or ring that surrounds the longitudinal axis 207.

The dose setting member 290 is connected to the housing 210 via a dose selector member 310 that is rotationally locked and axially movable relative to the housing 210 both during dose setting and during dose delivery. When increasing a set dose by turning the dose setting member 290 relative to the housing 210 and the dose selector member 310, the dose selector member 310 moves distally out of the housing 210, thereby also moving the dose setting member 290 in the distal direction.

The housing 210 comprises an outer housing 211, which, in the present embodiment, is made from metal, and an inner housing 180. The inner housing 180 is located within the outer housing 211. In the present embodiment, it is made from a plastic material. The housing 210 comprises a window, which is formed by a window 211a within the outer housing 211 through which a part of the inner housing 180 and a window 180a within the inner housing 180 is visible to a user of the device 200. Through the window of the housing 210, a dose indication member 330, which is located inside the housing 210, namely inside the generally tubular inner housing 180, is visible to the user.

The dose indication member 330 is also configured as a generally tubular member and carries on its outer cylindrical surface a dose scale comprising several optical markers 331 that correspond to the respective set dose. When setting a dose, the dose indication member 330 rotates within the inner housing 180, which changes the location of the scale and thus also the optical markers 331 visible through the windows 211a and 180a.

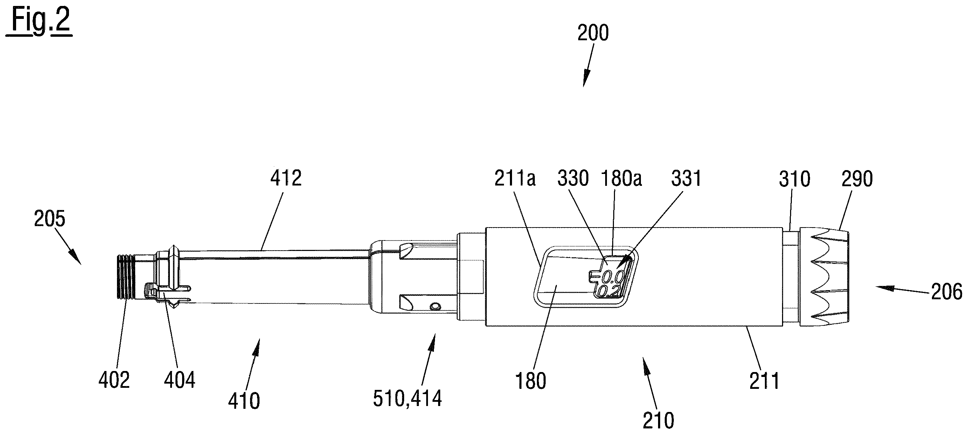

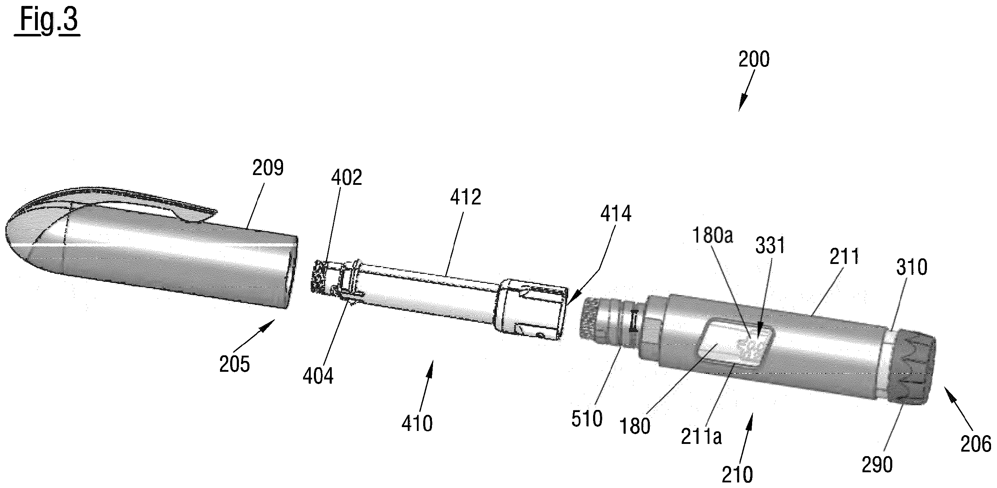

**Fig.** 2 shows the drug delivery device 200 with the cap 209 removed. A dispensing unit 410 that comprises the drug to be delivered by the device 200 is removably attached to the proximal end 205 of the housing 210. Fig. 3 shows the cap 209 and the dispensing unit 410 removed from the drug delivery device 200. With the cap 209 and the dispensing unit 410 attached to the housing 210 of the device 200, the dispensing unit 410 is fully received within the cap 209.

The dispensing unit 410 comprises a cartridge holder 412, which, in the current embodiment, is made from a plastic material. The cartridge holder 412 may, for example, be formed by injection molding. The cartridge holder 412 attaches to the outer housing 211 of the drug delivery device 200 via a connection, which comprises first connection means 510 located at the proximal end of the housing 210 and corresponding first connection means 414 located at the distal end of the dispensing unit 410. The first connection means 510 of the housing 210 are formed as integral part of the housing 210, namely as integral part of the outer housing 211, and the first connection means 414 of the dispensing unit 410 are formed as integral part of the cartridge holder 412.

At its proximal end, the cartridge holder 412 of the dispensing unit 410 comprises a needle connector 402 that is configured to receive a hollow needle or cannula through which the drug is delivered by the drug delivery device 200. In the present embodiment, the needle connector 402 is configured as a threaded connector. With other embodiments, the needle connector 402 may also be configured as, for example, a snap-fit, bayonet or Luer-Lok connection.

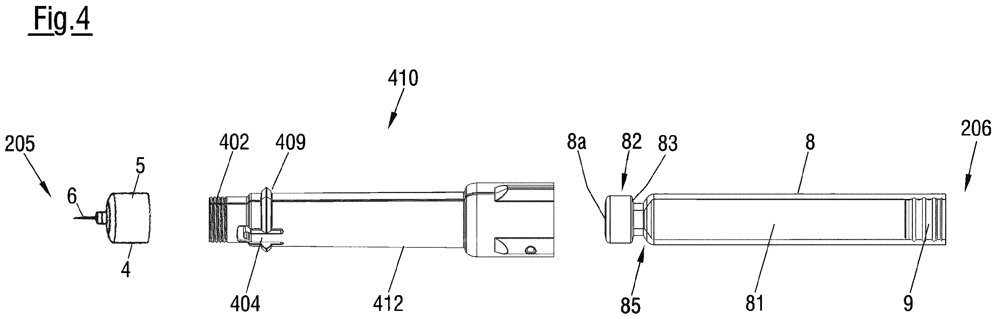

**Fig. 4** shows the cartridge holder 412 of the dispensing unit 410 and a cartridge 8 that may be inserted into the cartridge holder 412, as well as a needle 4 attachable to the needle connector 402.

The cartridge 8 has a generally cylindrical body, which, in the present embodiment, is made from glass, and which surrounds a drug compartment 81 that contains a liquid drug to be delivered by the drug delivery device 200. The drug compartment 81 is sealed at its distal end by an elastic plunger 9, which is movable along the longitudinal axis within the body of the cartridge 8. At its proximal end, the cartridge 8 comprises an annular rim 82, which is separated from the body by an annular detent 85 located distally from the annular rim 82. At a proximal front surface of the cartridge 8, which is orientated perpendicular to the longitudinal axis 207, the cartridge 8 comprises a sealing means or septum 8a, which seals the drug compartment 81 in the proximal direction.

When being fully inserted into the cartridge holder 412, the sealing means 8a is located at the proximal end of the cartridge holder 412 and accessible through an opening at the proximal end of the cartridge holder 412. The cartridge 8 is non-releasably held in its inserted position by a connector 404. The connector 404 is configured as a flexible member. In the present embodiment, it is configured as a snap hook. The connector 404 is formed by a cut-out portion of the cartridge holder 412. Upon insertion of the cartridge 8 into the cartridge holder 412, the connector 404 snaps over the annular rim 82 of the cartridge 8. A radially inwardly protruding finger of the connector 404 is then located within the annular detent 85 of the cartridge 8 and prevents distal movement of the cartridge 8 by abutting against a distal surface 83 of the annular rim 82.

This non-releasable connection between the cartridge 8 and the cartridge holder 412 prevents a removal of the cartridge 8 from the cartridge holder 412 during intended use of the dispensing unit 410. For example, it prevents removal of the cartridge 8 unless the connector 404 is intentionally and/or forcefully brought out of engagement with the annular rim 82. The non-releasable connection is thereby configured in a way that such disengagement is only possible using tools or excessive forces that are higher than the forces acting on the non-releasable connection during normal and/or intended use of the dispensing unit 410, for example during mounting of the dispensing unit 410 to the housing 210, during attachment of the needle 4 to the cartridge holder 412 or during handling of the dispensing unit 410 with the cartridge 8 inserted into the cartridge holder 412. This handling may also comprise shock forces that may occur during transport and/or unintentional dropping of the dispensing unit and that do not exert forces that would destroy the dispensing unit 410 and/or the cartridge holder 412 and/or the cartridge 8. The non-releasable connection between the cartridge 8 and the cartridge holder 412 allows to provide and sell the dispensing unit 410 with an inserted cartridge 8 as a single, pre-mounted unit.

The needle 4 is configured as a pen needle. It comprises a hub 5 that carries a double-ended cannula 6. The cannula 6 is longitudinally received within the hub 5. The hub 5 comprises at its distal end a hub connector that matches the needle connector 402 of the cartridge holder 412. In the present embodiment, the hub connector is configured as an inner thread matching the outer thread of the needle connector 402. The cannula 6 protrudes from the proximal end of the hub 5. It has sharp ends at both its proximal and distal ends. With its distal end, the cannula 6 penetrates the sealing means 8a of the cartridge 8 and thus establishes a fluid connection between the drug compartment 81 and the proximal end of the cannula 6. The proximal end of the cannula 6 is configured to be inserted into a delivery site, such as a skin of the user of the device 200, thereby permitting injection of the drug into the delivery site.

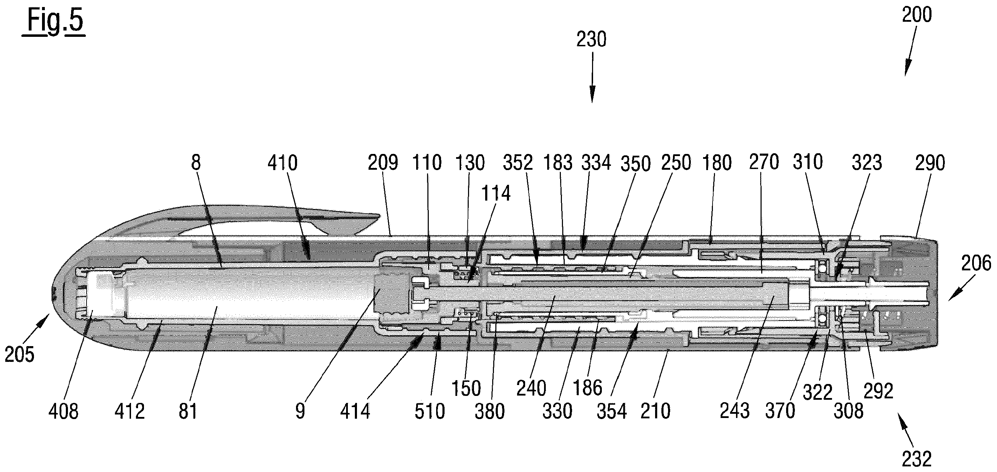

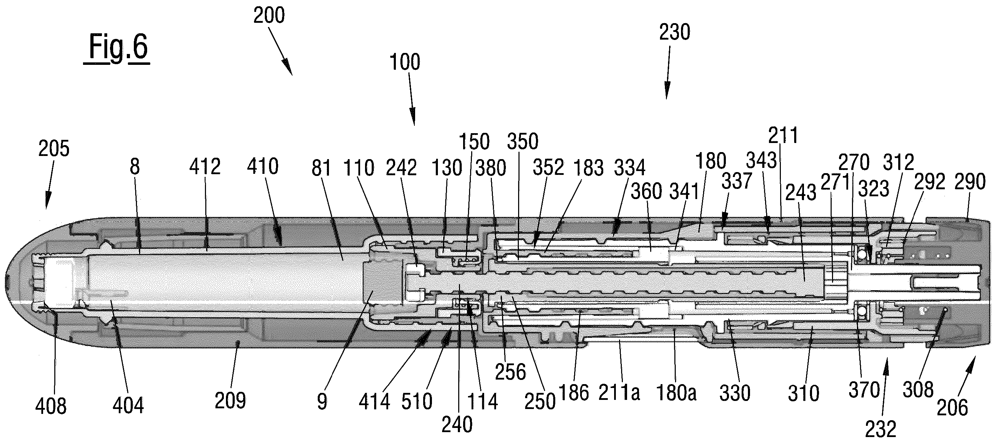

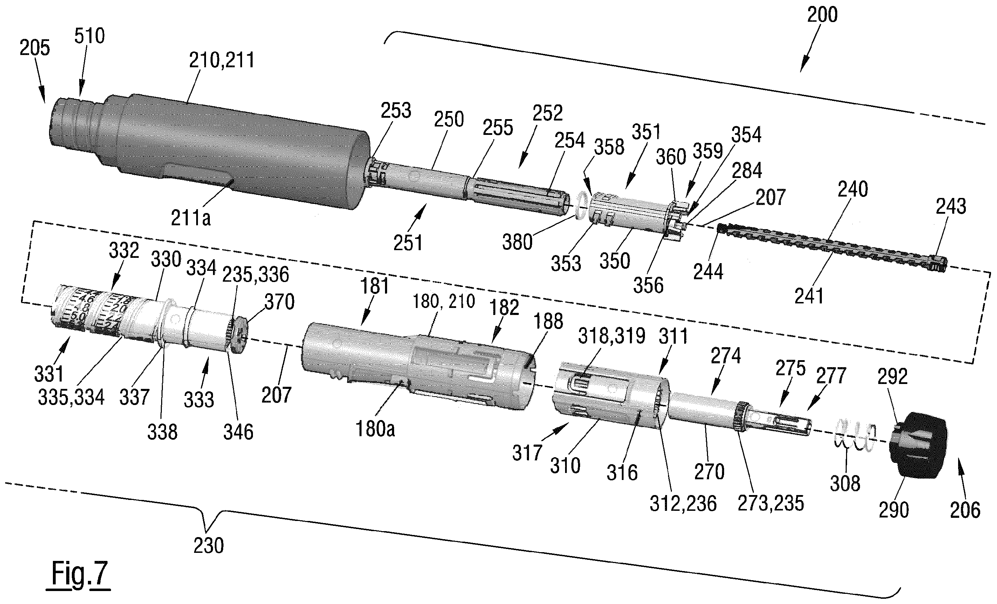

**Fig. 5** and **Fig. 6** show longitudinal cuts through the drug delivery device 200 along two different cutting planes that are orientated perpendicular to each other. **Fig. 7** shows a partial exploded view of the components of the drug delivery device 200 that are visible in Fig. 5 and Fig. 6. The drug delivery device 200 comprises a dosing mechanism 230 that is configured to set a dose of drug to be delivered by the drug delivery device 200 and to expel the set dose by moving the plunger 9 in the proximal direction.

The dosing mechanism 230 comprises a piston rod assembly with a piston rod 240, which is elongated along the longitudinal axis 207, and a plunger disc 242 (see Figs. 5 and 6) mounted to the proximal end of the piston rod 240. The piston rod assembly is configured to directly contact the plunger 9 by the plunger disc 242 and to advance the plunger 9 within the cartridge 8 upon movement of the piston rod assembly in the proximal direction. The piston rod 240 has a non-circular cross-section and an outer thread 241 that essentially covers its entire length. At its proximal end, the piston rod 240 comprises a disc connector 244 for receiving the plunger disc 242. At its distal end, the piston rod 240 comprises a stop feature 243, which terminates the outer thread 241 and is exemplarily configured as a thickened portion of the piston rod 240 having a larger radial extent than the minor diameter of the thread 241.

The piston rod 240 is located within the housing 210 that is within the outer housing 211 and the inner housing 180. In use, the piston rod 240 can protrude from the proximal end of the housing 210 such that the plunger disc 242 may be completely moved out of the housing 210 and into the cartridge 8. The piston rod 240 always protrudes from the proximal end of the inner housing 180. It may be completely retracted into the outer housing 211, for example, after resetting and/or prior to and/or directly after attaching a new dispensing unit 410 to the device 200. During the use of the device 200, the piston rod 240 is moved in the proximal direction to also protrude from the outer housing 211. The plunger disc 242 is permanently located outside the inner housing 180 and may be fully retracted into the outer housing 211, for example after completion of a resetting operation and/or prior to and/or directly after attaching a new dispensing unit 410 to the device 200.

The piston rod 240 is rotationally locked with respect to the housing 210 during both dose setting and dose delivery. In the present embodiment, the piston rod 240 is connected to the housing 210 via a resetting element 110 of a resetting mechanism 100 of the drug delivery device 200, see Figs. 5 and 6. The resetting element 110 is rotationally fixed with respect to the housing 210 during both dose delivery and dose setting. It comprises a longitudinal opening 114 that receives the piston rod 240 such that the plunger disc 242 is located at a proximal side of the opening 114 and the stop feature 243 is located at a distal side of the opening 114. The opening 114 is configured as a through hole with a non-circular cross section that matches the non-circular cross-section of the piston rod 240 thereby allowing axial movement but preventing rotational movement of the piston rod 240 with respect to the resetting element 110.

The piston rod 240 is surrounded by a hollow, generally cylindrical nut 250. The nut 250 is threadedly engaged with the thread 241 of the piston rod 240. In the present embodiment, the nut 250 comprises a threaded section with an inner thread 256 that engages the outer thread 241 of the piston rod 240. The threaded section is located in a proximal part 251 of the nut 250, at the proximal end of the nut 250. With other embodiments, the threaded section may also cover other parts of the nut 250 or be located at other portions of the nut 250. The nut 250 further permanently surrounds the stop feature 243 of the piston rod 240, irrespective of the set and/or delivered doses.

The nut 250 has a distal part 252 that is surrounded by a proximal part 274 of a clutch member 270 of the dosing mechanism 230. The nut 250 is rotationally fixed to the clutch member 270 and axially movable with respect to the clutch member 270.

In the present embodiment, the nut 250 is engaged with the clutch member 270 by a splined connection between the nut 250 and the clutch member 270. The splined connection exemplarily comprises longitudinal grooves 254 that are located on the outer surface of the distal part 252 of the nut 250 and that are distributed around the circumference of the nut 250. The grooves 254 run parallel to the longitudinal axis 207 and are engaged by corresponding longitudinal ridges 271 that are distributed on an inner surface of the clutch member 270, see Fig. 6.

With other embodiments, a rotationally fixed and axially movable connection between the nut 250 and the clutch member 270 may also be achieved by different means, for example by a splined connection between longitudinal ridges on the outer surface of the nut 250 and corresponding longitudinal grooves on the inner surface of the clutch member 270. Additionally or alternatively, the connection may also be mediated by one or more intermediate members.

The clutch member 270 is, at its distal end, fixedly connected to the dose setting member 290 by a connection 277 that prevents both relative axial and relative rotational movement between the clutch member 270 and the dose setting member 290. With other embodiments of the drug delivery device 200, the dose setting member 290 and the clutch member 270 may also be configured as a single component. Alternatively, the connection between the clutch member 270 and the dose setting member 290 may also be mediated by one or more intermediate members.

In its proximal part 251, the nut 250 is surrounded by a driver 350. The driver 350 is configured as a hollow, generally cylindrical member. Furthermore, the driver 350 is both axially and rotationally movable with respect to the housing 210 during both dose setting and dose delivery. Thereby, the driver 350 is threadedly engaged with the housing 210.

The inner housing 180 comprises at its proximal end an inner sleeve 183 that receives a proximal part 351 of the driver 350. The driver 350 comprises a thread 353 that engages with a drive thread 186 of the inner sleeve 183. In the exemplary embodiment, the thread 353 of the driver 350 is configured as an outer thread and the drive thread 186 is configured as an inner thread. The thread 353 is located on the proximal part 351 of the driver 350. With other embodiments, a threaded connection between the driver 350 and the housing 210 may also be achieved by other ways, for example by an outer thread on the housing 210 and an inner thread on the driver 350.

The dosing mechanism 230 furthermore comprises the dosing member 330. The dosing member 330 is configured as a hollow generally cylindrical member. It surrounds both the driver 350 and the clutch member 270. The dosing member 330 constitutes a dose setting sleeve of the drug delivery device 200.

The driver 350 is located within a proximal part 331 of the dosing member 330 and the clutch member 270 is located with its proximal part 274 in a distal part 333 of the dosing member 330.

The dosing member 330 is axially and rotationally movable with respect to the housing 210 during both dose setting and dose delivery. It is furthermore threadedly engaged with the housing 210 so that it is forced to move on a helical path with respect to the housing 210.

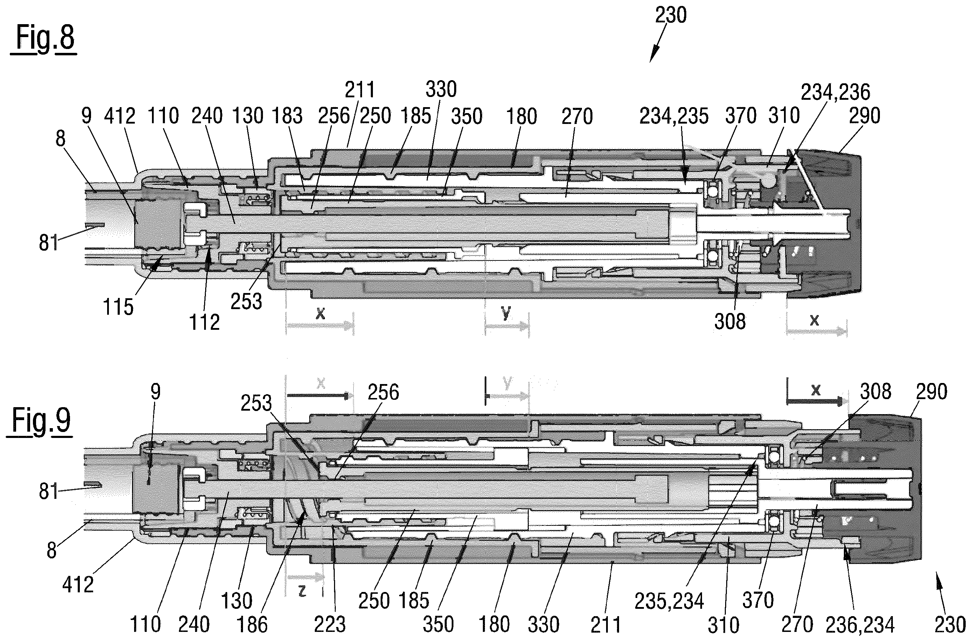

The dosing member 330 is located between the inner sleeve 183 and an outer wall of the inner housing 180. It has a thread 335 that is engaged with a dose thread 185 of the housing 210 (see Fig. 8). With the exemplary embodiment, the thread 335 of the dosing member 330 is configured as an outer thread and the dose thread 185 is configured as an inner thread located on an inner surface of the outer wall of the inner housing 180. With other embodiments, a threaded connection between the dosing member 330 and the housing 210 may also be realized in different ways. For example, the threaded connection could be provided between the dosing member 330 and the inner sleeve 183 of the inner housing 180.

The dosing member 330 is configured as a dose indication member and comprises the optical markers 331 on its outer surface. The optical markers 331 form a helical scale with a pitch that corresponds to the pitch of the thread 335 on the outer surface of the dosing member 330.

The driver 350 is axially movable and rotationally fixed with respect to the dosing member 330 during both dose setting and dose delivery. With the exemplary embodiment, this is achieved by a splined connection between the driver 350 and the dosing member 330.

The driver 350 comprises radially extending longitudinal splines 360 that engage with corresponding longitudinal grooves 341 provided on an inner surface of the dosing member 330 (see Fig. 6). The splines 360 are located in the distal part 359 of the driver 350 and the grooves 341 are located in a proximal part 332 of the dosing member 330. With other embodiments, the splined connection between the driver 350 and the dosing member 330 may also be achieved in different ways. For example, the driver 350 may comprise grooves that are engaged by corresponding splines of the dosing member 330.

The dose selector member 310 is configured as a hollow, generally cylindrical member. It constitutes a dose selector sleeve of the drug delivery device 200.

The dose selector member 310 is axially fixed and rotationally movable with respect to the dosing member 330. Therefore, the dose selector member 310 is forced to axially follow a movement of the dosing member 330 while the dosing member 330 is free to rotate with respect to the dose selector member 310, which itself is rotationally fixed with respect to the housing 210.

The dosing member 330 is received within the dose selector member 310. With the current embodiment, a proximal part 317 of the dose selector member 310 receives the distal part 333 of the dosing member 330. The clutch member 270, the proximal part 274 of which is located within the dosing member 330, axially extends with its distal part 275 from the dosing member 330. The distal part 275 of the clutch member 270 thereby extends through an opening 323 in a radially orientated inner wall 322 of the dose selector member 310 (see Fig. 5), which inner wall 322 separates the proximal part 317 of the dose selector member 310 from a distal part 311.

**Fig. 8** shows a longitudinal cut of the dosing mechanism 230 of the drug delivery device 200 through the first cutting plane prior to setting a dose to be delivered by the drug delivery device 200. To set the dose, the dose setting member 290 is gripped by a user and rotated with respect to the housing 210. This causes the clutch member 270 to rotate together with the dose setting member 290. Due to the rotationally fixed connection between the clutch member 270 and the nut 250, the nut 250 also rotates together with the dose setting member 290. Since the piston rod 240 is rotationally fixed with respect to the housing 210 and the piston rod 240 is threadedly engaged with the nut 250, rotation of the nut 250 causes the nut 250 to axially advance along the piston rod 240 in the distal direction. When increasing the set dose, the nut 250 travels in the distal direction, and when decreasing the set dose, the nut 250 travels in the proximal direction.

During dose setting, the dose setting member 290 is rotationally fixed with respect to the dosing member 330. This is achieved by a clutch mechanism 234 that comprises a first part 235 that acts between the dose setting member 290 and the dosing member 330.

The first part 235 of the clutch mechanism 234 comprises clutch elements 336 (see Fig. 7) that are located on the dosing member 330 and that engage, during dose setting, with corresponding clutch elements 273 located on the clutch member 270. The engagement between these clutch elements 336, 273 prevents relative rotational movement between the dose setting member 290 and the dosing member 330 while allowing axial movement for disengagement of the first part 235 of the clutch mechanism 234.

Since the first part 235 of the clutch mechanism 234 is closed during dose setting, the dosing member 330 rotates together with the dose setting member 270. The threaded engagement between the dosing member 330 and the housing 210 then causes the dosing member 330 to axially travel within the housing 210 during dose setting. Upon increasing the set dose, the dosing member 330 travels in the distal direction, and upon decreasing the set dose, the dosing member 330 travels in the proximal direction.

Since the dose selector member 310 is axially fixed with respect to the dosing member 330, distal movement of the dosing member 330 causes the dose selector member 310 to axially travel out of the housing 310 in the distal direction, thereby also moving the dose setting member 290 into the distal direction, while proximal movement of the dosing member 330 causes the dose selector member 310 to axially travel into the housing 210 thereby also moving the dose setting member 290 into the proximal direction.

As the dosing member 330 is rotationally fixed with respect to the driver 350, rotation of the dosing member 330 also causes the driver 350 to rotate together with the dose setting member 290. The threaded connection between the driver 350 and the housing 210 then causes the driver 350 to move in the distal direction when increasing the set dose and to move in the proximal direction when decreasing the set dose.

A first pitch of the threaded connection between the piston rod 240 and the nut 250 and a second pitch of the threaded connection between the driver 350 and the housing 210 are matched to each other to cause the nut 250 and the driver 350 to travel essentially the same axial distance upon rotational movement of the dose setting member 290. The first and second pitches are smaller than a third pitch of the threaded connection between the dosing member 330 and the housing 210. This causes the dosing member 330 to travel a larger axial distance upon rotation of the dose setting member 290 than the nut 250 and the driver 350.

With the device 200, the nut 250 and the clutch member 270 are only rotationally locked but free to move axially with respect to each other. This allows the clutch member 270 and the dose setting member 290 to travel larger distances in the axial direction during dose setting than the nut 250. Likewise, the driver 350 and the dosing member 330 are only rotationally locked but free to move axially with respect to each other. This allows the dosing member 330 to travel larger distances in the axial direction during dose setting than the driver 350.

**Fig. 9** shows the dose setting mechanism 232 after a dose has been set. During dose setting, the dosing member 330 has traveled a first distance x in the distal direction, while the driver 350 has traveled a second distance y and the nut 250 has traveled a third distance z. The first distance x is larger than the second and third distances y, *z.*

Due to manufacturing tolerances, the first pitch of the threaded connection between the piston rod 240 and the nut 250 varies among different threaded connections between a minimum first pitch and a maximum first pitch and the second pitch of the threaded connection between the driver 350 and the housing 210 varies among different threaded connections between a minimum second pitch and a maximum second pitch. With the drug delivery device 200, the maximum first pitch is smaller than or at most equal to the minimum second pitch. This ensures that the second distance y traveled by the driver 350 in the distal direction is always slightly larger than the third distance *z* traveled by the nut 250.

The dose setting member 290, which also acts as an actuation member to effect injection of the set dose, is axially movable with respect to the dose selector member 310 and the dosing member 330 between a distal position and a proximal position. A biasing member 308, which is configured as a compression spring, biases the dose setting member 290 into the distal position during dose setting.

To effect ejection of a set dose, the user of the device 200 pushes the actuation member, which is formed by the dose setting member 290, from the distal position into the proximal position. This transfers the dosing mechanism 230 from a dose setting state into a dose delivery state. The dosing mechanism 230 of the drug delivery device 200 is configured to allow for a setting of the dose to be injected when the dose delivery device 200 and the dosing mechanism 230 are in the dose setting state, while it is configured to allow for a delivery of the set dose when the dose delivery device 200 and the dosing mechanism 230 are in the dose delivery state.

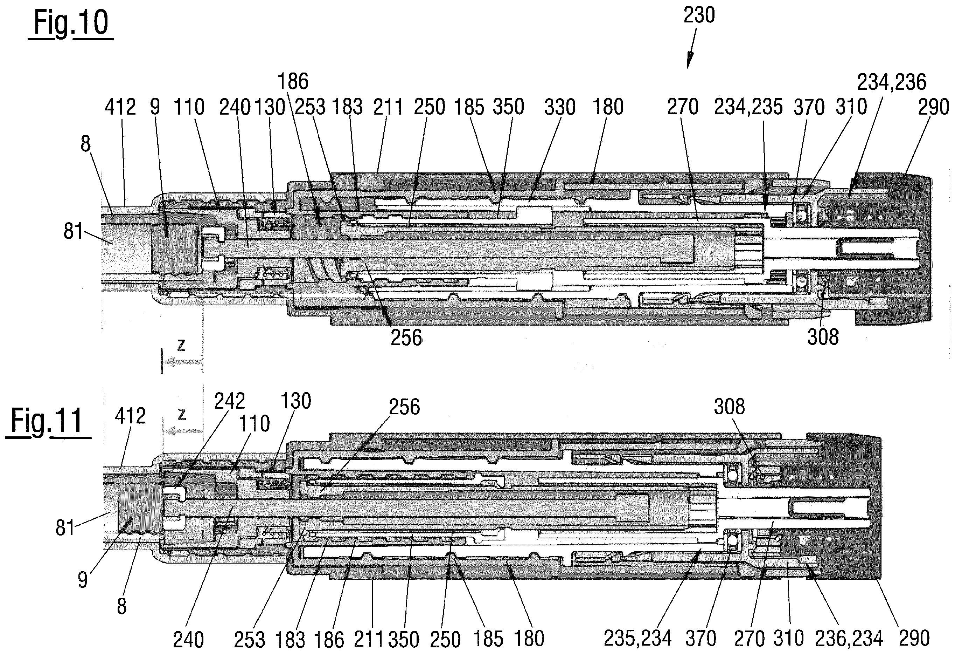

**Fig. 10** shows the dosing mechanism 230 after the dose has been set and the dosing mechanism 230 has been transferred from the dose setting state into the dose delivery state. Moving the dose setting member 290 into the proximal direction also causes the clutch member 270 to move into the proximal direction. Thereby, the first part 235 of the clutch mechanism 234 opens and the clutch elements 273 of the clutch member 270 are disengaged from the clutch elements 336 of the dosing member 330. Therefore, the dosing member 330 and the driver 350 are free to rotate with respect to the dose setting member 290, the clutch member 270 and the nut 250.

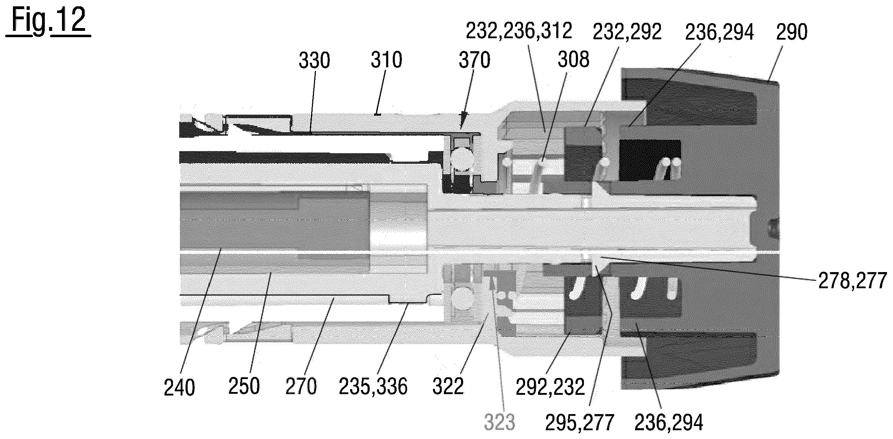

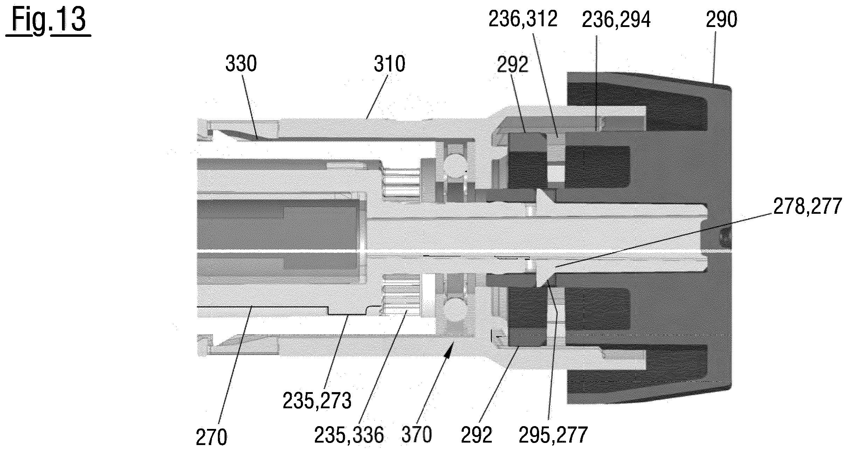

Proximal movement of the dose setting member 290 with respect to the dose selector member 310 at the same time causes a second part 236 of the clutch mechanism 234 to close and to rotationally lock the nut 250 with respect to the piston rod 240 and the housing 210. The second part 236 of the clutch mechanism 234 acts between the dose selector member 310 and the dose setting member 290 and is further described in connection with Fig. 12 and Fig. 13 below.

Further pushing the dose setting member 290 in the proximal direction then causes the dose selector member 310 to linearly move back into the housing 210. The dose selector member 310 thereby pushes against the dosing member 330, which causes the dosing member 330 to rotate due to its threaded engagement with the housing 210. Rotation of the dosing member 330 is transferred to the driver 350, which therefore also moves into the proximal direction due to its threaded engagement with the housing 210.

The difference in the pitches of the threaded connection between the dosing member 330 and the housing 210 and the threaded connection between the driver 350 and the housing 210 thereby causes a mechanical advantage that translates a first axial force exerted by the user and acting on the dosing member 330 into a second axial force exerted by the driver 350. With the dose delivery device 200, the second axial force is larger than the first axial force.

When moving in the proximal direction during dose delivery, the driver 350 pushes axially against the nut 250 and thereby advances the nut 250 in the proximal direction. Since the nut 250 is blocked from rotation with respect to the piston rod 240 during dose delivery due to its connection to the housing 210 via the clutch member 270, the dose setting member 290 and the dose selector member 310, the threaded connection between the nut 250 and the piston rod 240 axially fixes the nut 250 and the piston rod 240 with respect to each other during dose delivery. Therefore, the axially moving nut 250 urges the piston rod 240 to also move in the proximal direction and to thereby advance the plunger 9 to expel the drug from the drug compartment 81.

The housing 250, the dosing member 330, which is threadedly engaged with the housing 250 and rotationally respect to the driver 350, the driver 350, which is also threadedly engaged with the housing 250, and the nut 250, which is pushed in the proximal direction by the driver 350 during dose delivery, form an advancement mechanism of the drug delivery device 200. The advancement mechanism is configured to translate axial movement of the dosing member 330 into axial advancement of the piston rod 240 during dose delivery. Thereby, the advancement mechanism comprises a gearing mechanism provided by the differently pitched threaded connections between the housing 250 and the dosing member 330 on the one hand and between the housing 250 and the driver 350 on the other hand. The gearing mechanism effects a mechanical advantage that translates the first axial force exerted by the user and acting on the actuation member formed by the dose setting member 290 into a second axial force exerted by the piston rod 240 on the plunger 9. This second axial force corresponds to the second axial force exerted by the driver 350 on the nut 250. With the present embodiment, the second axial force is different from the first axial force, namely higher than the first axial force. With other embodiments, the second axial force may also be smaller than the first axial force or essentially equal the first axial force.

Closing of the second part 236 of the clutch mechanism 234 upon dose delivery also rotationally locks the dose setting member 290 to the housing 210 during dose delivery. This ensures that the dose setting member 290 does not rotate during dose delivery and therefore avoids the user being disturbed by a rotation of the dose setting member 290 when the user presses the dose setting member 290 to effect dose delivery. The drug delivery device 200 does not comprise any component that would be accessible by a user from the outside of the device 200 and that rotate during dose delivery. This helps to ensure a safe delivery of the drug during injection.

**Fig. 11** shows the dosing mechanism 230 after the dose has been delivered. The nut 250, the driver 350 and the dosing member 330 have returned to their initial positions while the piston rod 240 has been advanced in the proximal direction by the third distance z. Since the piston rod 240 presses against the plunger 9 via the plunger disc 242, the plunger 9 has also been moved by the third distance *z* in the proximal direction.

**Fig. 12** shows the clutch mechanism 234 of the dosing mechanism 230 in the dose setting state and **Fig. 13** shows the clutch mechanism 234 in the dose delivery state.

In the dose setting state shown in Fig. 12, the dose setting member 290 and the clutch member 270 are in their distal position with respect to the dose selector member 310 and the dosing member 330. The first part 235 of the clutch mechanism 234 is closed and rotationally fixes the clutch member 270 to the dosing member 330.

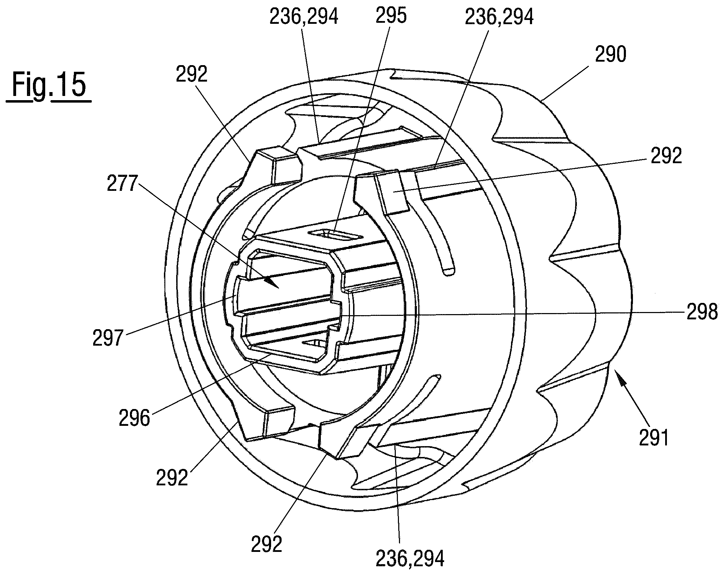

The second part 236 of the clutch mechanism 234 is configured to rotationally fix the dose setting member 290 to the dose selector member 310 during dose delivery. The second part 236 comprises clutch elements 294 (see also Fig. 15) that are provided at the dose setting member 290. As can be seen from Fig. 13, moving the dose setting member 290 into the proximal position brings the clutch elements 294 into engagement with functional features 312 of the dose selector member 311, thereby rotationally locking the dose setting member 290 to the dose selector member 311. The functional features 312 are configured as teeth. The functional features 312 are provided on the inner surface of the distal part 311 of the dose selector member 310. They constitute clutch elements of the dose selector member 310. As can also be seen from Fig. 13, pressing the dose setting member 290 into the proximal position disengages the clutch elements 273 of the clutch member 270 from the clutch elements 336 of the dosing member 330.

Generally speaking, the clutch mechanism 234 rotationally locks the nut 250 to the dosing member 330 and/or to the driver 350 during dose setting and rotationally decouples the nut 250 from the dosing member 330 and/or the driver 350 during dose delivery. Furthermore, generally speaking, the dosing mechanism 230 is configured to prevent relative rotation between the nut 250 and the piston rod 240 and/or the housing 210 during dose delivery and to allow rotation of the nut 250 with respect to the piston rod 240 and/or the housing 210 during dose setting. With the drug delivery device 200, this is achieved by the clutch mechanism 234.

The clutch mechanism 234 furthermore rotationally locks the dose setting member 290 to the dosing member 330 during dose setting and allows for relative rotation between the dose setting member 290 and the dosing member 330 during dose delivery. The clutch mechanism 234 also rotationally locks the dose setting member 290 to the housing 210 during dose delivery and allows for relative rotation between the dose setting member 290 and the housing 210 during dose setting.

With other embodiments of the drug delivery device 200, the dose setting member 290 may also be permanently rotationally locked to the dosing member 330. For example, such a dose setting member 290 may be configured as a part of the dosing member 330 that is accessible to a user of the device. Such an embodiment of the drug delivery device 200 may then comprise an actuation member that may be pushed by a user to effect dose delivery and that is separate from the dose setting member 290. The actuation member may then be rotationally movable with respect to the dose setting member 290 at least during dose delivery. Pushing the actuation member in the proximal direction upon initiating dose delivery may then rotationally decouple the nut 250 from the dosing member 330.

The dosing mechanism 230 of the drug delivery device 200 further comprises a dose definition mechanism 232 that acts between two members of the dosing mechanism 230 that are rotationally movable with respect to each other during dose setting. The dose definition mechanism 232 defines distinct and/or discrete rotational positions of the dose setting member 290 and the dosing member 330 with respect to the housing 210 that correspond to individual settable doses of the drug to be ejected by the dosing mechanism 230. Furthermore, the dose definition mechanism 232 provides audible and/or tactile feedback to a user of the drug delivery device 200, thereby indicating rotational positions of the dose setting member 290 and the dosing member 330 that correspond to settable doses.

With the exemplary embodiment of the drug delivery device 200, the dose setting member 290 is configured to perform more than one full rotation during dose setting. Therefore, one discrete rotational position of the dose setting member 290 may correspond to more than one settable dose. The dose setting member 219 than assumes different axial positions, for example discrete axial positions, relative to the housing 210 for each individual settable dose. With other embodiments of the drug delivery device 200, the dose setting member 290 may also be configured to perform less than one full rotation during dose setting. The discrete rotational positions of the dose setting member 290 defined by the dose definition mechanism 232 then also correspond to distinct rotational positions. In general, with distinct rotational positions, each individual rotational position corresponds to only a single dose value settable by the dose definition mechanism 232.

With the drug delivery device 200, the dose definition mechanism 232 acts between the dose selector member 310 and the dose setting member 290, as can be seen from Figs. 12 and 13. Thereby, the dose definition mechanism 232 is realized by direct engagement between the dose setting member 290 and the dose selector member 310. With other embodiments of dose delivery devices according to the present disclosure, the dose definition mechanism 232 may also act between the dose selector member 310 and the dose setting member 290 via additional elements that are located between the dose selector member 310 and the dose setting member 290. Such an additional element could be, for example, the clutch member 270 and/or the dosing member 330.

As can also be seen from Fig. 12, the dose definition mechanism 232 comprises at least one element 292 that engages with at least one corresponding functional feature 312, exemplarily with the one of the teeth, when the dose setting member 290 reaches a rotational position with respect to the housing 210 that corresponds to a respective dose defined by the functional feature 312. Engagement between the element 292 and the functional feature 312 then provides audible and/or tactile feedback to the user of the drug delivery device 200. As can be seen from Fig. 12, the element 292 is provided at the dose setting member 290. In particular, it is configured as an integral element of the dose setting member 290.

At least one of the element 292 and the functional feature 312 are configured as a flexible element that deflects in a radial direction upon engagement between the element 292 and the functional feature 312. With the drug delivery device 200, the element 292 is configured as such a flexible element. Additionally or alternatively, also the functional features 312 may be configured as flexible elements with other embodiments of the dose definition mechanism 232.

The functional features 312 constitute dose stops of the drug delivery device 200. With the teeth, the drug delivery device 200 comprises several functional features 312 that are circumferentially distributed around the longitudinal axis 207 to define a multitude of settable doses. The functional features 312 form rigid elements of the dose definition mechanism 232 that interact with the flexible elements formed by the elements 292. The elements 292 interact with the functional features 312 by riding over the functional features 312 during dose setting. Thereby, the flexible elements, exemplarily formed by the elements 292, bend in the radial direction.

With each individual functional feature 312, the drug delivery device 200 comprises at least one element that is involved in performing two functions of the dosing mechanism 230. As component of the clutch mechanism 234, the element constitutes a clutch element that serves to rotationally fix the nut 250 and/or the dose setting member 290 to the piston rod 240 and or the housing 210. As component of the dose definition mechanism, it constitutes a dose stop that defines rotational positions of the dosing member 330 and/or the dose setting member 290 with respect to the housing 210. With other embodiments of the drug delivery device 200, the functional features 312 may act only as dose stops and not as clutch elements or only as clutch elements and not as dose stops. With the drug delivery device 200, the elements performing said two functions are configured as rigid teeth. Other embodiments may comprise differently configured elements, such as elastic elements or the like. In particular, the elements acting as dose stops may be configured as elastic elements.

Furthermore, the dose definition mechanism 232 of the drug delivery device 200 comprises a multitude of elements 292, namely four elements 292, that are distributed around the longitudinal axis 207. A relative position between the individual functional features 312 and the individual elements 292 is chosen in a way that at each rotational position of the dose setting member 290 with respect to the housing 210, which correspond to a settable dose, all elements 292 engage with a respective one of the functional features 312. Other embodiments of the drug delivery device 200 may also comprise other numbers of elements 292, for example a single element 292.

With the drug delivery device 200, the functional features 312 are located on an inner surface of the dose selector member 310 and the elements 292 are located on an outer surface of the dose setting member 290. Furthermore, the element 292 and the three further elements 292 are configured as flexible arms. They constitute integral parts of the dose setting member 290 and are provided at a proximal end of the dose setting member 290.

With the drug delivery device 200, the functional features 312 comprise flat side surfaces that engage with corresponding flat side surfaces of the elements 292. Furthermore, the clutch elements 294 also comprise flat side surfaces that engage with the flat side surfaces of the functional features 312. As it is the case for the drug delivery device 200, the flat side surfaces of the functional features 312 and/or of the clutch elements 294 and/or of the elements 292 may be angled with respect to radial planes that comprises the longitudinal axis 207 and intersect the flat side surface of the respective functional feature 312 and/or clutch element 294 and/or element 292.

The functional features 312 provided on the dose selector member 310 constitute both clutch elements of the second part 236 of the clutch mechanism 234 and dose stops of the dose definition mechanism 232.

The dose definition mechanism 232 is configured to inhibit the tactile and/or audible feedback that is provided during dose setting to a user when the drug delivery device 200 is in the dose delivery state. With the drug delivery device 200, this is exemplarily achieved by preventing relative rotation between the two members that provide the dose definition mechanism 232, namely the dose setting member 290 and the dose selector member 310.

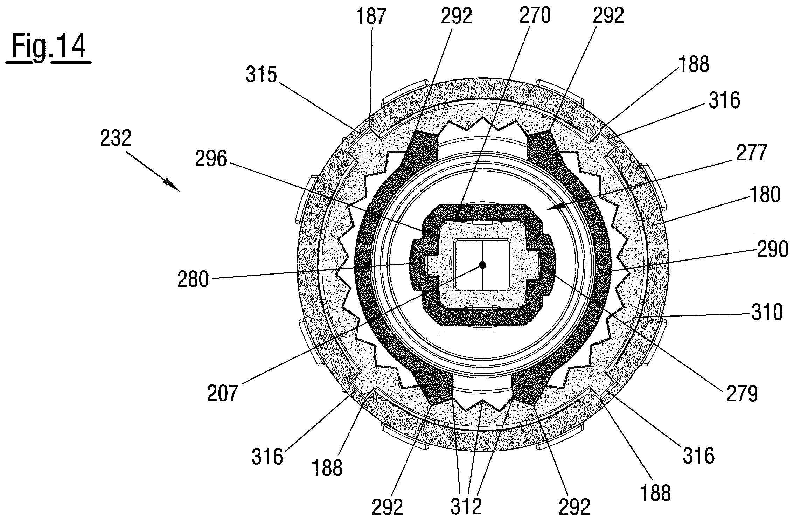

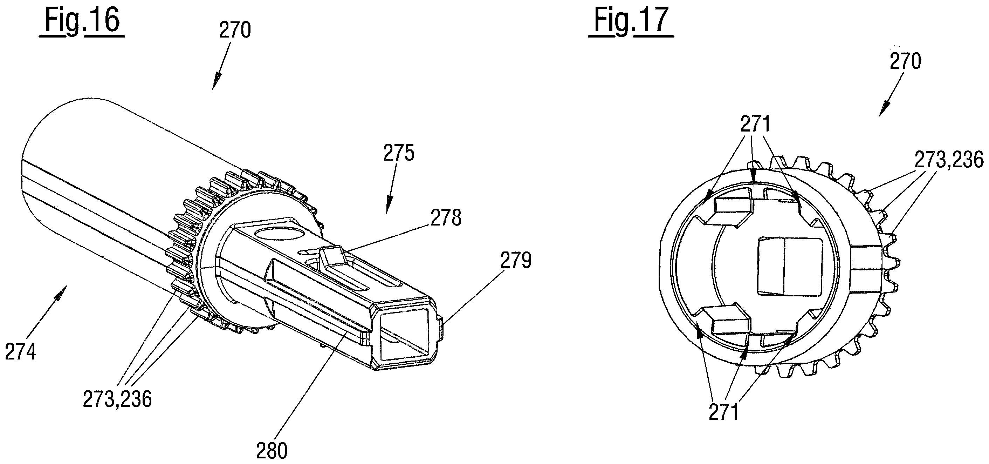

**Fig. 14** shows a radial cut through the dose definition mechanism 232 perpendicular to the longitudinal axis 207. **Fig. 15** shows a perspective view of a proximal side of the dose setting member 290 of the drug delivery device 200 and **Fig. 16** shows a perspective view of a distal side of the clutch member 270.

As can be seen from Fig. 14, the dose definition mechanism 232 defines an uneven number of discrete rotational positions of the dose setting member 290 with respect to the housing 210 that correspond to settable doses, namely 27 rotational positions/settable doses. To ensure correct rotational alignment between the first part 235 and the second part 236 of the clutch mechanism 234, the dose setting member 290 is connected to the clutch member 270 by a connection 277 having a coding feature that only allows a single relative rotational orientation between the clutch member 270 and the dose setting member 290.

The connection 277 comprises a non-circular, namely rectangular, opening 296 within the dose setting member 290, the opening 296 receiving the non-circular, namely rectangular, distal part 275 of the clutch member 270. The coding feature then comprises a first longitudinal ridge 279 and a second longitudinal ridge 280, whereby the longitudinal ridges 279, 280 radially extend from opposite sides of the distal part 275 of the clutch member 270. The first ridge 279 is received in a corresponding first longitudinal groove 297 located within the opening 296 of the dose setting member 290 and the second ridge 280 is received within a corresponding second longitudinal groove 298 of the dose setting member 290. The first ridge 279 and the first groove 297 have a different dimension, in particular a different width, that differs from the respective dimension, in particular width, of the second ridge 280 and the second groove 298. With other embodiments of the drug delivery device 200, the coding feature of the connection 277 could also be realized in a different way for example by ridges provided on the dose setting member 290 and corresponding grooves provided at the clutch member 270.

To permanently and non-releasably couple the dose setting member 290 to the clutch member 270 during assembly of the drug delivery device 200, the clutch member 270 is locked to the dose setting member 290 by a snap-fit connection 277. As can be seen, for example, in Fig. 15 and Fig. 16, this snap-fit connection 277 comprises two flexible snap hooks 278 that are located at opposing sides of the distal part 275 of the clutch member 270. Upon insertion of the distal part 275 into the opening 296 of the dose setting member 290, the snap hooks 278 engage with corresponding recesses 295 provided in the side surfaces of the opening 296. With other embodiments, the non-releasable connection 277 could also be provided in different ways, for example by at least one snap-hook located at the dose setting member 290 and at least one corresponding recess located on the clutch member 270.

As it will be described in further detail below, axial positions of the dosing member 330 that correspond to a minimum and a maximum settable dose are defined by interaction between the dosing member 330 and the inner housing 180. A connection between the dose selector member 310 and the inner housing 180 is therefore configured in a way that these axial positions correspond to settable doses defined by the dose definition mechanism 232.

With the drug delivery device 200, such a connection, which is shown in Fig. 14, is achieved by restricting a relative rotational orientation between the dose selector member 310 and the inner housing 180 to a single orientation. The connection is established by a first longitudinal ridge 315, which is provided on the outer surface of the dose selector member 310 and which is received in a corresponding first longitudinal groove 187 provided on an inner surface of the inner housing 180. The first longitudinal ridge 315 has a dimension, in particular a width, that is different than the corresponding dimension, in particular width, of at least one, in particular three, further longitudinal ridges 316 that are distributed over the remaining outer surface of the dose selector member 310. The further longitudinal ridges 316 engage with corresponding further longitudinal grooves 188 that are distributed over the remaining inner surface of the inner housing 180 and have corresponding widths that are different from the width of the first longitudinal groove 187.

In general, the first longitudinal ridge 315 and the first longitudinal groove 187 form a first longitudinal splined connection and the further longitudinal ridges 316 and the further longitudinal grooves 188 form at least a second longitudinal splined connection, the first longitudinal splined connection having a different dimension, in particular transverse width, than the second longitudinal splined connection. With other embodiments, the connection between the dose selector member 310 and the inner housing 180 could also be achieved in different ways, for example by splined connections having grooves located on the dose selector member 310 and ridges located on the inner housing 180.

**Fig. 17** shows a perspective view of a proximal side of the clutch member 270 of the drug delivery device 200. On the inner surface of its proximal part 274, the clutch member 270 has the longitudinal ridges 271 that engage with the longitudinal grooves 254 of the nut 250 to rotationally lock the clutch member 270 with respect to the nut 250 while at the same time allowing relative axial movement. Generally speaking, the longitudinal ridges 271 and the corresponding longitudinal grooves 254 form a splined connection between the clutch member 270 and the nut 250. With other embodiments, a rotationally fixed and axially movable connection between the clutch member 270 and the nut 250 could also be achieved by other means, for example, by longitudinal ridges provided on the nut 250 and corresponding grooves provided on the clutch member 270.

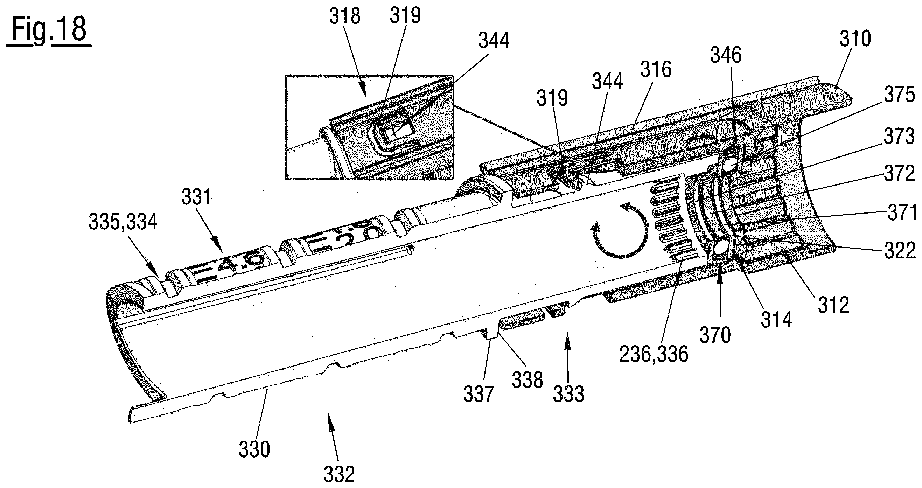

**Fig. 18** shows a longitudinal cut through the dosing member 330 and the dose selector member 310 of the drug delivery device 200. The drug delivery device 200 comprises a friction reduction mechanism that acts between the dosing member 330 and the dose selector member 310. The friction reduction mechanism is configured to reduce friction upon relative rotational movement between the dosing member 330 and the dose selector member 310.

The friction reduction mechanism comprises a ball bearing 370 which is provided between a distal surface 346 of the dosing member 330 and a contact surface 314 of the dose selector member 310. The contact surface 314 is thereby provided by the proximal front surface of the radial inner wall 322 of the dose selector member 310. The distal surface 346 generally is a distally facing surface of the dosing member 330. With the drug delivery device 200, the distal surface 346 is a distal end surface of the dosing member 330. With other embodiments, the distal surface 346 could also be located at a different position of the dosing member 330.

When increasing the dose during dose setting, a distally directed axial force is transferred from the dosing member 330 via the ball bearing 370 to the dose selector member 310. When pushing the dose selector member 310 in the proximal direction during injection, a proximally directed axial force is transferred from the dose selector member 310 via the ball bearing 370 to the dosing member 330.

The ball bearing 370 comprises several balls 375 that are sandwiched between a distal disc 371 touching the contact surface 314 of the dose selector member 310 and a proximal disc 372 contacting the distal surface 346 of the dosing member 330. Furthermore, the ball bearing 370 comprises a holder 372, which is sandwiched between the distal disc 371 and the proximal disc 372. The holder 372 surrounds the balls 375 in the radial direction and holds them into place.

The dose selector member 310 has a connection to the dosing member 330 that is configured to axially restrain movement between the dose selector member 310 and the dosing member 330 and to allow for relative rotation between the dose selector member 310 and the dosing member 330. Distal movement of the dose selector member 310 with respect to the dosing member 330 is prevented by a snap-fit connection. The snap-fit connection comprises a circumferential annular ridge 344 on an outer surface of the dosing member 330 and at least one, namely four, flexible members 319 formed on the dose selector member 310. When moving the dose selector member 310 in the proximal direction over the dosing member 330 during assembly, the flexible members 319 snap over the annular ridge 344 and engage with a proximal front surface of the annular ridge 344. With other embodiments, distal movement of the dose selector member 310 may also be achieved by a different connection, for example, by flexible members of the dosing member 330 engaging with an annular ridge of the dose selector member 310. Proximal movement of the dose selector member 310 with respect to the dosing member 330 is prevented by the contact surface 314 of the dose selector member 310 resting via the ball bearing 370 against the distal end surface 346 of the dosing member 330.

With other embodiments of the drug delivery device 200, the bearing element 370 could also be configured in other ways. For example, the bearing element 370 could also be configured as a disc bearing, such as a single annular disc made from a low-friction material, such as PTFE.

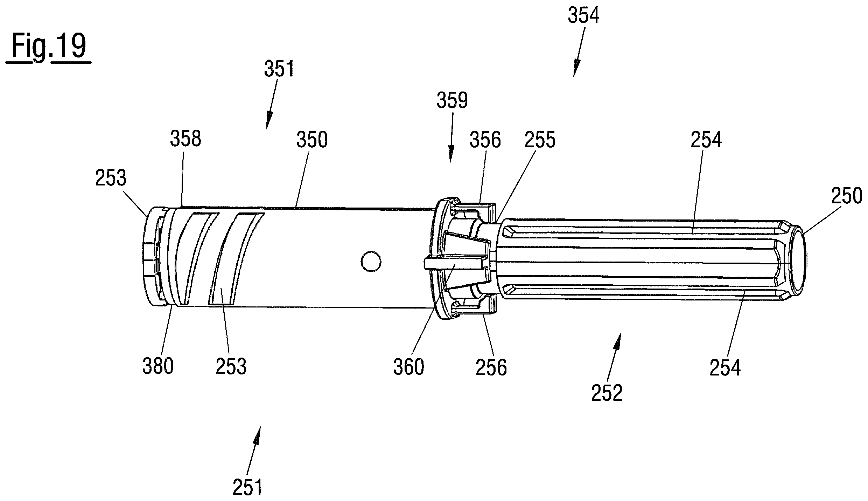

**Fig. 19** shows a perspective view of a connection 354 between the nut 250 and the driver 350 of the drug delivery device 200. The connection 354 is configured to axially restrain the driver 350 with respect to the nut 250 and to allow relative rotational movement between the nut 250 and the driver 350.

The connection 354 comprises two flexible arms 356 that are formed at a distal end of the driver 350 and that radially protrude inwardly to engage with an annular detent 255 between the proximal and distal parts 251, 252 of the nut 250. When moving the driver 350 distally with respect to the nut 250, the flexible arms 356 abut against the distal side surface of the annular detent 255. A clearance is provided between the distal side surface and the flexible arm 356 to allow the nut 250 and the driver 350 to travel different distances into the distal direction during dose setting.

The drug delivery device 200 comprises a further friction reduction mechanism that is configured to reduce friction between the nut 250 and the driver 350 upon relative rotational movement with respect to each other during dose delivery. The further friction reduction mechanism comprises a bearing element 380 that is positioned between the driver 350 and the nut 250.

The bearing element 380 is located between a proximal front surface 358 of the driver 350 and a protrusion 253 located at the proximal end of the nut 250. The proximal protrusion 253 defines a rim that radially extends from the nut 250. When rotating into the inner sleeve 183 of the inner housing 180 during dose delivery, the proximal front surface 358 of the driver 350 pushes via the further bearing element 380 against the protrusion 253 and thereby also pushes the nut 250 in the proximal direction.

The bearing element 380 is configured as a bearing disc made from a low-friction material, such as PTFE. With other embodiments, the bearing element 380 could also be configured as a different type of bearing, for example as a ball bearing.

With the drug delivery device 200, the driver 350 is in general configured to axially advance the nut 250 during dose delivery by indirectly transferring an axial force to the nut 250, that is by transferring the axial force to the nut 250 via one or more intermediate members, namely the bearing element 380.

The piston rod 240 is rotationally fixed with respect to the housing 210 at least during dose delivery and the nut 350 and the piston rod 240 are rotationally fixed with respect to each other during dose delivery so that the threaded connection 241, 256 between the nut 250 and the piston rod 240 axially locks the nut 250 with respect to the piston rod 240 during dose delivery. Therefore, the nut 250 and the piston rod 240 are configured to simultaneously move axially during dose delivery as if they were a single member.

During dose setting, the nut 250 is configured to rotate with respect to the piston rod 240. Thereby, the piston rod 240 is rotationally locked to the housing 210 also during dose setting and the nut 250 is configured to rotated with respect to the housing 210 during dose setting. Rotation of the nut 250 then axially advances the nut 250 with respect to the piston rod 240 during dose setting due to the threaded connection 241, 256 between nut 250 and piston rod 240. Axial advancement of the nut 250 with respect to the piston rod 240 and/or with respect to the housing 210 then also defines the axial advancement of the piston rod 240 with respect to the housing 210 during dose delivery.

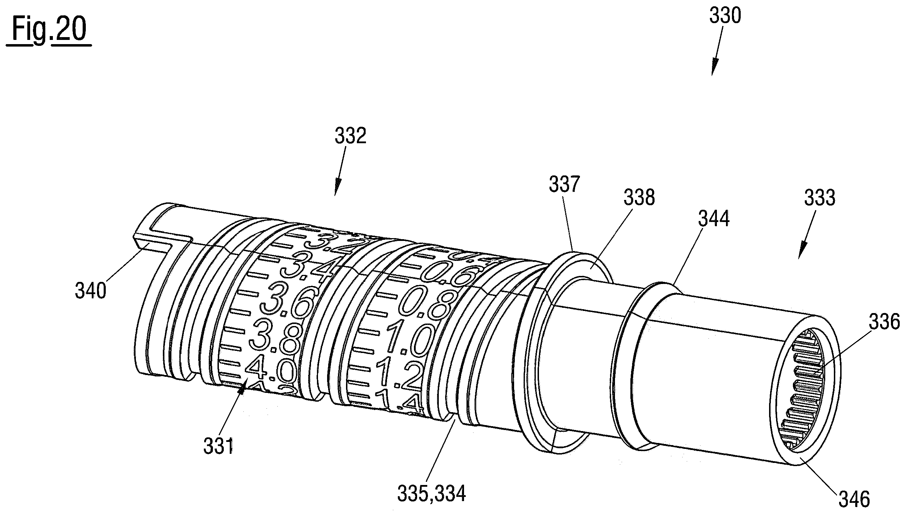

**Fig. 20** shows a perspective view of the dosing member 330 of the drug delivery device 200. The dosing member 330 comprises a maximum dose stop 337 that is configured to engage with the inner housing 180 upon setting a maximum dose. Engagement of the maximum dose stop 337 with the inner housing 180 thereby limits further axial movement of the dosing member 330 in the distal direction and defines the axial and rotational position of the dosing member 330 that corresponds to the maximum dose settable by the dosing mechanism 230.

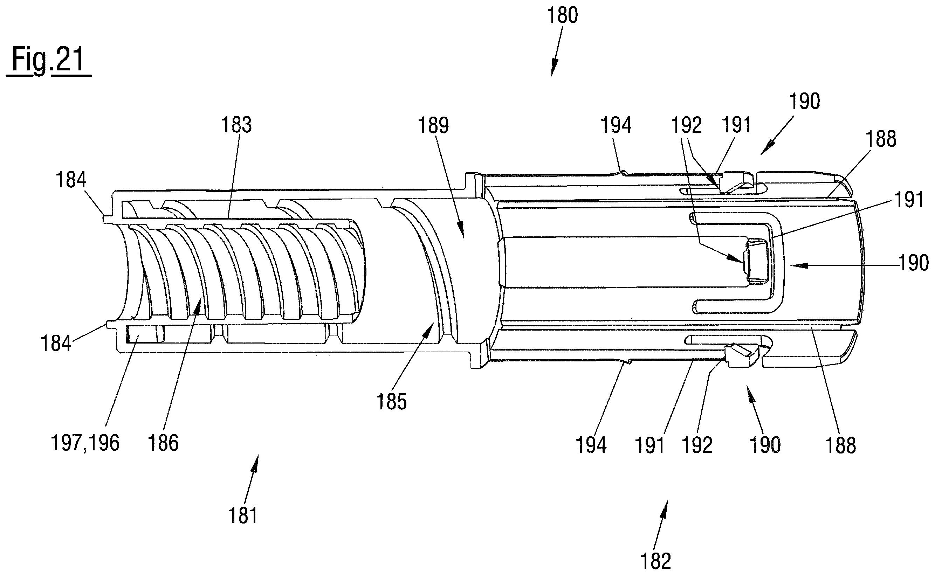

As can be seen from **Fig. 21****,** which shows the inner housing 180 in a longitudinal cut through the longitudinal axis 207, the inner housing 180 comprises at least one maximum stop feature 190, namely four maximum stop features 190. The maximum stop features 190 are formed as integral parts of the inner housing 180. They each comprise a flexible hook 191 that radially protrudes inwardly into a housing cavity 189 of the inner housing 180 that receives the dosing member 330. The flexible hooks 191 each comprise a limiting surface 192 that is orientated perpendicular to the longitudinal axis 207 and faces into the proximal direction.

Upon insertion of the dosing member 330 into the housing cavity 189, the flexible hooks 191 snap over the maximum dose stop 337 to subsequently limit axial movement of the dosing member 330 into the distal direction. When setting the maximum dose, a distal stopping surface 338 of the maximum dose stop 337 abuts against the limiting surfaces 192 of the maximum stop features 190. The distal stopping surface 338 is configured as a side surface of the maximum dose stop 337 and is orientated perpendicular to the longitudinal axis 207.

As can be seen from Fig. 20, the dosing member 330 also comprises a zero dose stop 340 that defines the rotational and axial position of the dosing member 330 that corresponds to a zero dose or no set dose. The zero dose stop 340 is located at the proximal end of the dosing member 330. It is configured as a limiting surface that is orientated parallel to the longitudinal axis 207. The limiting surface forms a side surface of a cut-out at the proximal end of the dosing member 330.

When reaching the zero-dose position, the zero dose stop 340 engages with a zero stop feature 196 of the inner housing 180, which is shown in Fig. 21. The zero stop feature 196 is located at the proximal end of the housing cavity 189. Like the zero dose stop 340, the zero stop feature 196 is also configured as a limiting surface 197 that is orientated parallel to the longitudinal axis 207. Furthermore, the limiting surface 197 of the zero stop feature 196 is orientated parallel to the limiting surface of the zero dose stop 340.

The zero dose stop 340 engages with the zero stop feature 196 in a contact plane that is angled with respect to a radial plane orientated perpendicular to the longitudinal axis 207. With the present embodiment, the contact plane is orientated perpendicular to the radial plane and parallel to the limiting surfaces 197 that are provided by the zero dose stop 340 and the zero stop feature 196. The limiting surface 197 of the zero stop feature 196 provided at the housing of the device 200 thereby coincides with the contact plane.

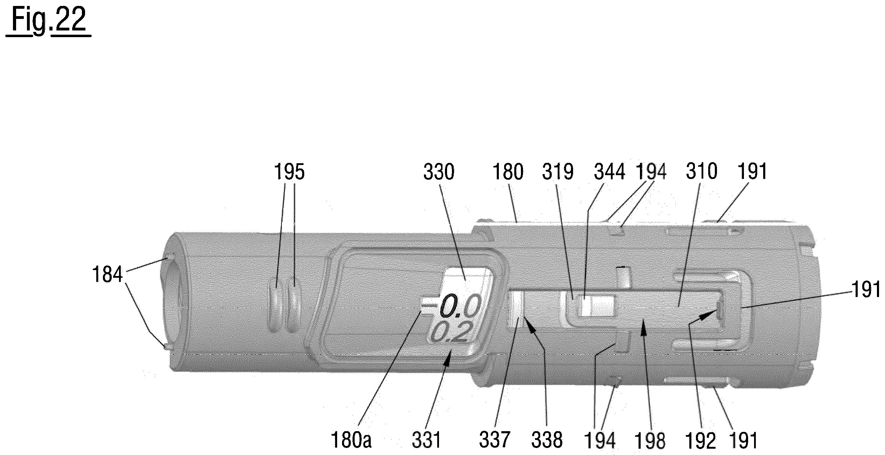

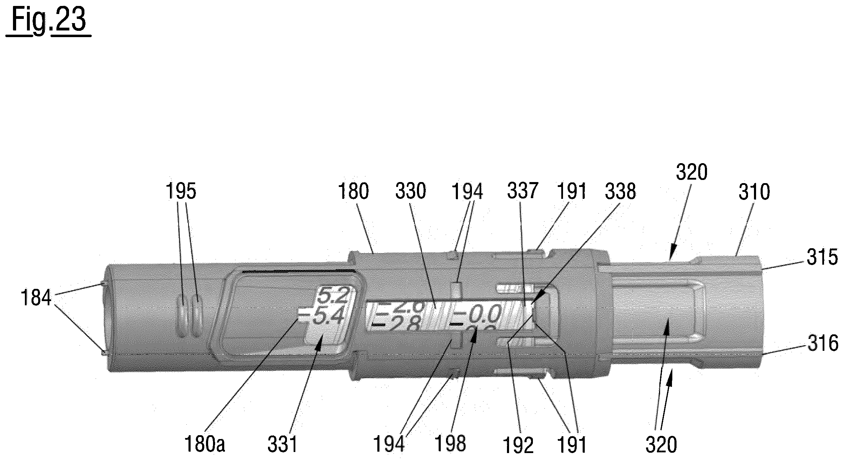

**Fig. 22** shows a perspective view of the inner housing 180 with the dosing member 330 in the zero-dose position and **Fig. 23** shows a perspective view of the inner housing 180 with the dosing member 330 in a maximum dose position.

The dosing member 330 is configured to perform two full rotations about the longitudinal axis 207 when moving from the zero-dose position to the maximum dose position. In the zero-dose position, a minimum dose marker is visible in the window 188a of the inner housing 180 indicating a set dose of 0.0, and in the maximum dose position a maximum dose marker is visible in the window 188a indicating a set dose of 5.4.

With other embodiments of the drug delivery device 200, the dosing member 330 may be configured to perform less or more than two full rotations about the longitudinal axis 207 when moving from the zero-dose position to the maximum dose position. In particular, the drug delivery device 200 may be configured to perform a non-integer rotation that deviates from a full rotation or an integer multiple of a full rotation. Likewise, the maximum dose marker may indicate any other dose that deviates from a set dose of 5.4, for example a set dose of 1.8 or 3.6.

The inwardly protruding maximum stop features 190 of the inner housing 180 are located inside longitudinal detents 320 of the dose selector member 310. This allows the limiting surfaces 192 to engage with the stopping surface 338 of the dosing member 330 despite the dose selector member 310 surrounding the dosing member 330 in its distal part 333.

The inner housing 180 is both axially and rotationally locked with respect to the outer housing 211. As can be seen from Fig. 22 and Fig. 23, the inner housing 180 comprises protrusions 194 that are circumferentially distributed around the outer surface of the distal part 182 of the inner housing 180. Furthermore, the inner housing 180 comprises radial protrusions 195 that are located on the outer surface of the proximal part 181 of the inner housing 180. With the embodiments shown in Fig. 22 and Fig. 23, two radial protrusions 195 are placed next to each other parallel to the longitudinal axis 207. The two protrusions 195 are both placed at the same circumferential position on the outer surface of the inner housing 180.

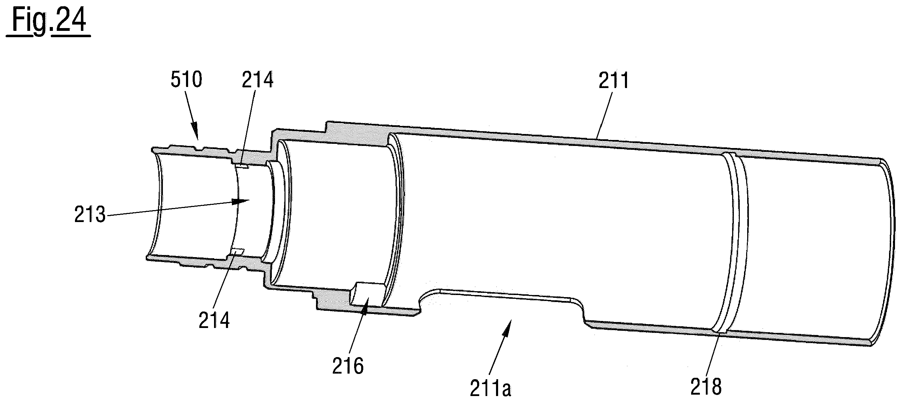

As can be seen from Fig. 24, which shows a longitudinal cut through the outer housing 211 of the drug delivery device 200, the outer housing 211 comprises, on its inner surface, a circumferential groove 218, which is located in the distal part of the outer housing 211. Furthermore, the outer housing 211 comprises a detent 216 in a proximal part of its inner surface.

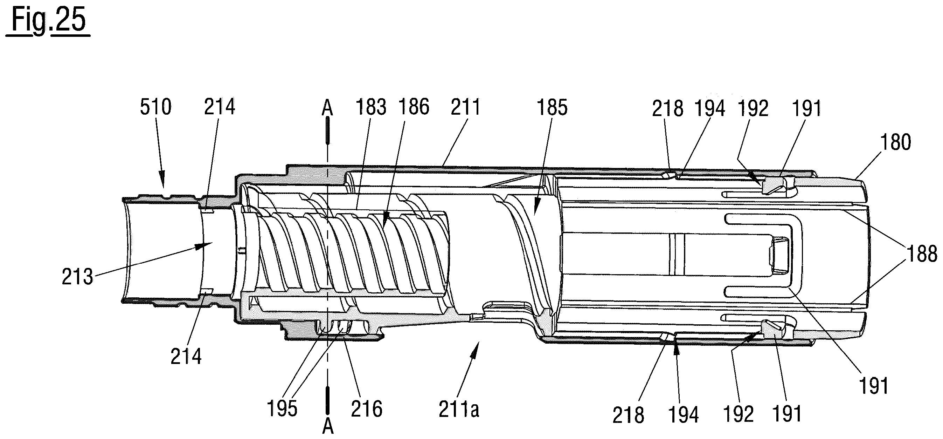

Fig. 25 shows longitudinal cut of the inner housing 180 mounted within the outer housing 211 of the drug delivery device 200. The protrusions 194 in the distal part 182 of the inner housing 180 are configured to prevent axial movement of the inner housing 180 with respect to the outer housing 211 in the distal direction. They snap into the circumferential groove 218 when mounting the inner housing 180 inside the outer housing 211 by inserting the inner housing 180 into the outer housing 211 from its distal end. When pushing the inner housing 180 in the distal direction after full insertion, the protrusions 194 engage with the distal end surface of the circumferential groove 218 and thereby prevent axial movement. In the proximal direction, the inner housing 180 abuts against a step within the inner surface of the outer housing 211, which step is limiting proximal movement of the inner housing.

With other embodiments of the drug delivery device 200, axial movement of the inner housing 180 with respect to the other housing 211 may also be prevented by other means. For example, the outer housing 211 may comprise flexible elements that engage with grooves positioned on the outer surface of the inner housing 180.

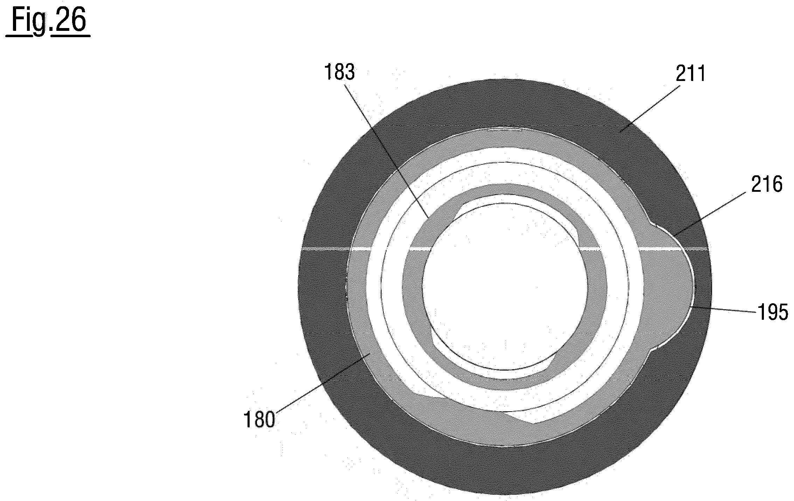

The radial protrusions 195 in the proximal part of the inner housing 180 are configured to prevent rotational movement of the inner housing 180 with respect to the outer housing 211. They engage with the detent 216 in the proximal part of the inner surface of the outer housing 211. This is further illustrated in **Fig. 26****,** which shows a radial cut through the outer and inner housing 211, 180 of the drug delivery device 200 through the line A-A shown in Fig. 25. With other embodiments of the drug delivery device 200, rotational movement of the inner housing 180 with respect to the other housing 211 may also be prevented by other means. For example, the outer housing 211 may comprise protrusions that engage with detents positioned on the outer surface of the inner housing 180.

Upon assembly of the drug delivery device 200, the dose selector member 310 and the dosing member 330 are first assembled to each other and inserted into the inner housing 180. The inner housing 180 is only then inserted into the outer housing 211. After insertion into the outer housing 211, the flexible hooks 191 rest against the inner surface of the outer housing 211 thus preventing outward bending of the flexible hooks 191. This prevents disengagement of the hooks 191 from the maximum dose stop 337 upon setting the maximum dose.

With all drug delivery devices according to the present disclosure, the design of the respective maximum and zero dose stops 337, 340 is generally independent of the design of the remaining device, in particular of the details of the rotational coupling between a respective dose setting member and a respective dose sleeve, of respective clutch mechanisms, dose definition mechanisms, resetting mechanisms or the like.

The drug delivery device 200 is configured to deliver a multitude of individual doses from the cartridge 8 attached to the device 200 via the cartridge holder 412. Furthermore, the drug delivery device 200 is configured as a reusable drug delivery device, which allows a user to replace an empty cartridge 8 by a new cartridge 8 after the last dose has been delivered from a given cartridge 8.

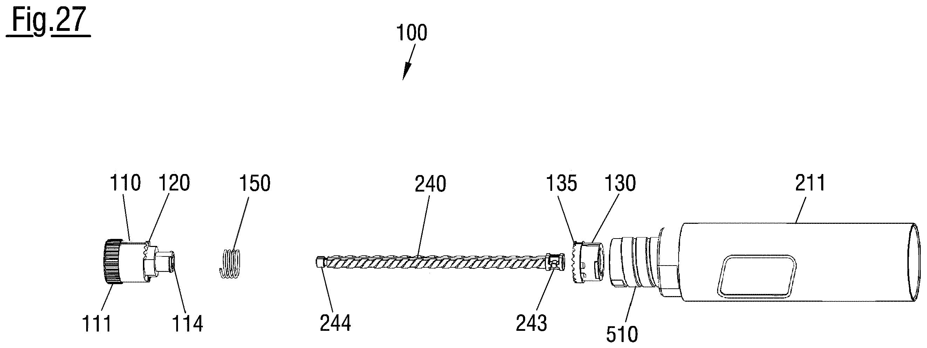

The resetting mechanism 100, which is shown in an exploded partial view in **Fig. 27****,** thereby allows to move the piston rod 240 back into the housing 210 after delivery of the last dose and disengagement of the cartridge holder 412 from the housing 210.

The resetting element 110 of the resetting mechanism 100, which guides the piston rod 240 in the non-circular opening 114, is mounted to the housing 210, namely the outer housing 211. Connection between the resetting element 110 and the housing 210 is achieved by a coupling part 130, which is both rotationally and axially fixed with respect to the housing 210. The coupling part 130 is configured as an insert received within the housing 210, namely within the outer housing 211.

According to the present disclosure, the housing 210 comprises all members that are permanently rotationally and axially fixed with respect to the outer housing 211 during intended use of the drug delivery device 200. As such, the coupling part 130 may also be considered as being part of the housing 210. With other embodiments of the drug delivery device 200, the coupling part 130 may be configured as an integral part of the housing 210.

A biasing element 150, which is configured as a compression spring, is mounted between the coupling part 130 and the resetting element 110 and therefore also between the housing 210 and the resetting element 110. The biasing element 150 biases the resetting element 110 in the proximal direction into a proximal position with respect to the housing 210 and the coupling part 130.

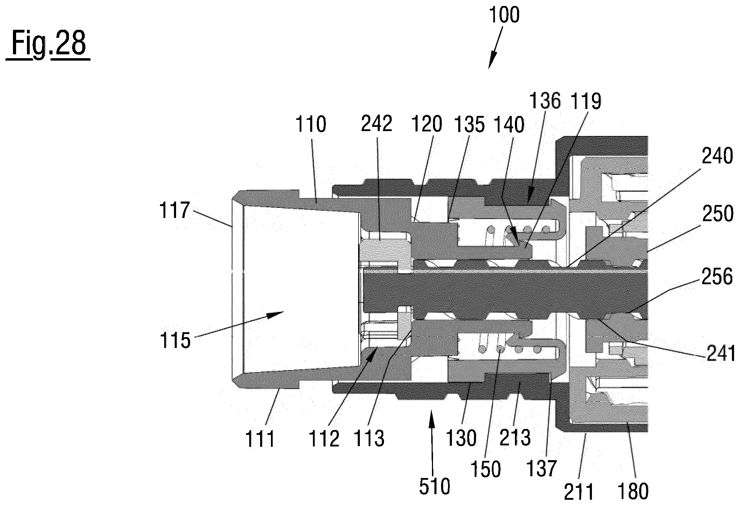

**Fig. 28** shows a longitudinal cut through the resetting mechanism 100 of the drug delivery device 200 with the resetting element 110 in the proximal position. In this configuration, the resetting element 110 is rotationally movable with respect to the housing 210. The resetting element 110 comprises a gripping zone 111 at its proximal end, which may be gripped by the user of the device 200 to rotate the resetting element 110. Within the gripping zone 111, the resetting element 110 as a rough outer surface, such as an undulated outer surface.

Due to the rotationally fixed connection between the resetting element 110 and the piston rod 240, the piston rod 240 is forced to rotate together with the resetting element 110 when the user rotates the resetting element 110. Engagement between the thread 241 of the piston rod 240 and the thread 256 of the nut 250 then forces the piston rod 240 to travel into the distal direction back into the housing 210 upon rotating the resetting element 110 in a resetting direction. In this way, the resetting element 110 is configured to move the piston rod 240 back into the housing 210 upon rotation by the user.

In general, the piston rod 240 is threadedly engaged with a member of the dose setting mechanism 230, namely with the nut 250, and the resetting element 110 is rotated with respect to this member during resetting the piston rod 240. As it is exemplarily the case for the drug delivery device 200, said member may be rotationally and/or axially fixed with respect to the housing 210 of the device 200 and the resetting element 110 may be configured to be rotated with respect to the housing 210 upon resetting the piston rod 240.

Furthermore, the piston rod 250 generally is rotationally fixed with respect to the resetting element 110 and axially movable with respect to the resetting element 110 at least during the resetting operation. With the drug delivery device 200, the piston rod 250 is permanently rotationally fixed with respect to the resetting element 110. Furthermore, it is permanently axially movable with respect to the resetting element 110.

After disengagement of the cartridge holder 412 from the housing 210, the piston rod 240 is accessible to a user of the device 200. The connection 354 that axially restrains the driver 350 with respect to the nut 250 serves to prevent unwanted movement of the piston rod 240 that could be caused by the piston rod 240 being directly pushed or pulled by the user without simultaneous rotation of the resetting element 110.

For example, if the user sets a dose while the cartridge holder 412 is detached from the housing 210, the nut 250 and the driver 350 move together in the distal direction. Without the connection 354, the nut 250 would not be prevented from moving proximally again if a user then pulls the piston rod 240 and the user would be able to pull the piston rod 240 out of the housing 210. This could lead to the impression that the device 200 is broken.

With the connection 354, pulling the piston rod 240 out of the housing 210 by the user without simultaneous rotation of the piston rod 240 is prevented. Axial movement of the piston rod 240 without rotation would namely require the nut 250 to move axially. Due to the connection 354 between the nut 250 and the driver 350 and due to the threaded connection 352 between the driver 350 and the inner housing 180, the driver 350 would also have to move axially and rotate with respect to the housing 210. However, due to the gearing or mechanical advantage that is caused by the different pitches of the threaded connection 352 between the driver 350 and the inner housing 180 and of the threaded connection 334 between the dosing member 330 and the inner housing 180, the forces that a user is typically able to exert by pulling or pushing the piston rod 240 are not large enough to overcome the resistance required to cause a rotation of the dosing member 330, the clutch member 270 and the dose setting member 290 by directly forcing the driver 350 to rotate. Therefore, the driver 350 and, via the connection 354, also the nut 250 are essentially rotationally and axially locked when the dose setting member 290 is not being actuated.

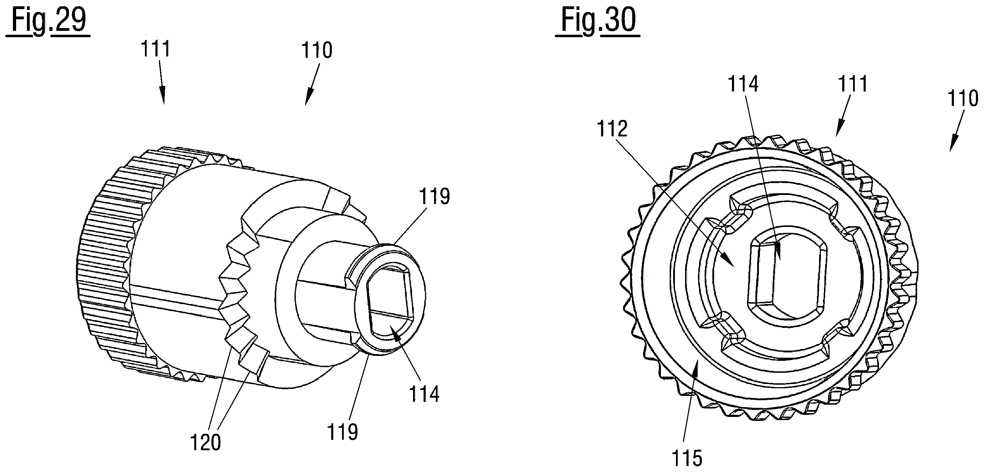

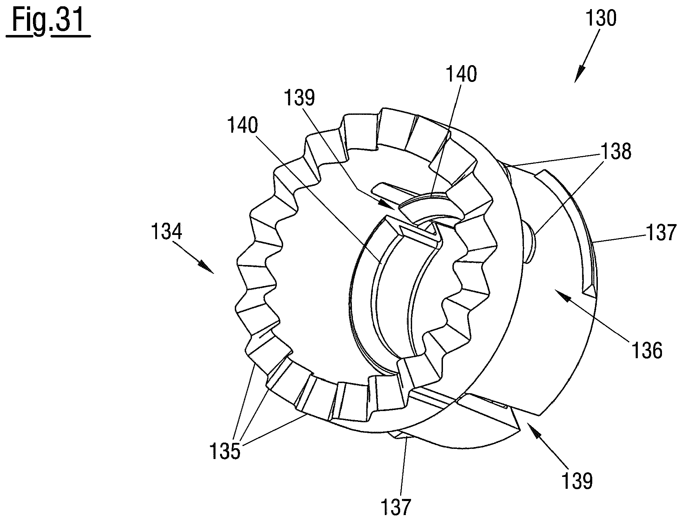

**Fig. 29** shows a distal perspective view of the resetting element 110, **Fig. 30** shows a proximal perspective view of the resetting element 110 and **Fig. 31** shows a proximal perspective view of the coupling part 130 of the resetting mechanism 110.

As can be seen from Fig. 28, a distal part of the resetting element 110 is received within the coupling part 130. In the proximal position shown in Fig. 28, further proximal movement of the resetting element 110 under the action of the biasing member 150 within the coupling part 130 is prevented by the resetting element 110 engaging with the coupling part 130. Thereby, a radial stop 119 located at the distal end of the resetting element 110 engages with a corresponding stop feature 140 on an inner surface of the coupling part 130. With other embodiments, further proximal movement of the resetting element 110 may also be prevented in other ways.

As can also be seen from Fig. 28, the coupling part 130 is axially locked with respect to the housing 210 by an annular notch 136 that is located on the outer surface of the coupling part 130, whereby the annular notch 136 is received in a corresponding collar 213 on an inner surface of the outer housing 211. The notch 136 is distally limited by a locking structure 137 that radially protrudes from the outer surface of the coupling part 130. Upon inserting the coupling part 130 into the outer housing 211 in the distal direction, the locking structure 137 flexes radially inwardly and snaps over the annular collar 213 of the outer housing 211. In this way, the coupling part 130 is axially fixed with respect to the housing 210 by a snap-fit connection. With other embodiments, axial movement between the coupling part 130 and the housing 210 could also be prevented with other means, for example by a notch located on the housing 210 and a collar or protrusion located at the coupling part 130.

To rotationally lock the coupling part 130 with respect to the housing 210, the coupling part 130 comprises protrusions 138 that are located within the notch 136. The protrusions 138 engage with corresponding detents 214 in the annular collar 213. These detents 214 are shown, inter alia, in Fig. 24. With other embodiments, rotation between the coupling part 130 and the housing 210 could also be prevented by other means, for example by protrusions provided at the housing 210 and corresponding detents provided at the coupling part 130.

The locking structure 137 of the coupling part 130 comprises two portions that are separated by longitudinal slots 139. This allows the portions of the locking structure 137 to radially bend inwardly when mounting the coupling part 130 to the outer housing 211. After mounting the coupling part 130 and after mounting the inner housing 180 to the outer housing 211, the portions of the locking structure 137 are prevented from bending inwardly by engagement with the inner housing 180. When assembling the device 200, the coupling part 130 and the resetting element 110 are first snapped to the outer housing 211 and only then the inner housing 180 is inserted into the outer housing 211.

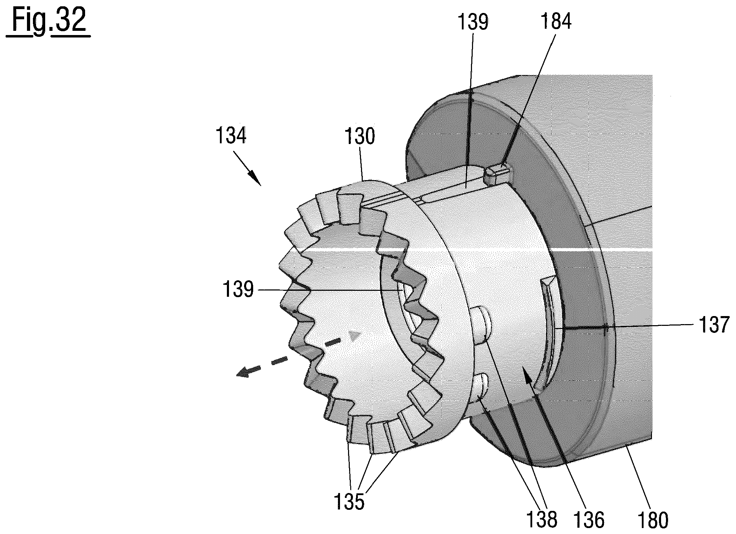

**Fig. 32** shows a perspective view of the coupling part 130 and the inner housing 180. The inner housing 180 comprises at its front surface two longitudinally protruding tappets 184, which are also visible, for example, in Fig. 23. The tappets 184 are received within the longitudinal slots 139 and thereby block the portions of the locking structure 137 from radially bending inwardly.

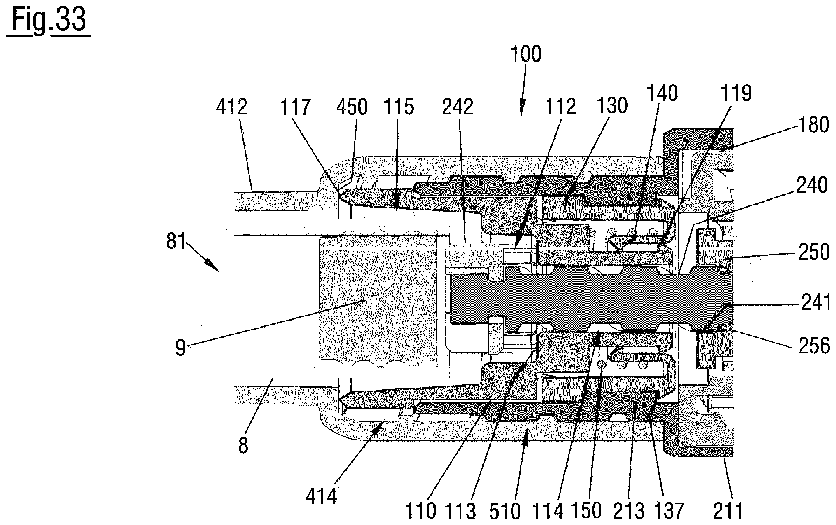

**Fig. 33** shows a longitudinal cut through the resetting mechanism 100 with the dispensing unit 410 attached the drug delivery device 200. When attaching the dispensing unit 410, the inner thread of the connection means 414 of the dispensing unit 410 is screwed onto the outer thread of the connection means 510 of the outer housing 211 until the distal end of the cartridge holder 412 rests against a step formed on the outer surface of the outer housing 211.

During mounting of the dispensing unit 410, the resetting element 110 is moved into the distal direction into its distal position to rotationally lock the resetting element 110 with respect to the housing 210. When being in its distal position, engagement features 120 of the resetting element 110 engage with corresponding engagement features 135 of the coupling part 130 and thereby rotationally lock the resetting element 110 with respect to the coupling part 130 and the housing 210.

The engagement feature 120 of the resetting element 110 are configured as distally facing teeth. The engagement features 135 of the coupling part 130 are located at a coupling site, which is formed by a front surface of the coupling part 130. The engagement features 135 are configured as proximally facing teeth that match between the distally facing teeth of the engagement feature 120 of the resetting element 110.

In the embodiment shown in Fig. 27 to Fig. 33 the engagement features 120, 135 are configured as symmetric teeth that have circumferential side surfaces that have the same slope. With other embodiments, the teeth of the engagement features 120, 135 may also be configured as asymmetric teeth. For example, the asymmetric teeth may have circumferential side surfaces with different slopes. Thereby, one side surface of the individual teeth may be orientated, for example, parallel to the longitudinal axis 207 and the respective other side surface may be inclined with respect to the longitudinal axis 207. Such asymmetric teeth may, for example, provide a saw-tooth profile.

With asymmetric engagement features 120, 135, side surfaces of the individual engagement features 120, 135 having a steeper slope than the respective other side surfaces may be configured to press against each other when the resetting element 110 is rotated in a circumferential direction that would screw the piston rod 240 back into the housing 210. This efficiently prevents a counter-rotation of the piston rod 240 with respect to the nut 250 during dose delivery or when overturning the dose setting member 290 and the nut 250 after the thread 256 of the nut 250 has engaged the stop feature 243 of the piston rod 240 upon increasing the dose during dose setting.

As can be seen from Fig. 33, the cartridge holder 412 of the dispensing unit 410 directly engages with the resetting element 110 to push the resetting element 110 into the distal direction upon mounting the dispensing unit 410 onto the housing 210. Thereby, a proximally facing contact structure 117 of the resetting element 110 rests against a distally facing contact feature 450 of the cartridge holder 412. The proximally facing contact structure 117 is exemplarily configured as a proximal circumferential edge of the resetting member 110. The distally facing contact feature 450 is exemplarily provided as a distally facing annular surface located at an inwardly protruding step of the cartridge holder 412.

The proximal position of the resetting element 110 is a resetting position of the resetting element 110 and the distal position of the resetting element 110 is a locking position of the resetting element 110. A locking distance between the resetting position and the locking position may, for example, be smaller than 2mm, 1.5mm, 1.25mm, 1.1mm or 1mm and/or larger than 0.5mm, 0.7mm or 0.8mm. It may, for example, amount to 0.8mm, 0.9mm, 1.0mm or 1.1mm.

With the cartridge holder 412 mounted to the housing 210, the cartridge 8 does not contact the resetting element 110. Therefore, the resetting element 110 is moved in the distal direction solely by its contact with the cartridge holder 412. The distal end of the cartridge 8 is received inside a cartridge cavity 115 of the resetting element 110, the cartridge cavity 115 being accessible from the proximal side of the resetting element 110.

The direct engagement between the cartridge holder 412 and the resetting element 110 allows, compared to an engagement between the cartridge 8 and the resetting element 110, to configure the engagement features 120, 135 with tighter axial tolerances and a smaller axial height. Typically, the individual cartridges 8 are made from glass and have larger variation in their longitudinal extent then individual cartridge holders 412, which are typically made from a plastic material. Therefore, the engagement features 120, 135 would have to have a comparably large axial height to provide a secure rotational locking between the resetting element 110 and the coupling part 130 irrespective of possible variations in the length of individual cartridges 8 due to manufacturing tolerances.

When being fully retracted into the housing 210, the plunger disc 242 of the piston rod 240 is located within a reception area 112 of the resetting element 110. The reception area 112 is configured as a further cavity that is accessible from the proximal side of the resetting element 110. Furthermore, the reception area 112 is located at and accessible from the distal end of the cartridge cavity 115. In its fully retracted position, the plunger disc 242 of the piston rod 240 rests against an inner surface 113 of the reception area 112. This inner surface 113 forms the distal end surface of the reception area 112 and surrounds the opening 114 of the resetting element 110 that guides the piston rod 240.

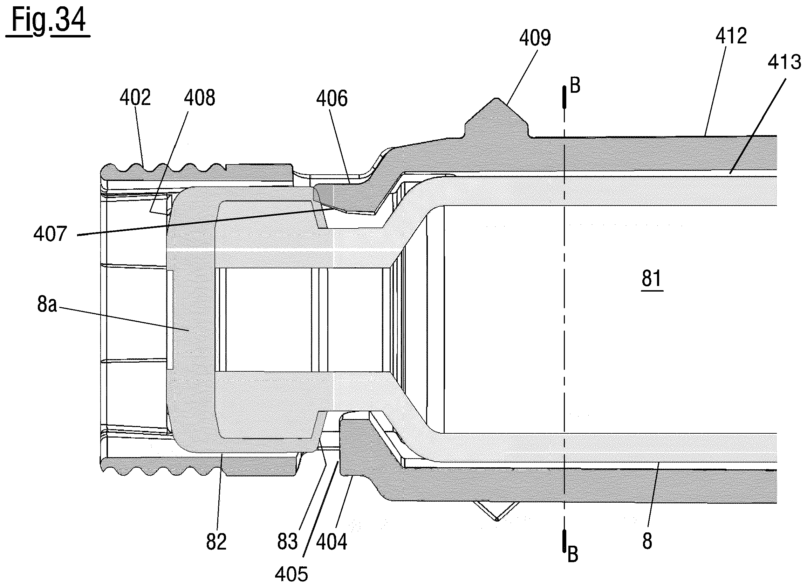

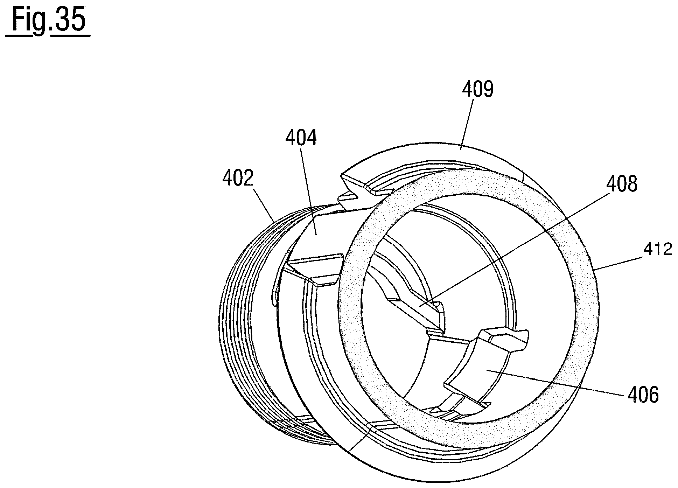

**Fig. 34** shows a longitudinal cut through a proximal end of the cartridge holder 412 attachable to the drug delivery device 200 with the cartridge 8 inserted into the cartridge holder 412. **Fig. 35** shows a perspective distal view of a radial cut through the proximal part of the cartridge holder 412 along the line B-B in Fig. 34. Inside the cartridge holder 412, the cartridge 8 is pushed against the stop 408 by a biasing element 406. The biasing element 406 engages with the distal surface 83 of the annular rim 82 of the cartridge 8. Thereby, it engages with the radially outer end of the distal surface 83. The biasing element 406 is configured as a flexible member that snaps over the annular rim 82 when the cartridge 8 is inserted into the cartridge holder 412. The biasing element 406 is configured as an integral part of the cartridge holder 412. The biasing element 406 is formed in a cut-out provided in an outer wall of the cartridge holder 412.

The outer wall of the cartridge holder 412 surrounds a cartridge cavity 413 that is configured to receive the cartridge 8. Both the biasing element 406 and the connector 404 radially protrude into the cartridge cavity 413. The cartridge cavity 413 has a longitudinal extent that is larger than the longitudinal extent of the cartridge 8. This prevents that a user of the dispensing unit 410 is able to touch or grip the cartridge 8 and remove it from the cartridge holder 412.

As can be seen from Fig. 34, the cartridge holder 412 comprises both the biasing element 406 and the connector 404, which are configured as separate elements of the cartridge holder 412. The biasing element 406 and the connector 404 are located at opposite sides of the cartridge holder 412 with respect to the longitudinal axis 207. Furthermore, both the biasing element 406 and the connector 404 are located at the same longitudinal position.

The biasing element 406 is thereby configured to bias, in particular permanently bias, the cartridge 8 in the proximal direction towards the stop 408. Thereby, the cartridge 8 is clamped between the stop 408 and the biasing element 406 so that both the stop 408 and the biasing element 406 simultaneously rest against the cartridge 8. The biasing element 406 prevents movement of the cartridge 8 within the cartridge holder 412. For example, the biasing element 406 biases the cartridge 8 in the proximal direction against the hollow cannula 6 when mounting the needle 4 to the cartridge holder 412.

The connector 404 generally constitutes a locking element that prevents removal of the cartridge 8 after insertion into the cartridge holder 412. Removal is thereby prevented by a contact surface 405 of the connector 404. The contact surface 405 is configured to engage with the cartridge 8 to prevent removal of the cartridge 8 from the cartridge holder 412. The contact surface 405 therefore acts as a blocking surface that prevents removal of the cartridge 8 from the cartridge holder 412. With the embodiments shown in Fig. 34, the contact surface 405 is provided by a proximal surface of the connector 404. The contact surface 405 is directed in the proximal direction. Furthermore, it is angled with respect to the longitudinal axis 207. It may be orientated generally perpendicular to the longitudinal axis 207, in particular, it may be orientated perpendicular to the longitudinal axis 207.

The contact surface 405 engages with a corresponding counter surface of the cartridge 8. The counter-surface of the cartridge 8 is a distally facing surface, which is angled with respect to the longitudinal axis 207. It also may be orientated generally perpendicular to the longitudinal axis 207, in particular, it may be orientated perpendicular to the longitudinal axis 207. The counter-surface is exemplarily provided by the distal surface 83 of the annular rim 82 of the cartridge 8.

The connector 404 is configured to be deflected towards the longitudinal axis 207 when the cartridge 8 engages with the connector404 upon attempted removal of the cartridge 8 from the cartridge holder 412. This further prevents removal of the cartridge 8 from the cartridge holder 412 by a locking the cartridge 8 inside the cartridge holder 412.

With the cartridge holder 412, the contact surface 405 has a larger angle with the longitudinal axis 207 than the counter surface 83. When contacting the counter surface 83 of the cartridge 8 upon distal movement of the cartridge 8, the connector404 is bent to orientate its contact surface 405 parallel to the counter surface 83. This deflects the connector404 radially towards the longitudinal axis 207 and towards the cartridge 8 when trying to remove the cartridge 8 from the cartridge holder 412.

When being fully inserted into the cartridge holder 412, the cartridge 8 is located away from the contact surface 405. The cartridge 8 then does not contact the contact surface 405. The action of the biasing element 406 biases the cartridge 8 into its fully inserted position.

A clamped end of the connector404 is connected to the body of the cartridge holder 412 and a free end of the connector404 is configured separate from the body of the cartridge holder 412. With the connector404, the free end is located at the proximal end of the connector 404 and the clamped end is located at the distal end of the connector 404. The connector404 is configured as a flexible member. Thereby, the free end of the connector404 may be radially deflected. Upon insertion of the cartridge 8 into the cartridge holder 412, the cartridge 8 first radially deflects the connector404 away from the longitudinal axis 207. Upon further movement of the cartridge 8 in the proximal direction, the connector 404 then snaps over the cartridge 8, namely over the annular rim 82 of the cartridge 8.

Likewise, a clamped end of the biasing element 406 is connected to the body of the cartridge holder 412 and a free end of the biasing element 406 is configured separate from the body of the cartridge holder 412. With the biasing element 406, the free end is located at the proximal end of the biasing element 406 and the clamped end is located at the distal end of the biasing element 406. The biasing element 406 is configured as a flexible member. Thereby, the free end of the biasing element 406 may be radially deflected. Upon insertion of the cartridge 8 into the cartridge holder 412, the cartridge 8 first radially deflects the biasing element 406 away from the longitudinal axis 207. Upon further movement of the cartridge 8 in the proximal direction, the biasing element 406 then snaps over the cartridge 8, namely over the annular rim 82 of the cartridge 8.

With the cartridge holder 412, a contact surface 407 of the biasing element 406 is configured to rest upon the cartridge 8 to exert the biasing force in the proximal direction. This contact surface 407 has an angle with the longitudinal axis 207 that is smaller than an angle of the contact surface 405 of the connector 404 with the longitudinal axis 207.

The biasing element 406 is configured to radially bend away from the longitudinal axis 207 and the cartridge 8 upon attempted removal of the cartridge 8 from the cartridge holder 412. With the cartridge holder 412, the contact surface 407 of the biasing element 406 has a larger angle with the longitudinal axis 207 than the counter surface 83 of the cartridge 8.

The cartridge holder 412 serves two functions. Firstly, it prevents the user from removing the cartridge 8 from the cartridge holder 412 without using tools. Secondly, it prevents the cartridge 8 from moving axially when the user attaches the needle 4 to the needle connector 402.

The first function is achieved by the connector 404 that safely snaps in after insertion of the cartridge 8. This is exemplarily achieved by the connector 404 having some space to the distal surface 83 of the cartridge 8 after insertion. The distance between the stop 408 and the connector 404 is adapted to accommodate varying thicknesses of the annular rim 82 of the cartridge 8. It thus is adapted to varying positions of the surface 83 from the stop 408. In general, the connector 404 is spaced apart from the surface 83 at least for cartridges 8 having an annular rim 8 that is axially shorter than the maximum thickness of cartridges 8 insertable into the cartridge holder 412. This allows the connector 404 to radially snap in even upon insertion a cartridge 8 having an axially long annular rim 82.

With the cartridge holder 412, the body of the cartridge 8 is not being held at its distal end (see Fig. 8). Without the biasing element 406, the cartridge 8 would be pushed in the proximal direction only by the plunger disc 242 which touches the piston 9 of the cartridge 8. When the user attaches a new needle 4, the cannula 6 pushes the cartridge 8 into the distal direction, which leads to the plunger disc 242 and the piston rod 240 pushing the piston 9 in the proximal direction with respect to the body of the cartridge 8. Once the cannula 6 penetrates the septum, the pressure on the piston 9 may lead to a loss of drug. To avoid this loss of drug, axial movement of the cartridge 8 upon mounting the needle 4 to the cartridge holder 412 and/or upon the cannula 6 penetrating the septum of the cartridge 8 is prevented by the biasing element 406.

The biasing element 406 is adapted to compensate for tolerances in the dimensions of the annular rim 82 of the cartridge 8, such as tolerances in its axial length and/or diameter. This is exemplarily achieved by the biasing element 406 being configured to rest against the cartridge 8 after full insertion and/or by the biasing element 406 being configured to radially bend outwards upon movement of the cartridge 8 into the distal direction after insertion.

The connector 404 does not bias the cartridge 8 in the proximal direction after insertion but allows for a bit of axial movement. The biasing element 406 is configured to exert a force on the cartridge 8 that prevents movement of the cartridge 8 during attachment of the needle 4. This force may act in addition to frictional forces acting on the cartridge 8 after insertion and/or attachment of the cartridge holder 412 to the drug delivery device 200. Thereby, the biasing element 406 does not completely inhibit distal axial movement of the cartridge 8 after insertion. For example, a user may still be able to move the cartridge 8 against the force of the biasing element 406.

With other embodiments, the drug delivery device 200 may be configured as a disposable device that has the cartridge holder 412 permanently and inseparably connected to the housing 210. Thereby, the cartridge holder 412 may not be disconnected from the housing 210 during intended use of the device 200 and/or without destroying the device 200. For example, these embodiments then may have a cartridge holder 412 that only features the biasing element 406 but not the connector 404.

The drug delivery device 200 is a reusable device that allows for detachment of a used cartridge holder 412 and reattachment of a new cartridge holder 412. As detailed below, the drug delivery device 200 furthermore is provided in different versions that are adapted to deliver drugs that differ at least in concentration. The different drugs are provided in cartridges 8 that are inserted into dedicated cartridge holders 412. Furthermore, the connection means 510 of the individual versions of the drug delivery device 200 and the connection means 412 of the individual versions of the cartridge holder 412 are configured as keyed connectors. Thereby, the connection means 510 of each individual version of the drug delivery device 200 only connect to the connection means 414 of the specific version of the cartridge holder 412 that holds the drug to be delivered by the respective drug delivery device 200 and does not connect to the connection means 414 of the other versions of the cartridge holder 412.

The connector 404 that prevents removal of the cartridge 8 from its cartridge holder 412 then increases safety during use of the devices 200 and cartridge holders 412. For example, if the user accidentally gets cartridge holders 412 for a version of the drug delivery device 200 that differ from the version used by the user, those cartridge holders 412 would contain the wrong drug and would not fit to the drug delivery device 200 of the user due to the keying feature of the connections means 414, 510. The connector 404 then prevents the user from removing the cartridges 8 holding the wrong drug from that version of the cartridge holder 412, inserting them into cartridge holders 412 adapted to the version of the drug delivery device 200 used by the user, and thus using the cartridges 8 with the wrong version of the drug delivery device 200 and/or using cartridges 8 holding the wrong drug.

Other embodiments of the cartridge holder 412 may only comprise the biasing element 406 and not the connector 404 or only the connector 404 and not the biasing element 406.

The proximal part of the cartridge holder 412 furthermore comprises an annular ridge 409 that radially extends from the outer surface of the cartridge holder 412. The annular ridge 409 is configured to be engaged by a flexible locking arm of the cap 209, which is provided on an inner surface of the cap 209. Engagement between the locking arm and the annular ridge 409 releasably locks the cap 209 to the drug delivery device 200 after attachment.

According to the present disclosure, the drug delivery device 200 may be part of a set of several drug delivery devices and the dispensing unit 410 may be part of a set of several dispensing units, whereby each drug delivery device comprises connection means that only allow attachment of a dedicated dispensing unit and prevents attachment of all other dispensing units of the set and vice versa. The connection means are thereby configured as keyed connection means, which provide a one-to-one assignment between the individual dispensing units and the individual drug delivery devices.

The set of drug delivery devices may comprise further variants of the drug delivery device 200 that have at least one mutual member that is identical among the drug delivery device 200 and the further variants. The set may also comprise different types of drug delivery devices that do not share such a mutual member with the drug delivery device 200.

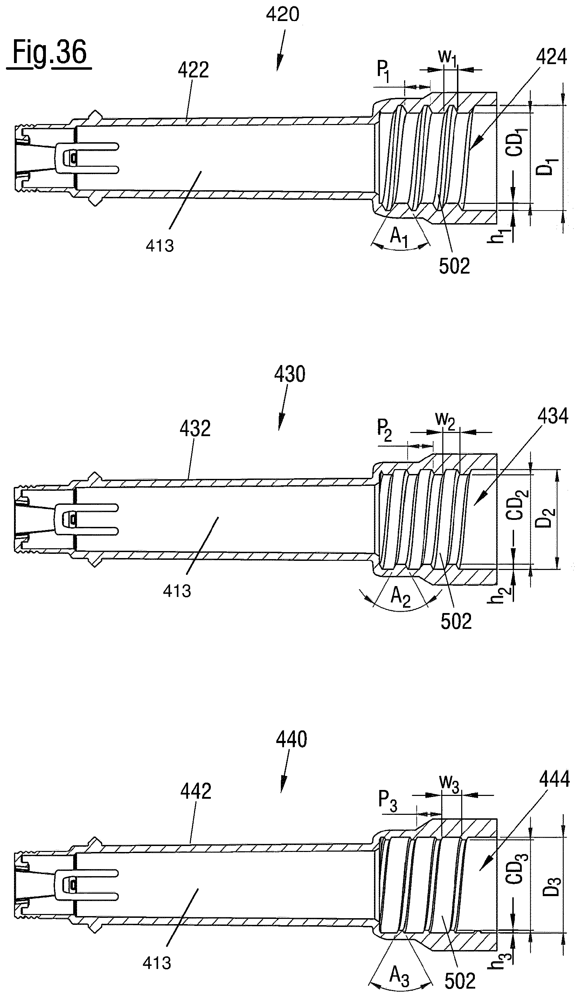

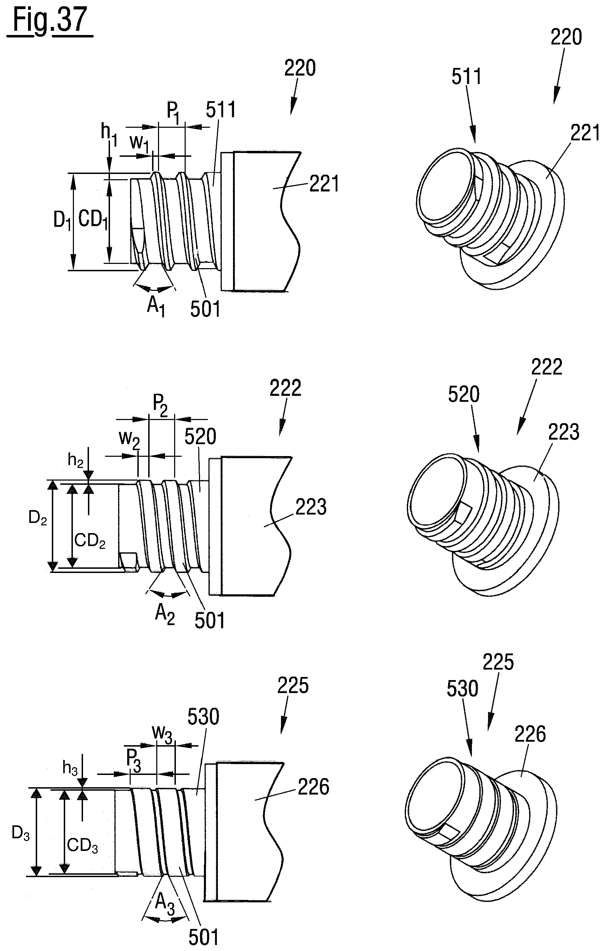

**Fig. 36** and **Fig. 37** show a set of three drug delivery devices and a set of three corresponding dispensing units according to the present disclosure. Each drug delivery device is connected to its corresponding dispensing unit by a keyed connection that prevents the respective drug delivery device from connecting to the other dispensing units and which, vice versa, prevents the corresponding dispensing unit from connecting to the other drug delivery devices.

Thereby, Fig. 36 shows a longitudinal cut through a first dispensing unit 420 attachable to a first housing 221 of a first drug delivery device 220 via first connection means 424 of a first cartridge holder 422, a longitudinal cut through a second dispensing unit 430 attachable to a second housing 223 of a second drug delivery device 222 via second connection means 434 of a second cartridge holder 432 of the second dispensing unit 430 and a longitudinal cut through a third dispensing unit 440 attachable to a third housing 226 of a third drug delivery device 225 via third connection means 444 of a third cartridge holder 442 of the third dispensing unit 440. Fig. 37 shows side views and perspective views of first connection means 511 of the first housing 221 of the first drug delivery device 220, of second connection means 520 of the second housing 223 of the second drug delivery device 222 and of third connection means 530 of the third housing 226 of the third drug delivery device 225.

The connection means 424, 434, 444 of the cartridge holders 422, 432, 442 and the corresponding connection means 511, 520, 530 of the drug delivery devices 220, 222, 225 form keyed connectors according to the present disclosure. Thereby, the connection means 424, 434, 444 are of the same type and the connection means 511, 520, 530 are also of the same type.

The individual connection means 424, 434, 444 of the cartridge holders 422, 432, 442 each form female parts of the connections and the individual connection means 511, 520, 530 of the drug delivery devices 220, 222, 225 form corresponding male parts. All connection means 424, 434, 444, 511, 520, 530 are configured as threads, whereby the connection means 424, 434, 444 of the cartridge holders 422, 432, 442 form inner threads and the connection means 511, 520, 530 of the drug delivery devices 220, 222, 225 form outer threads.

The geometries of the threads 424, 434, 444, 511, 520, 530 are defined by several thread dimensions. The thread dimensions comprise a core diameter or minor diameter that specifies the minimum inner diameter of the female part of the connections, an outer diameter or major diameter that specifies the maximum inner diameter of the female part of the connections, a pitch that specifies a distance between adjacent ridges 501 or valleys 502 of the threads, a width of the ridges 501 provided on the male part of the threads, which corresponds to a width of the valleys 502 provided on the female part of the threads, an opening angle between sidewalls of adjacent ridges 501 of the male parts, and aA height of the ridges 501 of the male parts and a corresponding height of the valleys 502 of the female parts that is given by the difference between the outer diameter and the core diameter.

Unless stated otherwise, the term "ridges" used in the present disclosure always refers to the ridges 501 of the male thread of a given threaded connection, irrespective of whether the part being described actually comprises a male thread or a female thread. These ridges may also be called crests of the threaded connections. The corresponding valleys on the female threads may also be called roots of the threaded connection.

Keying is achieved by at least one of the thread dimensions, such as at least one of the core diameter, the outer diameter, the pitch, the width of the ridges 501 and the opening angle, being mutually different among the individual pairs of corresponding connection means 424, 434, 444, 511, 520, 530 of the cartridge holders 422, 432, 442 and drug delivery devices 220, 222, 225.

With the embodiments shown in Fig. 36 and Fig. 37, the only thread dimensions that differ among the individual dispensing units 420, 430, 440 and therefore also among the individual drug delivery devices 220, 222, 225 are the width and the height of the individual ridges 501 of the male parts and the corresponding widths and heights of the valleys 502 of the female parts. Thereby, the ridges 501 of the first connection means 511 have a first width w₁, the ridges 501 of the second connection means 520 have a second width w₂, and the ridges 501 of the third connection means 530 have a third width w₃. The first width w₁ is smaller than the second width w₂, and the second width w₂ is smaller than the third width w₃. Exemplarily, the second width w₂ is twice the first width w₁ and the third width w₃ is three times the first width w₁.

Furthermore, the ridges 501 of the first connection means 511 have a first height h₁, the ridges 501 of the second connection means 520 have a second height h₂, and the ridges 501 of the third connection means 530 have a third height h₃. The first height h₁ is larger than the second height h₂, and the second height h₂ is larger than the third height h₃. Thereby, the second height h₂ is twice the third height h₃ and the first height h₁ is three times the third height h₃.

The aforementioned differences in the heights h₁, h₂, h₃, combined with the aforementioned differences in the widths w₁, w₂, w₃ reliably prevent mounting of the individual dispensing units 420, 430, 440 to other than their corresponding drug delivery device 220, 222, 225 with the matching connection means 511, 520, 530.

The different heights h₁, h₂, h₃ result from different outer diameters with a first outer diameter D₁ of the first connection means 424, 511 being larger than a second outer diameter D₂ of the second connection means 434, 520 and the second outer diameter D₂ of the second connection means 434, 520 being larger than a third outer diameter D₃ of the third connection means 444, 530. The first connection means 424, 511 have a first core diameter CD₁, the second connection means 434, 520 have a second core diameter CD₂, and the third connection means 444, 530 have a third core diameter CD₃ and all core diameters CD₁, CD₂, CD₃ are equal.

With other embodiments, the different heights h₁, h₂, h₃ may also result from differing core diameters CD₁, CD₂, CD₃ and, optionally, also differing outer diameters D₁, D₂, D₃. For example, the different heights h₁, h₂, h₃ may result from one of the core diameters CD₁, CD₂, CD₃ and the outer diameters D₁, D₂, D₃ being different among the connections means 424, 434, 444, 511, 520, 530 and the other one of the core diameters CD₁, CD₂, CD₃ and the outer diameters D₁, D₂, D3 being the same among the connections means 424, 434, 444, 511, 520, 530. According to still another embodiment, the core diameters CD₁, CD₂, CD₃ could be chosen to be mutually identical and also the outer diameters D₁, D₂, D₃ could be chosen to be mutually identical for all connections such that all devices 220, 222, 225 comprise threads 511, 520, 530 with ridges 501 of the same height.

With the embodiments shown in Fig. 36 and Fig. 37, a first pitch P₁ of the first connection means 424, 511, a second pitch P₂ of the second connection means 434, 520 and a third pitch P₃ of the third connection means 444, 530 are the same. Furthermore, a first angle A₁ of the first connection means 424, 511, a second angle A₂ of the second connection means 434, 520 and a third angle As of the third connection means 444, 530 are also the same.

With the exemplary embodiments shown in Fig. 36 and Fig. 37, the individual thread dimensions may be the following: CD₁=CD₂=CD₃=12.60 mm, D₁=14.70 mm, D₂=14.00 mm, D₃=13.30 mm, h₁=2.10 mm, h₂=1.40 mm, h₃=0.70 mm, w₁=0.65 mm, w₂=1.30 mm, w₃=1.95 mm, A₁=A₂=A₃=60°. The pitches of the individual threads may thereby all amount to P₁=P₂=P₃=3.80mm.

The thread dimensions may also be the following: CD₁=CD₂=CD₃=12.60 mm, D₁=14.70 mm, D₂=14.00 mm, D₃=13.30 mm, h₁=1.05 mm, h₂=0.70 mm, h₃=0.35 mm, w₁=0.65 mm, w₂=1.30 mm, w₃=1.95 mm, A₁=A₂=A₃=60°. The pitches of the individual threads may all amount to P₁=P₂=P₃=3.80mm.

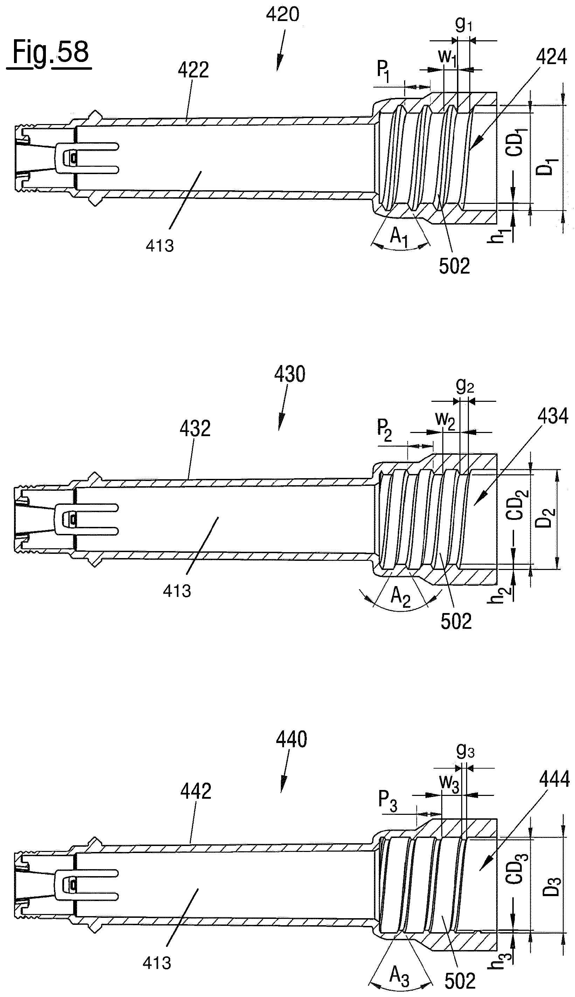

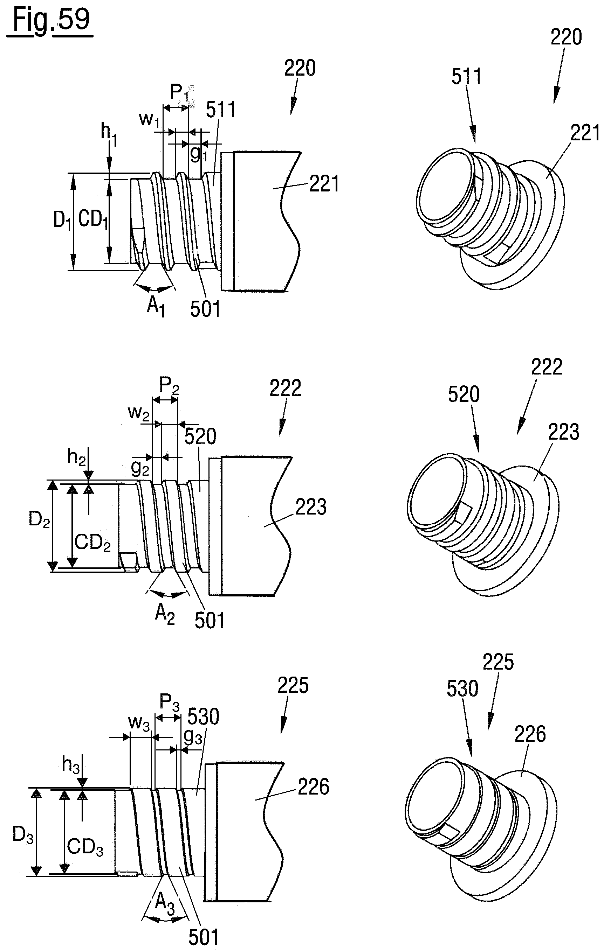

Alternatively, the aforementioned dimensions of the width *w* of the ridges of the male threads may apply to the widths *g* of the valleys of the male threads instead of to the width w of the ridges of the male threads. The widths *g* of the valleys of the individual male threads may thereby be defined as the bottom sections of the grooves of the male threads that are located at the core diameter and that extend between the angled side surfaces that delimit the ridges of the male thread, as depicted in Figs. 58 and 59.

The thread dimensions may then be the following: CD₁=CD₂=CD₃=12.60 mm, D₁=14.70 mm, D₂=14.00 mm, D₃=13.30 mm, h₁=1.05 mm, h₂=0.70 mm, h₃=0.35 mm, g₁=1.95 mm, g₂=1.30 mm, g₃=0.65 mm, A₁=A₂=A₃=60° and P₁=P₂=P₃=3.80mm. With this embodiment, the widths w of the ridges of the male threads may amount to: w₁=1.84 mm, w₂=2.50 mm, w₃=3.15 mm. The widths *w* of the ridges are thereby defined as the widths including the top surfaces that define the outer diameter D and both angled side surfaces that connect the individual top surfaces to neighbouring valleys. Therefore, the widths w amount to *w = P - g.* These dimensions are shown in Fig. 58, which shows the dispensing units 420, 430, 440, and in Fig. 59, which shows the corresponding connection means 511, 520, 530 of the dispensing units 220, 222, 225.

In general, among a set of N drug delivery devices 200, the N-th device may have a thread with ridges that have a width that is N-times the width of the ridges of the thread of the first device and the first device may have a thread with ridges that have a height that is N-times the height of the ridges of the N-th device. The m-th device (with 1 ≤ m ≤ N) may then have a thread that has ridges with a width that is m-times the width of the ridges of the thread of the first device and with a height that is (N-m+1)-times the height of the ridges of the thread of the N-th device.

Alternatively, the aforementioned relation may analogously apply to the widths *g* of the valleys of the male threads instead of the widths w of the ridges of the male threads. Thereby, the first device may have a thread with valleys that have a width *g* that is N-times the width *g* of the valleys of the thread of the N-th device and the first device may have a thread with ridges that have a height that is N-times the height of the ridges of the N-th device. The m-th device (with 1 ≤ m ≤ N) may then have a thread that has valleys with a width *g* that is (N-m+1)-times the width *g* of the valleys of the thread of the N-th device and with a height that is (N-m+1)-times the height of the ridges of the thread of the N-th device.

In one embodiment, the first, second and third drug delivery device 220, 222, 225 each are a variant of the drug delivery device 200 disclosed in connection with Fig. 1 to Fig. 35. As far as no differences are described or apparent from the Figures, the first, second and third drug delivery device 220, 222, 225 are then configured as it is disclosed in connection with the drug delivery device 200 and vice versa. Furthermore, the first, second and third dispensing unit 420, 430, 440 each are a variant of the dispensing unit 410 disclosed in connection with Fig. 1 to Fig. 35. As far as no differences are described or apparent from the Figures, the first, second and third dispensing unit 420, 430, 440 are then configured as it is disclosed in connection with the dispensing unit 410 and vice versa.

The second drug delivery device 222 and the first drug delivery device 220 share at least one mutual member that is identical among the first and second drug delivery device 220, 222 and the third drug delivery device 225 and the first drug delivery device 220 share at least one further mutual member that is identical among the first and third drug delivery device 220, 225. Thereby, the mutual member and the further mutual member are identical. With other embodiments, the mutual member and the further mutual member may also be different. Mutual members are thereby both mechanically identical, that is identical in shape, and identical in their appearance, such as in their color and printing.

The second drug delivery device 222 and the first drug delivery device 220 each comprise at least one distinguishing member that is different among the first and second drug delivery device 220, 222 and the third drug delivery device 225 and the first drug delivery device 220 each comprise at least one further distinguishing member that is different among the first and third drug delivery device 220, 225. Thereby, the distinguishing member and the further distinguishing member are the same functional member and therefore perform the same function during use of the dosing mechanism. With other embodiments, the mutual member and the further mutual member may also be different functional members.

Distinguishing members are at least different in their appearance, such as color and printing. Additionally, they may also be mechanically different, that is they may be different in shape. Despite being different in appearance and, optionally shape, the individual distinguishing members perform the same function during dose setting and dose delivery and thus constitute the same functional member among the individual drug delivery devices 220, 222, 225. The individual distinguishing elements are therefore designated by the same term in all drug delivery devices 200, 220, 222, 225.

The functional members constitute the individual parts of which the drug delivery devices 220, 225, 225 are assembled. While the individual parts may differ in their exact shape and appearance, for example to provide different dose increments among the individual drug delivery devices 220, 225, 225, they perform the same function and are located at the same positions within the dosing mechanisms 230 of the individual drug delivery devices 220, 225, 225. Furthermore, they interact and engage with the same further functional members of the dosing mechanism 230 among all drug delivery devices 220, 225, 225 of the set. Functional members may be composed of several sub-parts that are rigidly connected to each other to form a single mechanical part. With one embodiment of the present disclosure, a dosing member may, for example, constitute a functional member that is composed of two sub-parts, namely a dose sleeve and a snap element.

The first and second drug delivery device 220, 222 form a first set of drug delivery devices that mechanically differ only by their outer housings 221, 223, which carry the keyed connection means 510, 520. All other functional members of the drug delivery devices 220, 222 of the first set are mechanically identical. Therefore, the dosing mechanisms 240, the clutch mechanisms 234 and the dose definition mechanisms 232 of the two drug delivery devices 220, 222 are also the same. The two drug delivery devices 220, 222 are therefore configured to define identical rotational dose positions of the dose setting member 290 and to expel the same amount of liquid per settable dose increment.

One of the first set of drug delivery devices 220, 222 is configured to be used with its corresponding dispensing unit 420, 430 containing a drug having an active pharmaceutical ingredient in a first concentration and the other one of the first set of drug delivery devices 220, 222 is configured to be used with its corresponding dispensing unit 420, 430 containing the drug having the active pharmaceutical ingredient in a second concentration that is different from the first concentration.

Among the drug delivery devices 220, 222 of the first set, the piston rods 240, the plunger discs 242, the drivers 350, the nuts 250, the dose setting members 290, the first and second bearing elements 370, 380, the biasing members 308, the inner housings 180 and all elements of the resetting mechanism 110, namely the resetting elements 110, the coupling parts 130 and the biasing members 150, each form mutual members that are mutually identical both in appearance and shape among the two drug delivery devices 220, 222.

The dosing members 330 of the two drug delivery devices 220, 222 of the first set form a distinguishing member that differs in appearance but not in shape among the two drug delivery devices 220, 222. The difference in appearance thereby includes different numerals of the visual indicators 331, whereby the individual indicators 331 are located at the same positions on the dosing members 330 of the respective two drug delivery devices 220, 222.

The outer housings 211 of the two drug delivery devices 220, 222 of the first set form distinguishing elements that differ in shape due to the differences of their connection means 511, 520. Furthermore, the outer housings 211 differ in appearance, such as in color and/or labelling, to allow a user to clearly distinguish between the two devices 220, 222.

The dose selector members 310 and the caps 209 of the two drug delivery devices 220, 222 of the first set also form distinguishing members that differ in appearance but not in shape among the two drug delivery devices 220, 222. The difference in appearance thereby include different labelling on the dose selector member 310 and the cap 209. Furthermore, the caps 209 differ in color to match the colors of the respective body of their drug delivery device 220, 222. With other embodiments, the dose selector member 310 and/or the caps 209 could also be configured as mutual members. Furthermore, the caps 209 could also differ only in color and not in labelling or vice versa.

Each one of the first and second drug delivery device 220, 222 forms together with the third drug delivery device 225 a second set of drug delivery devices 200, 220, 225 that mechanically differ not only by their outer housings 211 but also by functional members of their dosing mechanisms 230, in particular of their dose definition mechanisms 232.

The dosing mechanism 230 of the third drug delivery device 225 is configured to provide a dialling resolution that is different from the dialling resolution of the first and second drug delivery device 220, 222. While the dosing mechanisms 230 of the first and second drug delivery device 220, 222 comprise the dose selector member 310 and the dose setting member 290 described in connection with Fig. 1 to Fig. 35, which are configured to define 27 settable dose positions, the third drug delivery device 225 comprises embodiments of the dose selector member 310 and the dose setting member 290 that are configured to define 18 settable dose positions.

The dose selector member 310 of the third drug delivery device 225 comprises 18 functional features 312 that are distributed around its inner surface. A position of the elastic elements 292 of the dose setting member 290 is thereby adapted to the larger distance between the individual functional features 312 to allow for reliable engagement between the elastic elements 292 and the functional features 312.

Since the dose definition mechanisms 332 of the third drug delivery device 225 define an even number of settable doses, the connection 277 between the clutch member 270 and the dose setting member 290 is configured to connect the clutch member 270 and the dose setting member 290 in two different relative rotational orientations that differ from each other by 180°. To achieve this, the first and second longitudinal grooves 297 and 298 of the dose setting member 290 and the corresponding first and second ridge 279, 280 of the clutch member 270 each have the same widths.

The clutch member 270 of the third drug delivery device 225 comprises 18 clutch elements 273, the circumferential positions of which are adapted to the circumferential positions of the functional features 312 of the dose selector member 310. Therefore, the number and circumferential positions of the clutch elements 273 of the clutch member 270 of the third drug delivery device 225 differs from the number and circumferential positions of the clutch elements 273 of the clutch member 270 of the first and second drug delivery device 220, 222.

The clutch members 270 of, on the one hand, the first and second drug delivery devices 220, 222 and of, on the other hand, the third drug delivery device 225 form distinguishing members that differ in shape among the second sets of drug delivery devices 220, 222 225. Likewise, the dose setting members 290 of, on the one hand, the first and second drug delivery devices 220, 222 and of, on the other hand, the third drug delivery device 225 also form distinguishing members that differ in shape among the second sets of drug delivery devices 220, 222 225.

The dosing member 330 of the third drug delivery device 225 comprises 18 clutch elements 336, the circumferential positions of which are adapted to the circumferential positions of the clutch elements 273 of the clutch member 270. Therefore, the dosing member 330 of the third drug delivery device 225 and each one of the dosing members 330 of the first and second drug delivery device 220, 222 form distinguishing members that differ in shape among the second sets of drug delivery devices 220, 222 225.

In general, the clutch mechanism 234 of the first drug delivery device 220 and the clutch mechanism 234 of the second drug delivery device 222 are configured to rotationally couple the nut 250 to the dosing member 330 and/or the housing 210 in the same relative rotational positions. The clutch mechanisms 234 of the first and second drug delivery device 220, 222 on the one hand and the clutch mechanism 234 of the third drug delivery device 225 on the other hand are configured to rotationally couple the nut 250 to the dosing member 330 and/or the housing 210 in different relative rotational positions.

Likewise, the clutch mechanisms 234 of the first drug delivery device 220 and the clutch mechanism 234 of the second drug delivery device 222 are configured to rotationally couple the dose setting member 290 to the dosing member 330 and/or the housing 210 in the same relative rotational positions. The clutch mechanisms 234 of the first and second drug delivery device 220, 222 on the one hand and the clutch mechanism 234 of the third drug delivery device 225 on the other hand are configured to rotationally couple the dose setting member 290 to the dosing member 330 and/or the housing 210 in different relative rotational positions.

With all drug delivery devices 200, 220, 222, 225, the clutch elements 273 of the clutch member 270, the clutch elements 336 of the dosing member 330, the clutch elements 312 of the dose selector member 310 and the clutch elements 294 of the dose setting member 290 are rotationally aligned with respect to each other in a way that in each rotational position of the dose setting member 290, in which the clutch elements 273 of the clutch member 270 and the clutch members 336 of the dosing member 360 are aligned with each other to allow mutual engagement, also the clutch elements 294 of the dose setting member 290 and the clutch elements 312 of the dose selector member 310 are aligned with each other to allow mutual engagement.

The dosing member 330 of the third drug delivery device 225 furthermore differs from the dosing member 330 of the first and second drug delivery device 220, 222 in appearance, as the positions of the optical markers 331 on the dosing member 330 of the third drug delivery device 225 differ from the positions of the optical markers 331 on the dosing members 330 of the first and second drug delivery devices 220, 222 to reflect the different number of doses settable per revolution of the dose setting member 290.

The numbering of the individual optical markers 331 on the dosing member 330 of the first drug delivery device 220 differs from the numbering of the individual optical markers 331 on the dosing member 330 of the third drug delivery device 225. This allows the first drug delivery device 220 to be used with a drug that has a first concentration of an active pharmaceutical ingredient and the third drug delivery device 225 to be used with a drug having a third concentration of an active pharmaceutical ingredient, whereby the product of the first concentration with the amount of liquid that is expelled by the first drug delivery device 220 per dose increment differs from the product of the third concentration with the amount of liquid that is expelled by the third drug delivery device 225 per dose increment.

The numbering of the individual optical markers 331 on the dosing member 330 of the second drug delivery device 222 equals the numbering of the individual optical markers 331 on the dosing member 330 of the third drug delivery device 225. This allows the second drug delivery device 220 to be used with a drug that has a second concentration of an active pharmaceutical ingredient and the third drug delivery device 225 to be used with a drug having a third concentration of an active pharmaceutical ingredient, whereby the product of the second concentration with the amount of liquid that is expelled by the second drug delivery device 222 per dose increment is equal to the product of the third concentration with the amount of liquid that is expelled by the third drug delivery device 225 per dose increment.

Due to the differences in shape and appearance, the dosing member 330 constitutes a distinguishing member among the second sets of drug delivery devices 220, 222, 225.

In total, mutual members of the second sets of drug delivery devices 220, 222, 225 are the piston rod 240, the plunger disc 242, the nut 250, the driver 350, the bearing elements 370, 380, the biasing member 308, the inner housing 180 and all elements of the resetting mechanism 110, namely the resetting element 110, the coupling part 130 and the biasing member 150.

Distinguishing members that only differ in appearance but not in shape among the second sets of drug delivery devices 220, 222, 225 are the caps 209, each of which has a different color. Distinguishing members that differ both in appearance and in shape among the second sets of drug delivery devices 220, 222, 225 are the outer housings 211, each of which has a different color and a differently shaped connection means 511, 520, 530, the dosing members 330, each of which has a different position and/or number and/or labelling of their optical markers 331 and differently shaped clutch elements 336, the dose selector members 310, each of which has a different labelling and a different amount of functional features 312, the clutch members 270, which differ in the shapes and/or number of their clutch elements 273 and hence also in their appearance, and the dose setting members 290, which differ in the positions of their elastic elements 292 and their clutch elements 294 and hence also in their appearance.

The first drug delivery device 220 is configured to be used with a drug containing the active pharmaceutical ingredient in a concentration of 5 mg / 1.5 ml, the second drug delivery device 222 is configured to be used with drug containing the active pharmaceutical ingredient in a concentration of 10 mg / 1.5 ml and the third drug delivery device 225 is configured to be used with a drug containing the active pharmaceutical ingredient in a concentration of 15 mg / 1.5 ml. Both the first and second drug delivery device 220, 222 have a dialing resolution of 0.015 ml per dose increment and the third drug delivery device 225 has a dialing resolution of 0.010 ml per dose increment.

The optical markers 331 on the dosing member 330 of the first drug delivery device 220 then display dose increments of 0.05 mg and the optical markers 331 on the dosing members 330 of the second and third drug delivery device 222, 225 then each display dose increments of 0.10 mg. All drug delivery devices 220, 222, 225 allow for two full rotations of the dose setting member 290 during dose setting. With 27 dose increments per revolution of the dose setting member 290, the first drug delivery device 220 is configured to expel a maximum dose of the active pharmaceutical ingredient of 1.80 mg and the second drug delivery device 222 is configured to expel a maximum dose of the active pharmaceutical ingredient of 3.60 mg. Since the third drug delivery device 225 provides 18 dose increments per revolution of the dose setting member 290, it is configured to deliver a maximum dose of the active pharmaceutical ingredient of 5.40 mg.

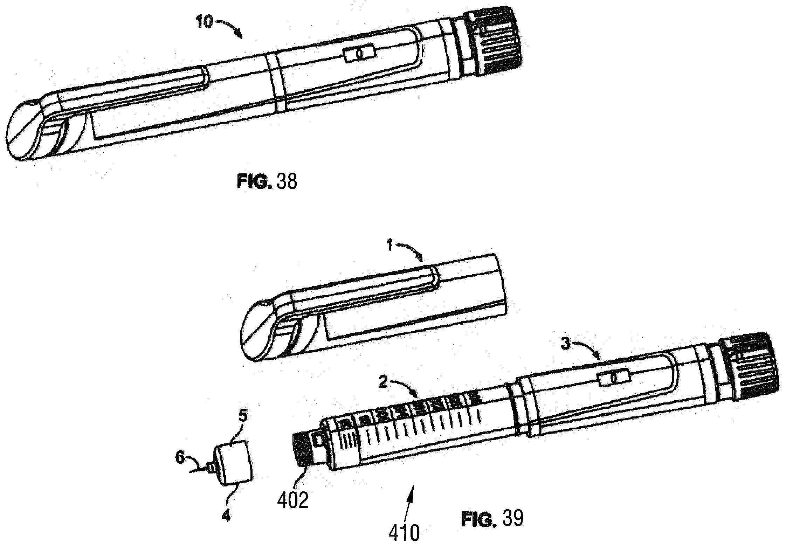

The resetting mechanisms according to the present disclosure are also applicable with other drug delivery devices, for example, injection devices. A further possible injection device is the pen-type further drug delivery device 10 illustrated in **Fig. 38 to Fig. 40****.** As far as no differences are described or apparent from the Figures, the further drug delivery device 10 is configured as it is disclosed in connection with the drug delivery device 200 and vice versa. The further drug delivery device 10 is also described in more detail in international applications WO2020/015980A1 and WO2019/011394A1, the disclosure of each of which is incorporated into this disclosure in its respective entirety by reference.

The further drug delivery device 10 has an outer housing 3 connected to a dispensing unit 410 with a cartridge holder 2 holding a cartridge 8. The cartridge holder 2 has a needle connector 402. The injection device 10 has a dosing mechanism 30 and is illustrated in the zero-dose state as indicated by an optical marker 40 showing a zero through a window 3a of the outer housing 3. The outer housing 3 terminates at its proximal end in a keyed connection means 510, which has a thread form.

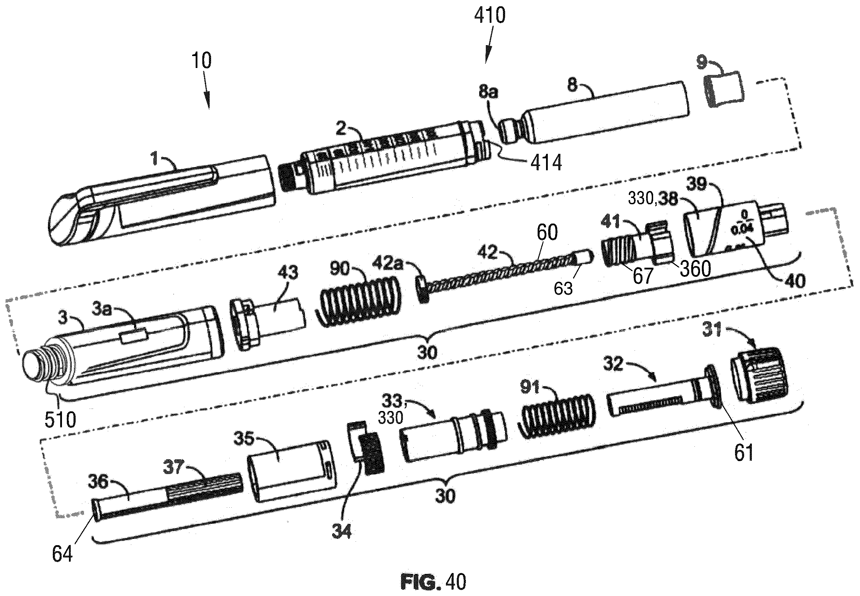

Fig. 40 schematically shows a simplified exploded view of the device 10 with a cap 1 removed to expose the cartridge holder 2 and the proximal needle connector 402. The needle 4 is typically attached to the needle connector 402 through a snap fit, thread, Luer-Lok, or other secure attachment with hub 5 such that a double ended needle cannula 6 can achieve a fluid communication with a drug contained in the cartridge 8 positioned within cartridge holder 2.

The particular design of the device 10 allows for setting of one or more of predetermined fixed doses through the interaction of a snap element 33 with a dose selector member 35. A rotation of a dose setting member 31 and the snap element 33 occurs during dose setting and is relative to outer housing 3. During the initiation of the dose delivery procedure the dose setting member 31 is pressed in the proximal direction causing it and the dose selector member 35 to move axially relative to the snap element 33. Like with the drug delivery device 200, the dose selector member 35 is axially movable and rotationally fixed with respect to the outer housing 3 of the further drug delivery device 10.

Part of the dosing mechanisms of most pen-type injectors, including device 10, is a piston rod 42 as illustrated in Fig. 40. The piston rod 42 has a non-circular cross-section and two flat surfaces that are designed to prevent the piston rod 42 from rotating with respect to the outer housing 3 but allowing it to move linearly in the proximal direction. A nut 36 and a clutch member 32 are permanently splined to each other during assembly of the dosing mechanism 30 through a splined connection 37. The splined connection 37 ensures that the clutch member 32 and the nut 36 are always rotationally fixed to each other during both dose setting and dose delivery. This splined connection 37 also allows the clutch member 32 and the nut 36 to move axially relative to each other during both dose setting and dose delivery.

The proximal end of the nut 36 has an internal thread that matches a corresponding outer thread 60 of the piston rod 42. The distal end of the clutch member 32 is configured as a dose button 61 and is permanently attached to the distal end of the dose setting member 31 through engagement of connectors, which may be configured as snap locks, an adhesive and/or a sonic weld. This connection ensures that the clutch member 32 is both rotationally and axially fixed to the dose setting member 31 during both dose setting and dose delivery. Alternatively, the clutch member 32 and the dose setting member 31 could also be configured as a single member.

At the terminal proximal end of the piston rod 42 is a connector, which is configured as a snap fit, that connects with a plunger disc or foot 42a. At the distal end of the piston rod 42 is a stop feature 63 of the dosing mechanism 30, illustrated as an enlarged section. This enlarged section 63 is designed to stop the rotation of the nut 36 about the thread 60 when the amount of medicament remaining in the cartridge 8 is less than the next highest predetermined dose setting. In other words, if the user tries to set one of the predetermined fixed dose settings that exceeds the amount of medicament remaining in the cartridge 8, then the enlarged section 63 will act as a hard stop preventing the nut 36 from further rotation along the thread 60 as the user attempts to reach the desired predetermined fixed dose setting. With the drug delivery device 200, the stop feature 243 interacts with the nut 250 in the same way and therefore also prevents setting of a dose larger than the remaining dose within the cartridge 8.

The piston rod 42 is held in a non-rotational state relative to the outer housing 3 during both dose setting and dose delivery by a piston rod guide 43. The piston rod guide 43 is both rotationally and axially fixed to the outer housing 3. Therefore, it forms part of a housing of the device 10. This fixation can be achieved when the piston rod guide 43 is a separate component from the outer housing 3 as illustrated or the piston rod guide 43 could be made integral with the outer housing 3, analogous to the inner sleeve 183 of the inner housing 180 of the drug delivery device 200. Although not shown in the Figures, the piston rod guide 43 may be configured as a resetting mechanism that, like the resetting mechanism 100 of the drug delivery device 200, prevents rotation of the piston rod 42 with respect to the housing 3 when the dispensing unit 410 is attached to the housing 3 of the drug delivery device 10 and that allows rotational movement of the piston rod 42 with respect to the housing 3 when the dispensing unit 410 is disengaged from the housing 3.

The resetting mechanism of the further drug delivery device 10 may be configured as it is disclosed in connection with the resetting mechanism 100 of the drug delivery device 200. In particular, the resetting mechanism of the further drug delivery device 10 may comprise the resetting element 110, the coupling part 130 and the biasing element 150.

The piston rod guide 43 also engages the proximal end of a rotational biasing member 90, shown as a torsion spring, the function of which will be explained below. This connection of the rotational biasing member 90 to the piston rod guide 43 anchors one end of the rotational biasing member 90 in a rotationally fixed position relative to the outer housing 3.

The distal end of the rotational biasing member 90 is connected to a driver 41. The driver 41 is connected to and rotationally fixed with respect to an inner surface of a dosing member 330 through a splined connection on the distal outer surface of the driver 41. This splined connection comprises at least one, such as two longitudinal ridges that are located on the outer diameter of the driver 41 and that engage with corresponding grooves on the inner surface of the dosing member 330. On the proximal end of the driver 41 on the outer surface is a thread 67 that is engaged with a matching thread on the inner distal surface of the piston rod guide 43.

The dosing member 330 comprises two parts that are rotationally and axially fixed to each other, for example by a snap-fit connection. One part forms a dose sleeve 38 that is connected to the driver 41 through the splined connection, the other part forms the snap element 33. As such, the dosing member 330 forms a single functional member.

The dosing member 330, namely the dose sleeve 38 is threadedly engaged with the body 3 by a helical groove 39 located on the outer surface of the dosing member 330 that engages with a corresponding helical ridge located on the inner surface of the body 3. The thread between the driver 41 and the piston guide 43 has a significantly different pitch than the thread between the dosing member 330 and the outer housing 3. The axially sliding connection between the nut 36 and the clutch member 32 allows to compensate for the differences in the pitch of the thread between the inner surface of the nut 36 and the outer surface of the piston rod 42 and the pitch of the thread between the dosing member 330 and the body 3. The thread between the driver 41 and the piston guide 43 has basically the same pitch as the thread between the piston rod 42 and the nut 36.

The nut 36 and the driver 41 rotate together both during dose setting and dose cancellation and, as such, they perform essentially the same axial movement. However, these movements are independent from each other, i. e., the nut 36 is turned by the clutch member 32 and performs an axial movement due to the thread to the piston rod 42, while the driver 41 is rotated by the dosing member 330 and performs an axial movement due to the thread to the piston guide 43. The driver 41 is rotating during injection also, and so it actively moves in the proximal direction during injection. But, the nut 36 does not rotate during injection and as such does not perform an active axial movement. The nut 36 is only moving in the proximal direction during injection because it is being pushed axially by the driver 41, which surrounds the nut 36 and abuts against a protrusion 64 located at the proximal end of the nut 36. The rotating driver 41 pushing the non-rotating nut 36 causes the injection because the piston rod 42 is pushed forward due to the threaded engagement with the nut 36.

Because the torsion spring 90 is attached to the driver 41 and the driver 41 is rotationally fixed to the dosing member 330, rotation of the dosing member 330 in a first direction during dose setting will wind the torsion spring 90 such that it exerts a counter rotational force on the dosing member 330 in an opposite second direction. This counter rotational force biases the dosing member 330 to rotate in a dose canceling direction.

In general, the further drug delivery device 10 comprises a biasing member, which is exemplarily configured as the torsion spring 90, that is strained upon increasing the set dose. Furthermore, the biasing member is released during dose delivery. Thereby, the biasing member at least assists delivery of the set dose by providing a force that advances the piston rod 60 in the proximal direction. Such a biasing member may also be provided in the drug delivery device 200. For example, it may also be configured as a torsion spring that is provided between the inner housing 180 and the driver 350 of the device 200 and that acts between the inner housing 180 and the driver 350 in the same way as the torsion spring 90 acts between the piston guide 43 and the driver 41 of the further drug delivery device 10.

The function of the complete further drug delivery device 10 and the dosing mechanism 30 will now be described. The further drug delivery device 10 is provided to a user as reusable or semi-reusable device. A semi-reusable means that only the dosing mechanism 30 housed in the outer housing 3 is reused each time a new dispensing unit 410 having a cartridge holder 2 containing a new cartridge 8 of medicament is connected to the outer housing 3. A reusable device would allow reattachment of an old or previously used cartridge holder 2 where the user has inserted a new full cartridge 8 of medicament. In one configuration according to the present disclosure, the device 10 has the semi-reusable design where each time the medicament in the cartridge 8 is expelled or emptied, the user would be required to disconnect the cartridge holder 2 containing the empty cartridge 8 that is not removable from the cartridge holder 2. As such, the user would dispose of both the cartridge holder 2 and the empty cartridge 8 together. A new cartridge holder 2 and cartridge 8 assembly would be connected to the outer housing 3 provided that the keyed connection means 510 on the outer housing 3 matches a keyed connection means 414 provided on the distal end of the cartridge holder 2.

With the further drug delivery device 10, the dose sleeve 38 and the snap element 33 are axially and rotationally fixed with each other via a snap-fit connection. Therefore, the dose sleeve 38 and the snap element 33 constitute a single functional element, namely the dosing member 330. With other embodiments of the further drug delivery device 10, the dosing member 330 could also be configured as a single component or member.

A housing of the further drug delivery device 10 comprises the outer housing 3 and the piston guide 43, which are rotationally and axially fixed with respect to each other.

Like the drug delivery device 200, the further drug delivery device 10 comprises a clutch mechanism 237. During dose setting, the clutch mechanism 237 rotationally fixes the nut 36 with respect to the driver 41 and the dosing member 330 and allows rotation of the nut 36 with respect to the housing 3, 43. During dose delivery, the clutch mechanism 237 rotationally fixes the nut 36 with respect to the dose selector member 35 and the housing 3, 43 and allows relative rotation between the nut 36 on the one hand and the driver 41 and the dosing member 330 on the other hand.

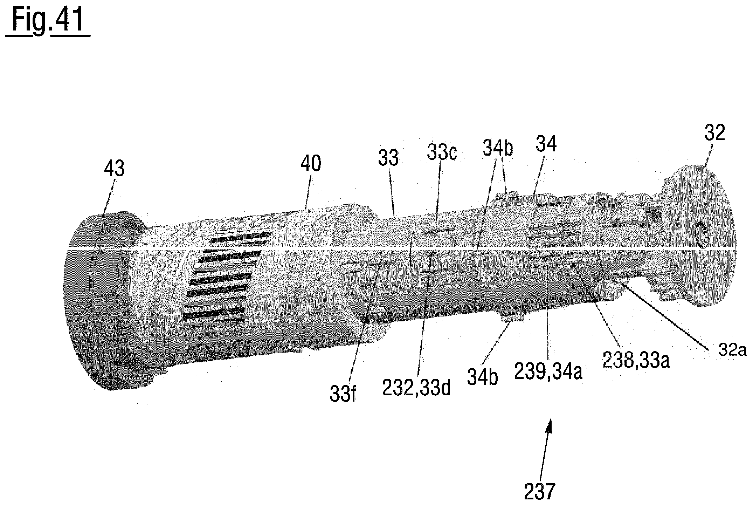

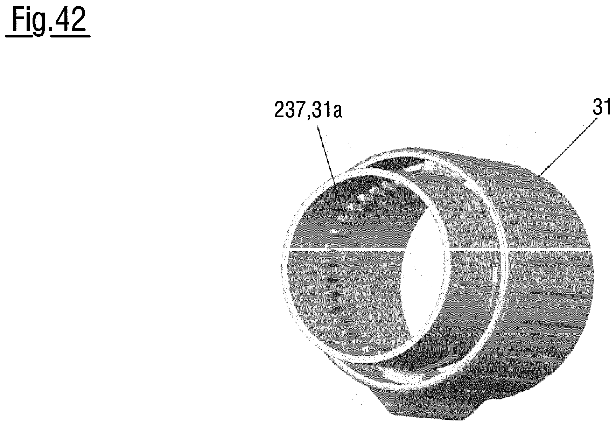

As can be seen from **Fig. 41** and **Fig. 42****,** a first part 238 of the clutch mechanism 237 comprises clutch elements 33a that are configured as radially extending teeth and that are provided on an outer surface at a distal end of the snap element 33 of the dosing member 330. A second part 239 of the clutch mechanism 237 comprises clutch elements 34a that are configured as radially extending teeth and that are provided on an outer surface at a distal end of a connector 34.

The connector 34 is located within an annular recess of the dosing member 330 and is thereby rotationally movable and axially fixed with respect to the dosing member 330. The connector 34 is axially movable and rotationally fixed with respect to the dose selector member 35. This is exemplarily achieved by radially protruding ridges 34b of the connector 34 that are received in corresponding longitudinal grooves on an inner surface of the dose selector member 35. The rotationally fixed connection to the dose selector member 35 also rotationally fixes the connector 34 to the housing 3, 34 of the further drug delivery device 10.

The dosing member 330 surrounds the clutch member 32 and the clutch member 32, together with the dose setting member 31 and the dose selector member 35, is axially movable with respect to the dosing member 330. Thereby, the dose setting member 31 and the clutch member 32 are biased into the distal direction by a compression spring 91 (shown in Fig. 40) that acts between the dosing member 330 and the clutch member 32. Axial movement of the clutch member 32 and the dose setting member 31 in the proximal direction is allowed until the dose setting member 31 pushes upon the dosing member 330 via the clutch member 32. Thereby, a push member 32a, which is exemplarily configured as a ridge protruding from an outer surface of a cylindrical portion of the clutch member 32, pushes upon the dosing member 330, namely on the distal end of the snap element 33.

During dose setting, the clutch member 32 and the dose setting member 31 are in their distal position with respect to the dosing member 330. In this position, the dose setting member 31 is rotationally coupled to the dosing member 330 via the first part 238 of the clutch mechanism 237 that comprises the clutch elements 33a at the distal end of the snap element 33 of the dosing member 330 and corresponding clutch elements 31a on an inner surface of the dose setting member 31, which are shown in Fig. 42. When rotating the dose setting member 31 during dose setting, the dosing member 330 is also rotated via the closed first part 238 of the clutch mechanism 237 between the dose setting member 31 and the dosing member 330 and screwed out of the outer housing 3. This forces the dose selector member 35 and the dose setting member 31 to also move in the distal direction.

Rotation of the dosing member 330 also forces a corresponding rotation of the driver 41, which is therefore also screwed out of the piston guide 43.

Since the nut 36 is rotationally fixed with respect to the clutch member 32, rotation of the dose setting member 31 also causes rotation of the nut 36 during dose setting. Thereby, the nut 36 is screwed along the piston rod 42 and also moves into the distal direction. The pitches of the threads of the piston rod 42 and the driver 41 are adapted so that the nut 36 and the driver 41 essentially move the same axial distance upon rotation. Thereby, the nominal pitch of the connection between the driver 41 and the piston guide 43 is slightly higher than the nominal pitch of the thread between the piston rod 42 and the nut 36 to prevent mutual blocking of the nut 36 and driver 41 irrespective of manufacturing tolerances.

To eject a set dose, the dose setting member 31, the clutch member 32 and the dose selector member 35 are moved into their proximal position with respect to the dosing member 330. This releases the first part 238 of the clutch mechanism 237 between the snap element 33 of the dosing member 330 and the dose setting member 31 and engages the second part 239 of the clutch mechanism 237, which is realized between the dose setting member 31 and the connector 34 that surrounds the dosing member 330. Upon engagement of the second part 239 of the clutch mechanism 237, the clutch elements 31a of the dose setting member 31 engage with the clutch elements 34a of the connector 34.

Engagement of the second part 239 of the clutch mechanism 237 rotationally locks the dose setting member 31 and the clutch member 32 to the connector 34 and, via the ridge 34b of the connector 34, also to the dose selector member 35 and the housing 3, 43. Therefore, the nut 36 is rotationally locked with respect to the housing 3, 43 and the piston rod 42 during dose delivery. This locking is achieved via the nut 36, the clutch member 32, the dose setting member 31, the connector 34 and the dose selector member 35.

Disengagement of the first part 238 of the clutch mechanism 237 allows rotational movement between the nut 36 and the driver 41 and the dosing member 330 during dose delivery.

When further pushing the dose setting member 31 into the proximal direction, the clutch member 32 abuts against the dosing member 330 and forces the dosing member 330 to move into the proximal direction. Due to the threaded connection between the dosing member 330 and the outer housing 3, the dosing member 330 rotates when moving into the proximal direction. This rotation is transferred to the driver 41, which is screwed into the proximal direction into the piston guide 43 and therefore also moves axially in the proximal direction. The driver 41 thereby abuts and advances the nut 36, which is now rotationally fixed to the outer housing 3 and the piston rod 42 via the clutch member 32, the dose setting member 31, the connector 34 and the dose selector member 35. Therefore, both the piston rod 42 and the nut 36 are rotationally fixed with respect to each other and axial advancement of the nut 36 causes a corresponding axial advancement of the piston rod 42, thus expelling the set dose.

Like the drug delivery device 200, also the further drug delivery device 10 may comprise one or more friction reduction mechanisms that reduce friction within the dosing mechanism 30 during delivery of a set dose. These friction reduction mechanisms may be configured in the same way as it is disclosed in connection with the drug delivery device 200.

For example, the first friction reduction mechanism may be provided between an actuation member of the further drug delivery device 10, which is formed by the clutch member 32, and the dosing member 330. The clutch member 32 acts as an actuation member that provides a force in the proximal direction that effects delivery of the set dose when a user pushes on the distal part of the clutch member 32.

Thereby, the first friction reduction mechanism may be directly contacted by the clutch member 32 and the dosing member 330. For example, the friction reduction mechanism may be provided between the distal end of the dosing member 330 and the protruding ridge 32a of the clutch member 32.

With other embodiments of the further drug delivery device 10, the first friction reduction mechanism that is provided between the actuation member and the dosing member 330 may also be contacted via one or more intermediate members. For example, the first friction reduction mechanism may be provided between the dose selector member 35 and the dosing member 330. When pushing the clutch member 32 and therefore also the dose setting member 31 in the proximal direction, the dose selector member 35, for example the proximal end of the dose selector member 35, may abut against the dose sleeve 38, for example against the distal end of the dose sleeve 38. The first friction reduction mechanism, such as the ball bearing 370, may then be provided between the dose selector member 35 and the dose sleeve 38, for example between the proximal end of the dose selector member 35 and the distal end of the dose sleeve 38.

Additionally or alternatively, the second friction reduction mechanism may be provided between the driver 41 and the nut 36 in the same ways as it is disclosed in connection with the second friction reduction mechanism, such as the disc bearing 380, of the drug delivery device 200.

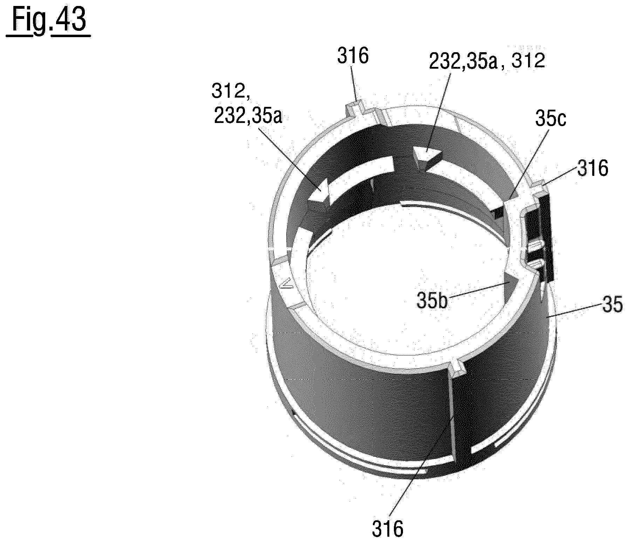

The further drug delivery device 10 comprises a dose definition mechanism 232 that acts between the dosing member 330 and the dose selector member 35. During dose setting, the dosing member 330 rotates with respect to the dose selector member 35. As can be seen from Fig. 41, the dosing member 330, namely the snap element 33, has, on its outer surface, a flexible arm 33c with a radial protrusion 33d, which forms an elastic element and engages with dose stops 35a on the inner surface of the dose selector 35. The dose stops 35a, which are shown in **Fig. 43****,** form functional features 312 of the dose definition mechanism 232.

The circumferential positions of the individual dose stops 35a thereby define individual relative rotational positions between the dosing member 330 and the housing 3, 43 that correspond to settable doses. To prevent the dialing of intermediate doses in between the individual dose stops 35a, the torsion spring 90 is provided between the piston guide 43 and the driver 41. This torsion spring 90 is loaded when increasing the set dose and causes the dosing member 330 to rotate back to the last set dose in cases where the dose setting member 31 is released while the protrusion 33d on the dosing member 330 is positioned in between two dose stops 35a.

With the further drug delivery device 10, the dosing member 330 is limited to perform less than one full rotation upon dose setting. The further drug delivery device 10 comprises a stop mechanism that defines a maximum and minimum rotational position of the dosing member 330 during dose setting.

The stop mechanism acts between the snap element 33 of the dosing member 330 and the dose selector member 35. It comprises a further protrusion 33f that is located on the outer surface of the dosing member 330 and that radially protrudes towards the dose selector member 35. The dose selector member 35 comprises a maximum stop feature 35b that is located on an inner surface of the dose selector member 33 and that is configured as a side surface of a step located on the inner surface. Furthermore, the dose selector member 35 comprises a zero stop feature 35c that is located also on the inner surface of the dose selector member 33. The zero stop feature 35c is exemplarily configured as a side surface of the step that opposes the side surface forming the maximum stop feature 35b. With other embodiments of the dose selector member 33, the zero stop feature 35c and the maximum stop feature 35b may also be provided at separate protrusions or steps on the inner surface of the dose selector member 33.

The further protrusion 33f of the dosing member 330 is configured to abut the maximum stop feature 35b upon rotation of the dosing member 330 into a rotational position that corresponds to or exceeds a maximum settable dose and thereby prevents further rotation of the dosing member 330. Likewise, the further protrusion 33f of the dosing member 330 is configured to abut the zero stop feature 35c upon rotation of the dosing member 330 into a rotational position that corresponds to a zero dose setting and thereby prevents further rotation of the dosing member 330.

The further drug delivery device 10 may also comprise an alternative embodiment of the stop mechanism that defines a maximum dose position and/or a zero dose position of the dosing member 330 with respect to the housing 3, 43. The alternative embodiment may be configured like the stop mechanism of the drug delivery device 200. Thereby, a maximum dose stop may be provided at the dosing member 330, such as at the dose sleeve 38 or the snap element 33, and a corresponding maximum stop feature may be provided at the housing 3, 43. The maximum dose stop and/or the maximum stop feature may be configured as it is described in connection with the maximum dose stop 337 and the maximum stop feature 190 of the drug delivery device 200.

Likewise, the alternative embodiments of the stop mechanism of the further drug delivery device 10 may comprise a zero dose stop that is provided at the dosing member 330, such as at the dose sleeve 38 or the snap element 33, and a corresponding zero stop feature that is provided at the housing 3, 43, for example at the piston guide 43. The zero dose stop and/or the zero stop feature may be configured as it is described in connection with the maximum dose stop 337 and the maximum stop feature 190 of the drug delivery device 200.

Like the drug delivery device 200, the further dose delivery device 10 may be provided in several variants that are distinguished by their connection means 510 to be configured to only connect to a dedicated variant of the dispensing unit 410. The connection means 510 may thereby be configured as it is disclosed in connection with Fig. 36 and Fig. 37.

In one embodiment, the several variants of the further drug delivery device 10 comprise as distinguishing members the outer housing 3, the cap 1, the dose sleeve 38 and the dose selector member 35. The outer housings 3 differ in shape due to the differences in the connection means 510 and also in appearance due to different colors and/or labeling. The dose selector members 35 differ in shape due to different numbers and/or different positions of the dose stops 35a, which allows to realize different dialing resolutions or settable doses. Alternatively or additionally, the dose selector members 35 may also differ in the position of the maximum stop feature 35c. The dose sleeves 38 are mechanically identical among the individual variants but differ in appearance due to different positions and/or numbering of their optical markers. The caps 1 are identical in shape but differ in their appearance, like color and/or labelling. With other embodiments, the caps 1 could also be configured as mutual members.

Mutual members of the variants of the further drug delivery devices 10 then may be all other elements of the dosing mechanism 30.

With both types of drug delivery devices 10, 200 the mechanical advantage of the dosing mechanisms 230 during dose dispensing may be different among devices of the individual sets. For example, a set may comprise one device having a higher mechanical advantage than another device of the respective set. Among these devices, the driver 41, 350 and the part of the housing 210 that is threadedly connected to the driver 41, 350, like the inner housing 180 and the piston guide 43, may be distinguishing members that mechanically differ from each other due to different pitches of their threads 67, 186, 353. Additionally or alternatively, the dosing member 330, in particular the dose sleeve 38, and the part of the housing 210 that is threadedly connected to the dosing member 330, like the inner housing 180 and the housing 3, may be distinguishing members that mechanically differ from each other due to different pitches of their threads 39, 185, 335. All sets of drug delivery devices 10, 200 described in the present disclosure may comprise drug delivery devices 10, 200 that differ by the mechanical advantage of their dosing mechanisms 230 during dose dispensing.

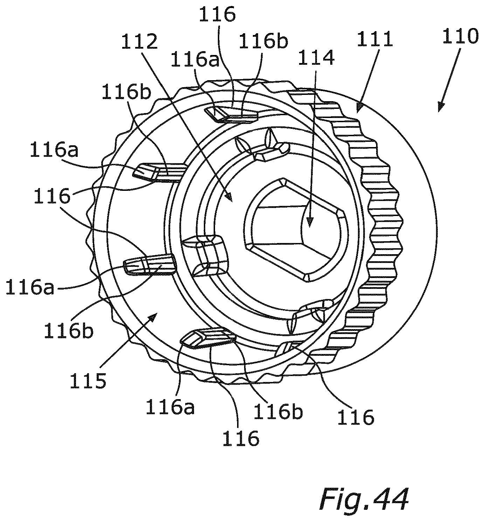

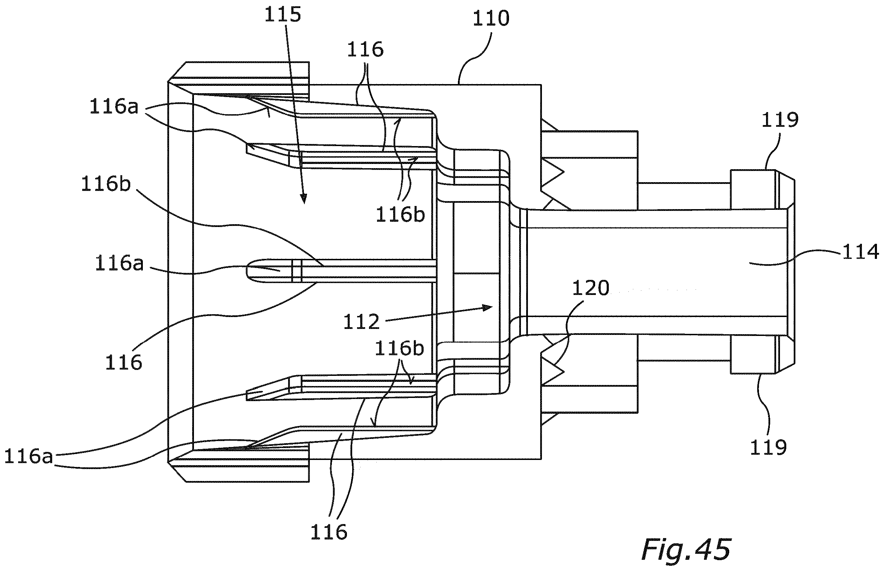

**Fig. 44** and **Fig. 45** show an alternative embodiment of the resetting element 110 of the drug delivery device 200. As far as no differences are described or apparent from the Figures, the resetting element 110 according to the alternative embodiment is configured as it is described above in connection with the resetting element 110 of the drug delivery device 200 and vice versa.

The resetting element 110 comprises guiding structures 116 that are located within the cartridge cavity 115. The guiding structures 116 have an elongated shape and extend parallel to the longitudinal axis 207. They are placed on the circumferential side wall of the cartridge cavity 115. The guiding structures 116 are thereby equally spaced apart from each other. With the embodiment shown in Figs. 44 and 45, the resetting element 110 exemplarily comprises eight of the guiding structures 116. With other embodiments, the resetting element 110 may comprise more or less guiding structures 116.

The guiding structures 116 are configured to center the distal end of the cartridge 8 with respect to the longitudinal axis 207 when the dispensing unit 410 is attached to the drug delivery device 200. The guiding structures 116 radially touch a cartridge 8 that is inserted into the cartridge holder 412. As such, they only define the lateral position of the cartridge 8 with respect to the longitudinal axis 207 but not the axial position of the distal end of the cartridge 8. Furthermore, the axial position of the distal end of the cartridge 8 also does not define the axial position of the resetting element 110.

The guiding structures 116 are configured to not be pushed upon by the cartridge 8 during attachment of the dispensing unit 410 to the drug delivery device 200. The guiding structures 116 comprise an inclined front surface 116a that faces in the proximal direction. The inclined front surface 116a centers the cartridge 8 but prevents the resetting element 110 from receiving an axial force via the cartridge 8 that would axially displace the resetting element 110. The guiding structures 116 also comprise an inclined back surface 116b that faces in the distal direction.

Both the front surfaces 116a and the back surfaces 116b may have an angle with the longitudinal axis 207 that is at most 45°, for example at most 30°, 20° or 10°. For example, the front surfaces 116a may have an angle with the longitudinal axis 207 that is larger than 5°, larger than 10°or larger than 15° and/or smaller than 45°, smaller than 30°, or smaller than 25°. The angle may, for example, equal 20°. The back surfaces116b may have, for example, an angle with the longitudinal axis 207 that is larger than 0° or larger than 0.5° and/or smaller than 10°, smaller than 5°, or smaller than 2.5°. The angle may, for example, equal 1°.

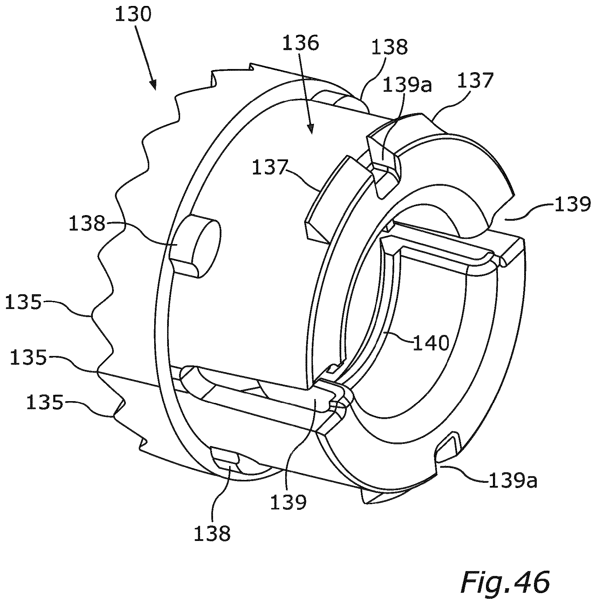

Fig. 46 shows an alternative embodiment of the coupling part 130 of the drug delivery device 200. As far as no differences are described or apparent from the Figures, the coupling part 130 according to the alternative embodiment is configured as it is described above in connection with the coupling part 130 of the drug delivery device 200 and vice versa.

The alternative embodiments of the coupling part 130 comprises four of the protrusions 138. The protrusions 138 are circumferentially distributed around the longitudinal axis 207 and equally spaced apart from each other in the circumferential direction.

Furthermore, the alternative embodiment of the coupling part 130 comprises, in addition to the slots 139, recesses 139a. In Fig. 46, the coupling part 130 is exemplarily shown having two of the recesses 139a. The recesses 139a are located at the distal end of the coupling part 130. Each recess 139a is centered with one of the first locking structures 137 and divides the respective first locking structure 137 into two parts. As can further be seen from Fig. 46, the slots 139 and the recesses 139a are alternately distributed in the circumferential direction and equally spaced from each other.

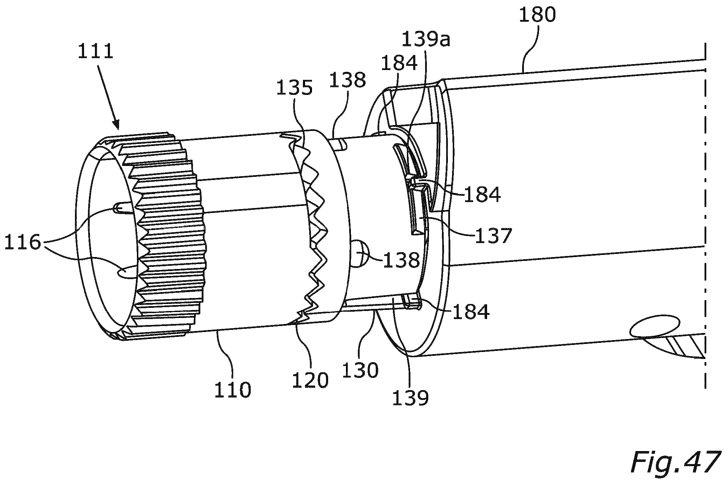

**Fig. 47** shows the alternative embodiment of the resetting element 110 and the alternative embodiment of the coupling part 130 mounted to an alternative embodiment of the inner housing 180. As far as no differences are described or apparent from the Figures, the alternative embodiment of the housing 180 is configured as it is described above in connection with the inner housing 180 of the drug delivery device 200 and vice versa.

The alternative embodiment of the inner housing 180 comprises one of the tappets 184 for each one of the slots 139 and recesses 139a. In total, the inner housing 180 therefore comprises four tappets 184. The tappets 184 are provided at the proximal end of the inner housing 180. Furthermore, they are equally spaced from each other in the circumferential direction around the longitudinal axis 207.

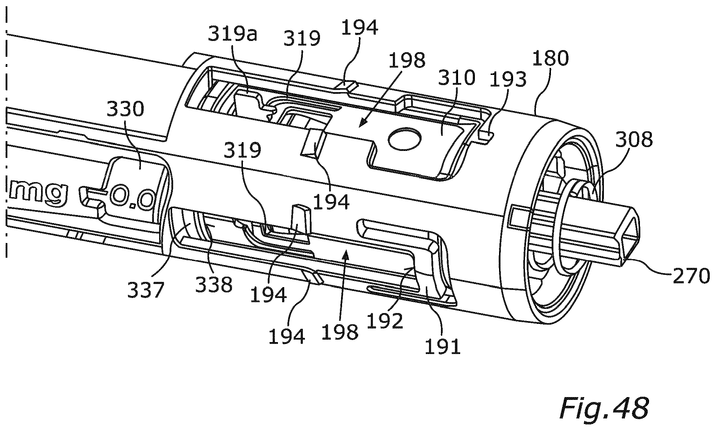

**Fig. 48** to **Fig. 49** show an alternative connection between an alternative embodiment of the inner housing 180 and an alternative embodiment of the dose selector member 310. As far as no differences are described or apparent from the Figures, the alternative embodiments of the inner housing 180 and/or the alternative embodiments of the dose selector member 310 are configured as it is described in connection with the other embodiments of the inner housing 180 and the dose selector member 310 according to the present disclosure.

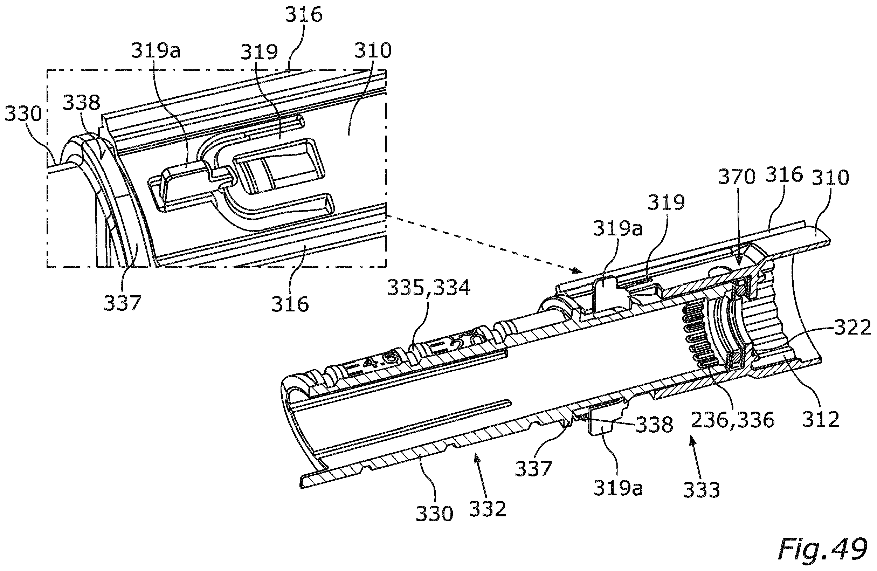

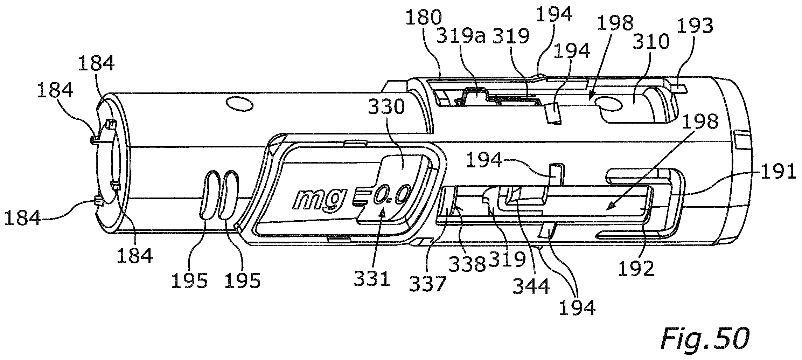

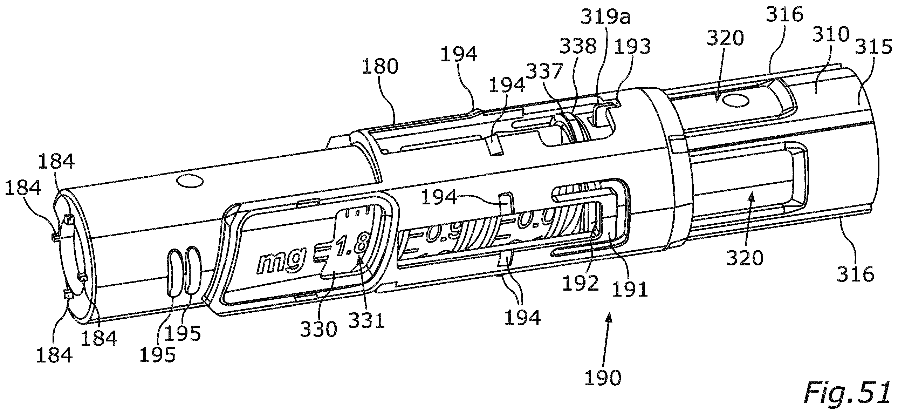

The dose selector member 310 shown in Fig. 48 and Fig. 49 comprises longitudinal protrusions 319a on two of the flexible members 319, wherein the longitudinal protrusions 319a project radially outward into longitudinal slots 198 within the inner housing 180. As can be seen from Fig. 48, the longitudinal slots 198 that receive the protrusions 319a have a recess 193 at their distal end. The recess 193 of each slot 198 is configured to receive the protrusion 319a that is located within the respective slot 198 when the dose selector member 310 is fully extended from the inner housing 180 in the distal direction, for example upon setting the maximum settable dose. This is further illustrated in **Fig. 50****,** which shows the inner housing 180, the dose selector member 310 and the dosing member 330 with no dose set, and **Fig. 51****,** which shows the inner housing 180, the dose selector member 310 and the dosing member 330 with the maximum dose set.

When the maximum dose is set, the stopping surfaces 338 of the maximum dose stops 337 abut against the limiting surfaces 192 of the maximum stop features 190. Furthermore, the radial protrusions 198a are received within the recesses 193. With the embodiments of the drug delivery device 200 shown in Figs. 48 to 51, the inner housing 180 comprises two maximum stop features 190 that are located opposite to each other with respect to the longitudinal axis 207. Instead of two further maximum stop features 190, the inner housing 180 comprises two longitudinal slots 198 that have the recesses 193 at the distal end. The longitudinal slots 198 with the recesses 193 are also located opposite to each other with respect to the longitudinal axis 207. In the circumferential direction, the inner housing 180 alternately comprises longitudinal slots 198 that feature the limiting surfaces 192 and longitudinal slots 188 that feature the recesses 193.

The radial protrusions 319a and the recesses 193 may serve as a further maximum dose stop mechanism that is provided between the dose selector member 310 and the inner housing 180 and that limits axial movement of the dosing member 330 and the dose selector member 310 upon having set the maximum settable dose. Alternatively or additionally, they may provide locking means that prevent detachment of the dose selector member 310 from the housing 210 after assembly of the drug delivery device 200. For example, the radial protrusions 319a and the recesses 193 may be configured in a way that they do not touch each other upon engagement between the stopping surface 338 and the limiting surfaces 192 but only touch upon further forceful movement of the dose selector member 310 in the distal direction. Alternatively, the radial protrusions 319a and the recesses 193 may be configured to touch essentially simultaneously with the stopping surface 338 touching the limiting surfaces 192.

As can be seen from Figs. 49 and 50, an inner housing 180 that is configured to receive the alternative embodiments of the dose selector member 310 having the radial protrusions 319a may also have four of the tappets 183 and be configured to be used in a drug delivery device 200 that features the alternative embodiment of the coupling part 130 shown in Figs. 46 and 47. Alternatively, such an inner housing 118 may also feature only two of the tappets 184 and be configured to be used with the coupling part 130 described in connection with Figs. 27 to 33.

As further can be seen from Figs. 51, the dose definition mechanism 232 of the drug delivery device 200 having the alternative embodiments of the dose selector member 310 and the inner housing 180 is exemplarily configured as it is described in connection with Figs. 36 and 37 for the first drug delivery device 220 that is configured to expel a maximum dose of the active pharmaceutical ingredient of 1.8 mg.

As it has been described in connection with the first, second and third drug delivery device 220, 222, 225, the clutch mechanisms 234 of the individual drug delivery devices 220, 222, 225 of the individual sets may define a different number of rotational coupling positions in which the first part 235 of the clutch mechanism 234 may be closed to rotationally couple the nut 250 and/or the clutch member 270 to the dosing member 330. These rotational coupling positions are defined by the circumferential positions of the clutch elements 273, 336.

An angular spacing between the rotational coupling positions corresponds to an angular spacing between the dose positions that are settable by rotating the dose setting member 290. With the type of drug delivery device 200 described in connection with Figures 1 to 37 and 44 to 51, the angular spacing between the rotational coupling positions equals the angular spacing between the dose positions. In general, these positions may correspond in a way that the angular spacing between the dose positions is an integer multiple of the angular spacing between the coupling positions. For example, depending on the circumferential positions of the dose stops 35a on the inner surface of the dose selector member 35 of the further drug delivery device 10, the angular spacing between the dose positions defined by the dose stops 35a may be an integer multiple of the rotational coupling positions defined by the clutch elements 34a on the connector 34 and the clutch elements 33a on the snap element 33.

The embodiment of the clutch member 270 of the drug delivery device 200 shown in Figs. 16 and 17 comprises one clutch element 273 for each rotational coupling position. So, in principle, a single clutch element 336 on the dosing member 330 would suffice to define the rotational coupling positions. With alternative embodiments of the clutch member 270, the number of clutch elements 273 may also differ from the number of rotational coupling positions. For example, a number of clutch elements 273 may be smaller than the number of rotational coupling positions per revolution of the dose setting member 290. The number of clutch elements 273 may thereby be smaller by at least one, at least two, such as by one or two, or by more clutch elements 273.

The embodiment of the dosing member 330 shown in Fig. 20 comprises one clutch element 336 for each rotational coupling position. So, in principle, a single clutch element 273 on the clutch member 270 would suffice to define the rotational coupling positions. With alternative embodiments of the dosing member 330, the number of clutch elements 336 may also differ from the number of rotational coupling positions. For example, the number of clutch elements 336 may be smaller than the number of rotational coupling positions per revolution of the dose setting member 290. The number of clutch elements 336 may thereby be smaller by at least one, by at least two, such as by one or two, or by more clutch elements 336.

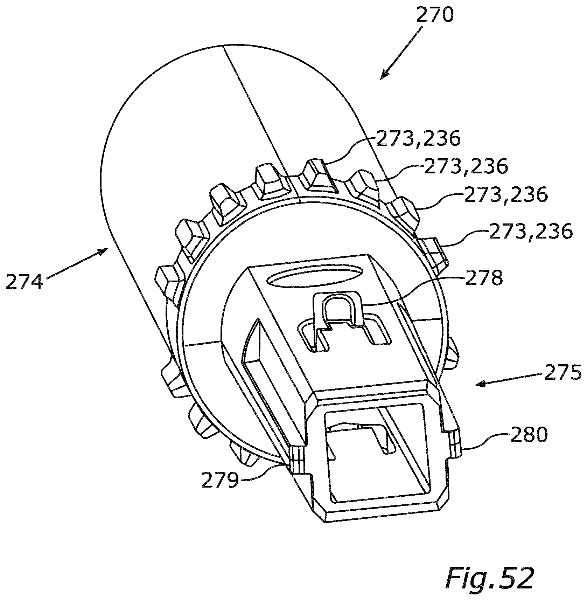

**Fig. 52** shows an alternative embodiment of the clutch member 270 of the drug delivery device 200. As long as no differences are described or apparent from the Figures, the alternative embodiment of the clutch member 270 is configured as it is disclosed in connection with the clutch member 270 described above.

A number of clutch elements 273 of the alternative embodiments of the clutch member 270 is by two smaller than the number of rotational coupling positions. The clutch elements 273 are located next to each other in two groups, wherein each group comprises the same number of clutch elements 273, that is, exemplarily, eight clutch elements 273, and wherein the clutch elements 273 of the individual groups are equally spaced apart from each other. In the gaps between the two groups, a ninth clutch element 273 is missing. The two groups of clutch elements 273 are circumferentially spaced apart from each other by twice the distance between the clutch elements 273 of the individual groups.

The drug delivery devices 10, 200, 220, 222, 225 according to the present disclosure may comprise a balancing weight. The balancing weight may be located at a position offset from the longitudinal axis 207 of the device 10, 200, 220, 222, 225, so that a position of the center of mass of the device 10, 200, 220, 222, 225 is shifted away from the longitudinal axis 207 towards the outer circumferential shell of the device 10, 200, 220, 222, 225. This prevents rolling of the device 10, 200, 220, 222, 225 when it is placed on a flat surface.

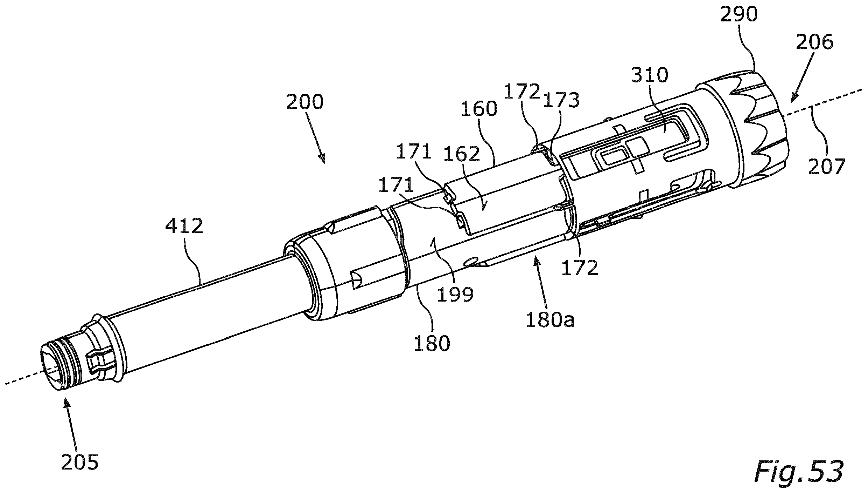

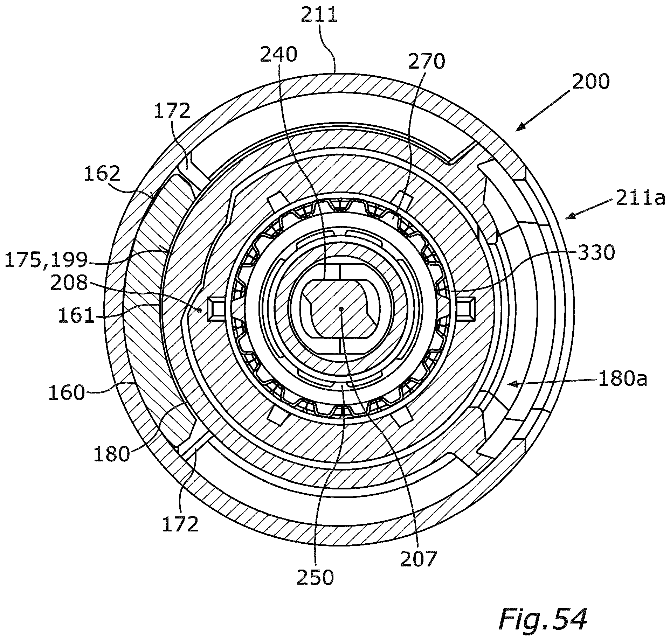

Fig. 53 shows a perspective view of the drug delivery device 200, without the outer housing 211, that is equipped with such a balancing weight 160 and Fig. 54 shows a radial cut perpendicular to the longitudinal axis 207 through the device 200 and the balancing weight 160. The balancing weight 160 is located within the housing 210 of the device 200, namely within the outer housing 211. It is thereby placed between the inner housing 180 and the outer housing 211, as well as between the dosing mechanism 230 and the outer housing 211.

The balancing weight 160 is placed on an outer surface 199 of the inner housing 180. It has a curved bottom surface 161, which faces towards the longitudinal axis 207, and a curved top surface 162, which faces away from the longitudinal axis 207. The bottom surface 161 forms a segment of a circular cylindrical shell with a rotational axis that coincides with the longitudinal axis 207. Likewise, the top surface 162 forms a segment of a circular cylindrical shell with a rotational axis that coincides with the longitudinal axis 207. The bottom and top surfaces 161, 162 are orientated parallel to each other.

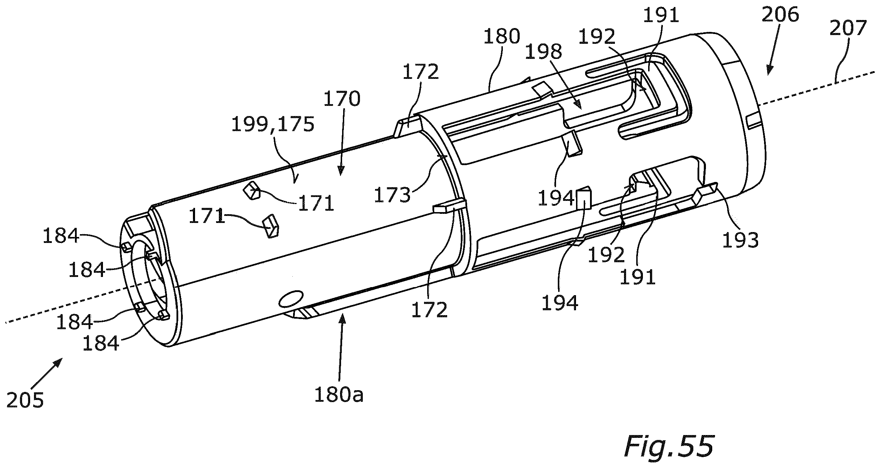

The balancing weight 160 is laid in a seat 170, which is formed on the outer surface 199 of the inner housing 180 and which is, inter alia, depicted in Fig. 55. The seat 170 comprises a support surface 175, which carries the balancing weight 160 and against which the bottom surface 161 of the balancing weight 160 rests. The support surface 175 is formed by the outer surface 199 of the inner housing 180. Furthermore, the seat 170 comprises at least one, namely two, first longitudinal stop elements 171 that delimit the seat 170 towards the proximal end 205 and a second longitudinal stop element 173 that delimits the seat 170 towards the distal end 206. To prevent rotation of the balancing weight 160 in the circumferential direction, the seat 170 comprises two circumferential stop elements 172 that limit the seat 170 in the circumferential direction.

The first longitudinal stop elements 171 are configured as protrusions located on the outer surface 199 of the inner housing 180. The first longitudinal stop elements 171 are spaced apart from each other in the circumferential direction and located at the same axial position along the longitudinal axis 207. The first longitudinal stop elements 171 have an elongated shape that is orientated perpendicular to the longitudinal axis 207.

The second longitudinal stop element 173 is configured as a protrusion that forms a step in the outer surface 199 of the inner housing 180. The second longitudinal stop element 173 runs perpendicular to the longitudinal axis 207 and forms a radial surface that is orientated perpendicular to the longitudinal axis 207.

The circumferential stop elements 172 are configured as individual protrusions located on the outer surface 199 of the inner housing 180. They are placed at the distal end of the seat 170. Furthermore, they are configured as protrusions that extend in the proximal direction from the second longitudinal stop element 173. The longitudinal stop elements 172 have an elongated shape that is orientated parallel to the longitudinal axis 207.

As can be seen from Fig. 54, the seat is covered by the outer housing 211. The balancing weight 160 is configured to abut with its top surface 162 against an inner surface of the outer housing 211. The balancing weight 160 is sandwiched between the inner housing 180 and the outer housing 211. The covered seat 170 forms a cavity in which the balancing weight 160 is inserted. Thereby, the balancing weight 160 is only held in place by the stop elements 171, 172, 173, the support surface 175 and the inner surface of the outer housing 211.

As further can be seen from Fig. 54, the balancing weight 160 causes a center of mass 208 of the drug delivery device 200 to be located away from the longitudinal axis 207 of the device 200 towards the balancing weight 160. The center of mass 208 is located between the longitudinal axis 207 and the balancing weight 160. Furthermore, a distance between the balancing weight 160 and the center of mass 208 is smaller than a distance between the center of mass 208 and the longitudinal axis 207.

The balancing weight 160 and the window in the housing 210, which is exemplarily formed by the window 211a in the outer housing 211 and the window 180a in the inner housing 180, are located at different angular positions with respect to the longitudinal axis 207. In the exemplary embodiment of Fig. 54, the balancing weight 160 and the window in the housing 210 are located at angular positions that differ by 180° and thus correspond to opposite sides of the longitudinal axis 207.

A contact surface of the drug delivery device 200 with the cap 209 removed comprises all surface elements of the drug delivery device 200 that touch a planar surface when rolling the drug delivery device 200 without the cap 209 over the surface. With the drug delivery device 200, the contact surface has a circular cylindrical outer surface that lacks protrusions that would inhibit rolling of the housing 210 when being placed on a flat surface. Due to the balancing weight 160, the drug delivery device 200 will rotate on a flat surface until it assumes a stable position and the center of mass 280 is located between the surface and the longitudinal axis 207.

In the stable position, the window in the housing 210 is located on the upper side of the drug delivery device 200 that faces away from the surface that the drug delivery device 200 is placed on. With other embodiments of the device 200 and other placings of the balancing weight 160, the window could also be located on another side of the drug delivery device 200, for example on a lateral side.

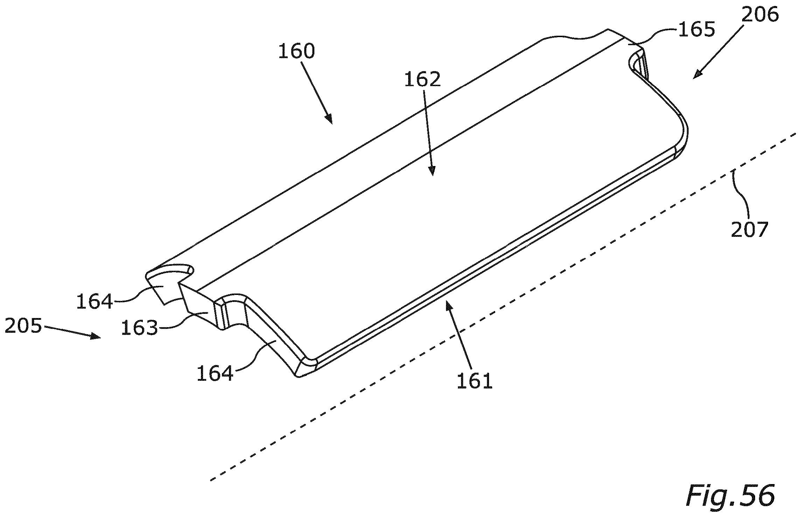

**Fig. 56** shows a perspective view of the balancing weight 160. It is configured as a metal part and has a higher density than the plastic parts of the dosing mechanism 230 and the inner housing 180.

The balancing weight 160 is curved around the longitudinal axis 207 of the drug delivery device 200. It is symmetrical with respect to its center plane, which is orientated perpendicular to the longitudinal axis 207.

The balancing weight 160 has a first protrusion 163 at one longitudinal end surface and a second protrusion 165 at an opposing longitudinal end surface. When being inserted into the seat 170, one of the protrusions 163, 165, for example the first protrusion 163 as shown in Fig. 56, is placed as a proximal protrusion in between the first longitudinal stop elements 171. Two front faces 164 of the balancing weight 160 that radially extend from the proximal protrusion and which are set back along the longitudinal axis 207 with respect to the proximal protrusion are configured to abut against the first longitudinal stop elements 171. The other one of the protrusions 163, 165, for example the second protrusion 165 shown in Fig. 56, is then configured to abut as a distal protrusion against the second longitudinal stop element 173. A width of the balancing weight 160 perpendicular to the longitudinal axis 207 is adapted to allow the balancing weight 162 being placed in between the circumferential stop elements 172.

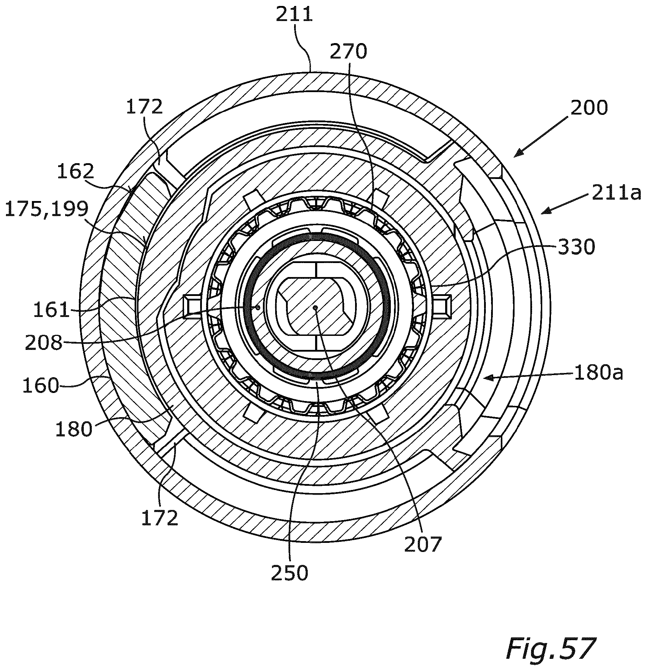

With other embodiments, the distance between the balancing weight 160 and the center of mass 208 may also be smaller than the distance between the center of mass 208 and the longitudinal axis 207, as can be seen from Fig. 57, which shows a radial cut perpendicular to the longitudinal axis 207 through an alternative embodiment of the drug delivery device 200 with the balancing weight 160. Positioning the center of mass 208 at a smaller distance from the longitudinal axis 207 than from the balancing weight 160 allows to use a comparatively small balancing weight 160 while still shifting the center of mass away from the longitudinal axis 207.

The sectional views of Figs. 54 and 57 only schematically depict the radial position of the center of mass 208. With the drug delivery device 200, the longitudinal position of the center of mass 208 may not be located within the sectional plane depicted in Figs. 54 and 57 but in other cross-sectional planes. The longitudinal position of the center of mass 208 may, for example, be positioned distally from the longitudinal center of the window 211a within the outer housing 211 along the longitudinal axis 207 or it may be positioned proximally from the longitudinal center of the window 211a within the outer housing 211 along the longitudinal axis 207.

The present disclosure is also generally directed at the dose definition mechanisms 232 of the drug delivery devices 10, 200, 220, 222, 225. The construction and details of these dose definition mechanisms 232 are independent of other constructional details of the drug delivery devices 10, 200, 220, 222, 225, such as the friction reduction mechanisms 370, 380, the maximum and/or minimum dose stops 35a, 35b, 35c, the connection means 414, 424, 434, 444, 510, 511, 520, 530, the resetting mechanism 100 or the balancing weight 160.

### Reference numeral list

- 1: cap
- 2: cartridge holder
- 3: outer housing
- 3a: window
- 4: needle
- 5: hub
- 6: cannula
- 8: cartridge
- 8a: sealing means
- 9: piston
- 10: further drug delivery device
- 30: dosing mechanism
- 31: dose setting member
- 32: clutch member
- 32: ridge
- 33: snap element
- 33a: clutch elements
- 33c: flexible arm
- 33d: protrusion
- 33f: further protusion
- 34: connector
- 34a: clutch elements
- 34b: ridges
- 35: dose selector member
- 35a: dose stop
- 35b: maximum stop feature
- 35c: minimum stop feature
- 36: nut
- 37: splined connection
- 38: dose sleeve
- 40: optical marker
- 41: driver
- 42: piston rod
- 42a: disc
- 43: piston guide
- 60: outer thread
- 61: dose button
- 67: thread
- 63: stop feature
- 64: protrusion
- 81: drug compartment
- 82: annular rim
- 83: distal surface
- 85: annular detent
- 90: rotational biasing member
- 100: resetting mechanism
- 110: resetting element
- 111: gripping zone
- 112: reception area
- 113: inner surface
- 114: opening
- 115: cartridge cavity
- 116: guiding structure
- 116a: front surface
- 116b: back surface
- 117: contact structure
- 119: stop
- 120: engagement features
- 130: coupling part/insert
- 134: coupling site
- 135: engagement features
- 136: notch
- 137: first locking structure
- 138: protrusions
- 139: slot
- 139a: recess
- 140: second locking structure
- 150: biasing element
- 160: balancing weight
- 161: bottom surface
- 162: top surface
- 163: proximal protrusion
- 164: front faces
- 165: distal protrusion
- 170: seat
- 171: longitudinal stop element
- 172: circumferential stop element
- 173: longitudinal stop element
- 175: support surface
- 180: inner housing
- 180a: window
- 181: proximal part
- 182: distal part
- 183: inner sleeve
- 184: tappet
- 185: dose thread
- 186: drive thread
- 187: groove
- 188: further groove
- 189: housing cavity
- 190: maximum stop feature
- 191: hook
- 192: limiting surface
- 193: recess
- 194: protrusion
- 195: bulge
- 196: zero stop feature
- 197: zero stop surface
- 198: longitudinal slot
- 199: outer surface
- 200: drug delivery device
- 205: proximal end
- 206: distal end
- 207: longitudinal axis
- 208: center of mass
- 209: cap
- 210: housing
- 211: outer housing
- 211a: window
- 213: collar
- 214: detents
- 216: detent
- 218: groove
- 220: first drug delivery device
- 221: first housing
- 222: second drug delivery device
- 223: second housing
- 225: third drug delivery device
- 226: third housing
- 230: dosing mechanism
- 232: dose definition mechanism
- 234: clutch mechanism
- 235: first part
- 236: second part
- 237: clutch mechanism
- 238: first part
- 239: second part
- 240: piston rod
- 241: thread
- 242: plunger disc
- 243: stop feature
- 244: disc connector
- 250: nut
- 251: proximal part
- 252: distal part
- 253: proximal protrusion
- 254: longitudinal groove
- 255: annular detent
- 256: thread
- 270: clutch member
- 271: longitudinal ridges
- 273: clutch elements
- 274: proximal part
- 275: distal part
- 277: connection
- 278: snap hook
- 279: first ridge
- 280: second ridge
- 290: dose setting member
- 292: elastic elements
- 294: clutch elements
- 295: recess
- 296: opening
- 297: first longitudinal groove
- 298: second longitudinal groove
- 308: biasing member
- 310: dose selector member
- 311: distal part
- 312: functional features
- 314: contact surface
- 315: ridge
- 316: further ridge
- 317: proximal part
- 318: connector
- 319: flexible member
- 319a: protrusion
- 320: detent
- 322: inner wall
- 323: opening
- 330: dosing member
- 331: optical marker
- 332: proximal part
- 333: distal part
- 334: threaded connection
- 335: outer thread
- 336: clutch elements
- 337: maximum dose stop
- 338: stopping surface
- 340: zero dose stop
- 341: groove
- 343: connector
- 344: annular ridge
- 346: distal end surface
- 350: driver
- 351: proximal part
- 352: threaded connection
- 353: thread
- 354: connection
- 356: flexible arm
- 358: front surface
- 359: distal part
- 360: spline
- 370: first bearing element
- 371: distal disc
- 372: holder
- 373: proximal disc
- 375: ball
- 380: second bearing element
- 402: needle connector
- 404: connector
- 405: contact surface
- 406: biasing element
- 407: contact surface
- 408: stop
- 409: ridge
- 410: dispensing unit
- 412: cartridge holder
- 413: cartridge cavity
- 414: connection means
- 420: first dispensing unit
- 422: first cartridge holder
- 424: first connection means
- 430: second dispensing unit
- 432: second cartridge holder
- 434: second connection means
- 440: third dispensing unit
- 442: third cartridge holder
- 444: third connection means
- 450: contact feature
- 501: ridge
- 502: valleys
- 510: connection means
- 511: first connection means
- 520: second connection means
- 530: third connection means
- P₁: first pitch
- w₁: first width
- h₁: first height
- CD₁: first core diameter
- D₁: first outer diameter
- A₁: first angle
- P₂: second pitch
- w₂: second width
- h₂: second height
- CD₂: second core diameter
- D₂: second outer diameter
- A₂: second angle
- P₃: third pitch
- w₃: third width
- h₃: third height
- CD₃: third core diameter
- D₃: third outer diameter
- A₃: third angle

## Claims

1. Drug delivery device (10, 200, 220, 222, 225) having
a housing (3, 43, 210, 221, 223, 226) with a longitudinal axis (207),
a dose setting member (31, 290) that is actuatable by a user and rotatable around the longitudinal axis (207) to set a dose to be delivered by the drug delivery device (10, 200, 220, 222, 225),
a piston rod (42, 240) that is configured to be axially advanced in a proximal direction to deliver the set dose, and
a dosing member (330) for defining the axial advancement of the piston rod (42, 240) upon delivery of the set dose,
wherein the dosing member (330) is axially movable along the longitudinal axis (207) and rotationally movable around the longitudinal axis (207) during dose setting,
wherein the dosing member (330) is rotationally fixed to the dose setting member (31, 290) during dose setting,
wherein the dosing member (330) comprises a maximum dose stop (337), wherein the maximum dose stop (337) is configured to engage with a maximum stop feature (190) to limit movement of the dosing member (330) with respect to the housing (3, 43, 210, 221, 223, 226) when having set a maximum dose,
wherein the maximum stop feature (190) is provided at the housing (3, 43, 210, 221, 223, 226),
**characterised in that** the drug delivery device (10, 200, 220, 222, 225) comprises a dose definition mechanism (232) for defining rotational dose positions of the dose setting member (31, 290) with respect to the housing (3, 43, 210, 221, 223, 226),
wherein the dose setting member (31, 290) is connected to the housing (3, 43, 210, 221, 223, 226) via a dose selector member (35, 310),
wherein the dose selector member (35, 310) is rotationally fixed and axially movable with respect to the housing (3, 43, 210, 221, 223, 226),
wherein the dose definition mechanism (232) acts between the dose selector member (35, 310) and the dose setting member (31, 290) or wherein the dose definition mechanism (232) acts between the dose selector member (35, 310) and the dosing member (330).

2. The drug delivery device (10, 200, 220, 222, 225) according to claim 1,
wherein the dosing member (330) is rotationally movable with respect to the dose setting member (31, 290) during dose delivery, and/or
wherein the dosing member (330) is threadedly connected to the housing (3, 43, 210, 221, 223, 226), for example via an outer thread (335) provided on the dosing member (330) and a corresponding inner thread (185) provided on the housing (3, 43, 210, 221, 223, 226).

3. The drug delivery device (10, 200, 220, 222, 225) according to one of the preceding claims,
wherein the maximum dose stop (337) comprises a stopping surface (338) that is configured to axially abut the maximum stop feature (190) of the housing (3, 43, 210, 221, 223, 226) upon setting the maximum dose,
wherein, for example, the stopping surface (338) is orientated perpendicular to the longitudinal axis (207), and/or the stopping surface (338) is an annular surface surrounding the longitudinal axis (207).

4. The drug delivery device (10, 200, 220, 222, 225) according to one of the preceding claims,
wherein the maximum dose stop (337) protrudes from an outer surface of the dosing member (31, 290), and/or
wherein the maximum stop feature (190) protrudes from an inner surface of the housing (3, 43, 210, 221, 223, 226), and/or
wherein the maximum dose stop (337) is spaced apart from a distal end of the dosing member (330), and/or
wherein the maximum stop feature (190) of the housing (3, 43, 210, 221, 223, 226) has a limiting surface (192) that is orientated perpendicular to the longitudinal axis (207) and the maximum dose stop (337) engages with the limiting surface (192) upon setting the maximum dose.

5. The drug delivery device (10, 200, 220, 222, 225) according to one of the preceding claims,
wherein the maximum stop feature (190) of the housing (3, 43, 210, 221, 223, 226) is provided on a flexible element (191) that is configured to snap over the maximum dose stop (337) of the dosing member (330) upon assembly of the drug delivery device (10, 200, 220, 222, 225),
wherein, for example, the flexible element (191) rests against a backing element, such as an outer housing (211) that surrounds an inner housing (1806) having the flexible element, after assembly of the drug delivery device (10, 200, 220, 222, 225) to prevent disengagement of the maximum stop feature (190) from the maximum dose stop (337).

6. The drug delivery device (10, 200, 220, 222, 225) according to one of the preceding claims,
wherein the dosing member (330) comprises a zero dose stop (340),
wherein the zero dose stop (340) is configured to engage with a zero stop feature (196) to limit movement of the dosing member (330) with respect to the housing (3, 43, 210, 221, 223, 226) upon the dosing member (330) reaching a zero dose position,
wherein the zero stop feature (196) is provided at the housing (3, 43, 210, 221, 223, 226),
wherein, for example, the zero dose stop (340) engages with the zero stop feature (196) in a contact plane that is angled with respect to a radial plane perpendicular to the longitudinal axis (207) and, for example, the contact plane is orientated perpendicular to the radial plane.

7. The drug delivery device (10, 200, 220, 222, 225) according to claim 6,
wherein the zero dose stop (340) is provided at a proximal end of the dosing member (330) and/or
wherein the zero stop feature (196) is provided at a proximal end of a housing cavity (189) of the housing (3, 43, 210, 221, 223, 226), and/or
wherein the zero stop feature (196) and the maximum stop feature (190) are provided at the same structural element of the housing (3, 43, 210, 221, 223, 226), for example at an inner housing (180) and, for example, the structural element comprises a dose thread (185) that threadedly engages with the dosing member (330).

8. The drug delivery device (10, 200, 220, 222, 225) according to one of the preceding claims,
wherein the dose selector member (35, 310) is axially fixed with respect to the dosing member (330).

9. The drug delivery device (10, 200, 220, 222, 225) according to one of the preceding claims,
wherein the dose selector member (35, 310) is connected to the housing (3, 43, 210, 221, 223, 226) via a connection (187, 188, 315, 316) that allows the dose selector member (35, 310) to be mounted to the housing (3, 43, 210, 221, 223, 226) only in rotational orientations that ensure that the dose setting member (31, 290) is set to a dose position upon engagement of the maximum dose stop (337) with the maximum stop feature (190),
wherein, for example, the connection allows a single rotational orientation only.

10. The drug delivery device (10, 200, 220, 222, 225) according to one of claims 9,
wherein the connection (187, 188, 315, 316) comprises a splined connection that allows axial movement and prevents rotational movement of the dose selector member (35, 310) with respect to the housing (3, 43, 210, 221, 223, 226),
wherein the splined connection comprises a set of coding splines wherein the coding splines have respective dimensions that differ from each other, for example in width and/or height,
wherein, for example, the splined connection comprises a single coding spline (187, 315) that differs from the remaining splines (188, 316) of the connection (187, 188, 315, 316).

11. The drug delivery device (10, 200, 220, 222, 225) according to one of the preceding claims,
wherein the dosing member (330) is coupled to the piston rod (42, 240) via an advancement mechanism that translates axial movement of the dosing member (330) into the axial advancement of the piston rod (42, 240) during dose delivery so that the axial movement of the dosing member (330) during dose delivery causes the piston rod (42, 240) to axially advance in the proximal direction,
wherein, for example, the advancement mechanism is configured as a gearing mechanism that reduces the axial movement of the dosing member (330) to a smaller axial advancement of the piston rod (42, 240).

12. The drug delivery device (10, 200, 220, 222, 225) according to one of claims 11,
wherein the piston rod (42, 240) is rotationally fixed to the housing (3, 43, 210, 221, 223, 226),
wherein the advancement mechanism comprises a nut (36, 250) that is coupled between the piston rod (42, 240) and the dosing member (330),
wherein the nut (36, 250) is threadedly connected to the piston rod (42, 240),
wherein the nut (36, 250) is rotationally fixed with respect to the dosing member (330) and rotatable with respect to the housing (3, 43, 210, 221, 223, 226) during dose setting,
wherein the nut (36, 250) is rotatable with respect to the dosing member (330) and rotationally fixed with respect to the housing (3, 43, 210, 221, 223, 226) during dose delivery.

13. Drug delivery device (10, 200, 220, 222, 225) having
a housing (3, 43, 210, 221, 223, 226) with a longitudinal axis (207),
a dose setting member (31, 290) that is actuatable by a user and rotatable around the longitudinal axis (207) to set a dose to be delivered by the drug delivery device (10, 200, 220, 222, 225),
a piston rod (42, 240) that is configured to be axially advanced in a proximal direction to deliver the set dose, and
a dosing member (330) for defining the axial advancement of the piston rod (42, 240) upon delivery of the set dose,
wherein the dosing member (330) is axially movable along the longitudinal axis (207) and rotationally movable around the longitudinal axis (207) during dose setting,
wherein the dosing member (330) is rotationally fixed to the dose setting member (31, 290) during dose setting,
wherein the dosing member (330) comprises a zero dose stop (340),
wherein the zero dose stop (340) is configured to engage with a zero stop feature (196) to limit movement of the dosing member (330) with respect to the housing (3, 43, 210, 221, 223, 226) upon setting a zero dose,
wherein the zero stop feature (196) is provided at the housing (3, 43, 210, 221, 223, 226),
**characterised in that** the drug delivery device (10, 200, 220, 222, 225) comprises a dose definition mechanism (232) for defining rotational dose positions of the dose setting member (31, 290) with respect to the housing (3, 43, 210, 221, 223, 226),
wherein the dose setting member (31, 290) is connected to the housing (3, 43, 210, 221, 223, 226) via a dose selector member (35, 310),
wherein the dose selector member (35, 310) is rotationally fixed and axially movable with respect to the housing (3, 43, 210, 221, 223, 226),
wherein the dose definition mechanism (232) acts between the dose selector member (35, 310) and the dose setting member (31, 290) or wherein the dose definition mechanism (232) acts between the dose selector member (35, 310) and the dosing member (330).

14. The drug delivery device (10, 200, 220, 222, 225) according to claim 13,
wherein the dosing member (330) is rotationally movable with respect to the dose setting member (31, 290) during dose delivery, and/or
wherein the zero dose stop (340) engages with the zero stop feature (196) in a contact plane that is angled with respect to a radial plane perpendicular to the longitudinal axis (207) and, for example, the contact plane is orientated perpendicular to the radial plane, and/or
wherein the zero dose stop (340) of the dosing member (330) comprises a stop surface that is configured to abut against a corresponding stop surface of the housing (3, 43, 210, 221, 223, 226), and/or.
wherein the zero dose stop (340) is provided at a proximal end of the dosing member (330) and/or
wherein the zero stop feature (196) is provided at a proximal end of a housing cavity (189) of the housing (3, 43, 210, 221, 223, 226).

15. The drug delivery device (10, 200, 220, 222, 225) according to one of the preceding claims,
wherein the dose definition mechanism (232) acts between the dose selector member (35, 310) and the dose setting member (31, 290) via additional elements that are located between the dose selector member (35, 310) and the dose setting member (31, 290), such as via the dosing member (330).

16. The drug delivery device (10, 200, 220, 222, 225) according to one of claims 1 to 14,
wherein the dose definition mechanism (232) is realized by direct engagement of the dose setting member (31, 290) or a part permanently fixed to the dose setting member (31, 290) with the dose selector member (35, 310) or a part permanently fixed to the dose selector member (35, 310).

## Patentansprüche

1. Medikamenten-Verabreichungsvorrichtung (10, 200, 220, 222, 225), umfassend:
ein Gehäuse (3, 43, 210, 221, 223, 226) mit einer Längsachse (207),
ein Dosiseinstellelement (31, 290), das von einem Benutzer betätigbar ist und um die Längsachse (207) drehbar ist, um eine von der Medikamenten-Verabreichungsvorrichtung (10, 200, 220, 222, 225) zu verabreichende Dosis einzustellen,
eine Kolbenstange (42, 240), die konfiguriert ist, um in einer proximalen Richtung axial vorgeschoben zu werden, um die eingestellte Dosis zu verabreichen, und
ein Dosierelement (330) zum Definieren des axialen Vorschubs der Kolbenstange (42, 240) bei Verabreichung der eingestellten Dosis,
wobei das Dosierelement (330) während der Dosiseinstellung entlang der Längsachse (207) axial bewegbar und um die Längsachse (207) rotatorisch bewegbar ist,
wobei das Dosierelement (330) während der Dosiseinstellung an dem Dosiseinstellelement (31, 290) rotatorisch fixiert ist,
wobei das Dosierelement (330) einen Maximaldosisanschlag (337) umfasst,
wobei der Maximaldosisanschlag (337) konfiguriert ist, um mit einem Maximalanschlagmerkmal (190) in Eingriff zu kommen, um die Bewegung des Dosierelements (330) in Bezug auf das Gehäuse (3, 43, 210, 221, 223, 226) zu begrenzen, wenn eine Maximaldosis eingestellt wurde,
wobei das Maximalanschlagmerkmal (190) an dem Gehäuse (3, 43, 210, 221, 223, 226) vorgesehen ist,
**dadurch gekennzeichnet, dass** die Medikamenten-Verabreichungsvorrichtung (10, 200, 220, 222, 225) einen Dosisdefinitionsmechanismus (232) zum Definieren von Drehdosispositionen des Dosiseinstellelements (31, 290) in Bezug auf das Gehäuse (3, 43, 210, 221, 223, 226) umfasst,
wobei das Dosiseinstellelement (31, 290) über ein Dosisauswahlelement (35, 310) mit dem Gehäuse (3, 43, 210, 221, 223, 226) verbunden ist,
wobei das Dosisauswahlelement (35, 310) in Bezug auf das Gehäuse (3, 43, 210, 221, 223, 226) rotatorisch fixiert und axial bewegbar ist,
wobei der Dosisdefinitionsmechanismus (232) zwischen dem Dosisauswahlelement (35, 310) und dem Dosiseinstellelement (31, 290) wirkt oder
wobei der Dosisdefinitionsmechanismus (232) zwischen dem Dosisauswahlelement (35, 310) und dem Dosierelement (330) wirkt.

2. Medikamenten-Verabreichungsvorrichtung (10, 200, 220, 222, 225) nach Anspruch 1,
wobei das Dosierelement (330) während der Dosisverabreichung in Bezug auf das Dosiseinstellelement (31, 290) rotatorisch bewegbar ist, und/oder
wobei das Dosierelement (330) über ein Gewinde mit dem Gehäuse (3, 43, 210, 221, 223, 226) verbunden ist, zum Beispiel über ein Außengewinde (335), das an dem Dosierelement (330) vorgesehen ist, und ein entsprechendes Innengewinde (185), das an dem Gehäuse (3, 43, 210, 221, 223, 226) vorgesehen ist.

3. Medikamenten-Verabreichungsvorrichtung (10, 200, 220, 222, 225) nach einem der vorhergehenden Ansprüche,
wobei der Maximaldosisanschlag (337) eine Anschlagfläche (338) umfasst, die konfiguriert ist, um beim Einstellen der Maximaldosis axial an dem Maximalanschlagmerkmal (190) des Gehäuses (3, 43, 210, 221, 223, 226) anzuliegen,
wobei zum Beispiel die Anschlagfläche (338) senkrecht zu der Längsachse (207) ausgerichtet ist, und/oder die Anschlagfläche (338) eine ringförmige Fläche ist, die die Längsachse (207) umgibt.

4. Medikamenten-Verabreichungsvorrichtung (10, 200, 220, 222, 225) nach einem der vorhergehenden Ansprüche,
wobei der Maximaldosisanschlag (337) von einer Außenfläche des Dosierelements (31, 290) vorsteht, und/oder
wobei das Maximalanschlagmerkmal (190) von einer Innenfläche des Gehäuses (3, 43, 210, 221, 223, 226) vorsteht, und/oder
wobei der Maximaldosisanschlag (337) von einem distalen Ende des Dosierelements (330) beabstandet ist, und/oder
wobei das Maximalanschlagmerkmal (190) des Gehäuses (3, 43, 210, 221, 223, 226) eine Begrenzungsfläche (192) aufweist, die senkrecht zu der Längsachse (207) ausgerichtet ist, und der Maximaldosisanschlag (337) mit der Begrenzungsfläche (192) beim Einstellen der Maximaldosis in Eingriff kommt.

5. Medikamenten-Verabreichungsvorrichtung (10, 200, 220, 222, 225) nach einem der vorhergehenden Ansprüche,
wobei das Maximalanschlagmerkmal (190) des Gehäuses (3, 43, 210, 221, 223, 226) an einem flexiblen Element (191) bereitgestellt ist, das konfiguriert ist, um über den Maximaldosisanschlag (337) des Dosierelements (330) beim Zusammenbau der Medikamenten-Verabreichungsvorrichtung (10, 200, 220, 222, 225) zu schnappen,
wobei zum Beispiel das flexible Element (191) nach dem Zusammenbau der Medikamenten-Verabreichungsvorrichtung (10, 200, 220, 222, 225) an einem Stützelement, wie etwa einem äußeren Gehäuse (211), das ein inneres Gehäuse (1806) umgibt, das das flexible Element aufweist, anliegt, um ein Lösen des Maximalanschlagmerkmals (190) von dem Maximaldosisanschlag (337) zu verhindern.

6. Medikamenten-Verabreichungsvorrichtung (10, 200, 220, 222, 225) nach einem der vorhergehenden Ansprüche,
wobei das Dosierelement (330) einen Nulldosisanschlag (340) umfasst,
wobei der Nulldosisanschlag (340) konfiguriert ist, um mit einem Nullanschlagmerkmal (196) in Eingriff zu kommen, um die Bewegung des Dosierelements (330) in Bezug auf das Gehäuse (3, 43, 210, 221, 223, 226) zu begrenzen, wenn das Dosierelement (330) eine Nulldosisposition erreicht,
wobei das Nullanschlagmerkmal (196) an dem Gehäuse (3, 43, 210, 221, 223, 226) vorgesehen ist,
wobei zum Beispiel der Nulldosisanschlag (340) mit dem Nullanschlagmerkmal (196) in einer Kontaktebene in Eingriff kommt, die in Bezug auf eine radiale Ebene senkrecht zu der Längsachse (207) angewinkelt ist, und zum Beispiel die Kontaktebene senkrecht zu der radialen Ebene ausgerichtet ist.

7. Medikamenten-Verabreichungsvorrichtung (10, 200, 220, 222, 225) nach Anspruch 6,
wobei der Nulldosisanschlag (340) an einem proximalen Ende des Dosierelements (330) vorgesehen ist und/oder
wobei das Nullanschlagmerkmal (196) an einem proximalen Ende eines Gehäusehohlraums (189) des Gehäuses (3, 43, 210, 221, 223, 226) vorgesehen ist, und/oder
wobei das Nullanschlagmerkmal (196) und das Maximalanschlagmerkmal (190) an demselben Bauelement des Gehäuses (3, 43, 210, 221, 223, 226) vorgesehen sind, zum Beispiel an einem Innengehäuse (180), und zum Beispiel das Bauelement ein Dosisgewinde (185) umfasst, das über ein Gewinde mit dem Dosierelement (330) in Eingriff kommt.

8. Medikamenten-Verabreichungsvorrichtung (10, 200, 220, 222, 225) nach einem der vorhergehenden Ansprüche,
wobei das Dosisauswahlelement (35, 310) in Bezug auf das Dosierelement (330) axial fixiert ist.

9. Medikamenten-Verabreichungsvorrichtung (10, 200, 220, 222, 225) nach einem der vorhergehenden Ansprüche,
wobei das Dosisauswahlelement (35, 310) mit dem Gehäuse (3, 43, 210, 221, 223, 226) über eine Verbindung (187, 188, 315, 316) verbunden ist, die es dem Dosisauswahlelement (35, 310) ermöglicht, an dem Gehäuse (3, 43, 210, 221, 223, 226) nur in Drehorientierungen montiert zu werden, die sicherstellen, dass das Dosiseinstellelement (31, 290) auf eine Dosisposition eingestellt wird, wenn der Maximaldosisanschlag (337) mit dem Maximalanschlagmerkmal (190) in Eingriff kommt,
wobei zum Beispiel die Verbindung nur eine einzige Drehorientierung ermöglicht.

10. Medikamenten-Verabreichungsvorrichtung (10, 200, 220, 222, 225) nach Anspruch 9,
wobei die Verbindung (187, 188, 315, 316) eine Keilverbindung umfasst, die eine axiale Bewegung ermöglicht und eine Drehbewegung des Dosisauswahlelements (35, 310) in Bezug auf das Gehäuse (3, 43, 210, 221, 223, 226) verhindert,
wobei die Keilverbindung einen Satz von Codierkeilen umfasst, wobei die Codierkeile jeweilige Abmessungen aufweisen, die sich voneinander unterscheiden, zum Beispiel in Breite und/oder Höhe,
wobei zum Beispiel die Keilverbindung einen einzigen Codierkeil (187, 315) umfasst, der sich von den verbleibenden Keilen (188, 316) der Verbindung (187, 188, 315, 316) unterscheidet.

11. Medikamenten-Verabreichungsvorrichtung (10, 200, 220, 222, 225) nach einem der vorhergehenden Ansprüche,
wobei das Dosierelement (330) mit der Kolbenstange (42, 240) über einen Vorschubmechanismus gekoppelt ist, der eine axiale Bewegung des Dosierelements (330) in den axialen Vorschub der Kolbenstange (42, 240) während der Dosisverabreichung umsetzt, so dass die axiale Bewegung des Dosierelements (330) während der Dosisverabreichung bewirkt, dass die Kolbenstange (42, 240) in der proximalen Richtung axial vorgeschoben wird,
wobei zum Beispiel der Vorschubmechanismus als ein Getriebemechanismus konfiguriert ist, der die axiale Bewegung des Dosierelements (330) auf einen kleineren axialen Vorschub der Kolbenstange (42, 240) reduziert.

12. Medikamenten-Verabreichungsvorrichtung (10, 200, 220, 222, 225) nach Anspruch 11,
wobei die Kolbenstange (42, 240) an dem Gehäuse (3, 43, 210, 221, 223, 226) rotatorisch fixiert ist,
wobei der Vorschubmechanismus eine Mutter (36, 250) umfasst, die zwischen der Kolbenstange (42, 240) und dem Dosierelement (330) gekoppelt ist,
wobei die Mutter (36, 250) über ein Gewinde mit der Kolbenstange (42, 240) verbunden ist,
wobei die Mutter (36, 250) während der Dosiseinstellung in Bezug auf das Dosierelement (330) rotatorisch fixiert und in Bezug auf das Gehäuse (3, 43, 210, 221, 223, 226) drehbar ist,
wobei die Mutter (36, 250) während der Dosisverabreichung in Bezug auf das Dosierelement (330) drehbar und in Bezug auf das Gehäuse (3, 43, 210, 221, 223, 226) rotatorisch fixiert ist.

13. Medikamenten-Verabreichungsvorrichtung (10, 200, 220, 222, 225), umfassend:
ein Gehäuse (3, 43, 210, 221, 223, 226) mit einer Längsachse (207),
ein Dosiseinstellelement (31, 290), das von einem Benutzer betätigbar ist und um die Längsachse (207) drehbar ist, um eine von der Medikamenten-Verabreichungsvorrichtung (10, 200, 220, 222, 225) zu verabreichende Dosis einzustellen,
eine Kolbenstange (42, 240), die konfiguriert ist, um in einer proximalen Richtung axial vorgeschoben zu werden, um die eingestellte Dosis zu verabreichen, und
ein Dosierelement (330) zum Definieren des axialen Vorschubs der Kolbenstange (42, 240) bei Verabreichung der eingestellten Dosis,
wobei das Dosierelement (330) während der Dosiseinstellung entlang der Längsachse (207) axial bewegbar und um die Längsachse (207) rotatorisch bewegbar ist,
wobei das Dosierelement (330) während der Dosiseinstellung an dem Dosiseinstellelement (31, 290) rotatorisch fixiert ist,
wobei das Dosierelement (330) einen Nulldosisanschlag (340) umfasst,
wobei der Nulldosisanschlag (340) konfiguriert ist, um mit einem Nullanschlagmerkmal (196) in Eingriff zu kommen, um die Bewegung des Dosierelements (330) in Bezug auf das Gehäuse (3, 43, 210, 221, 223, 226) beim Einstellen einer Nulldosis zu begrenzen,
wobei das Nullanschlagmerkmal (196) an dem Gehäuse (3, 43, 210, 221, 223, 226) vorgesehen ist,
**dadurch gekennzeichnet, dass** die Medikamenten-Verabreichungsvorrichtung (10, 200, 220, 222, 225) einen Dosisdefinitionsmechanismus (232) zum Definieren von Drehdosispositionen des Dosiseinstellelements (31, 290) in Bezug auf das Gehäuse (3, 43, 210, 221, 223, 226) umfasst,
wobei das Dosiseinstellelement (31, 290) über ein Dosisauswahlelement (35, 310) mit dem Gehäuse (3, 43, 210, 221, 223, 226) verbunden ist,
wobei das Dosisauswahlelement (35, 310) in Bezug auf das Gehäuse (3, 43, 210, 221, 223, 226) rotatorisch fixiert und axial bewegbar ist,
wobei der Dosisdefinitionsmechanismus (232) zwischen dem Dosisauswahlelement (35, 310) und dem Dosiseinstellelement (31, 290) wirkt oder
wobei der Dosisdefinitionsmechanismus (232) zwischen dem Dosisauswahlelement (35, 310) und dem Dosierelement (330) wirkt.

14. Medikamenten-Verabreichungsvorrichtung (10, 200, 220, 222, 225) nach Anspruch 13,
wobei das Dosierelement (330) während der Dosisverabreichung in Bezug auf das Dosiseinstellelement (31, 290) rotatorisch bewegbar ist, und/oder
wobei der Nulldosisanschlag (340) mit dem Nullanschlagmerkmal (196) in einer Kontaktebene in Eingriff kommt, die in Bezug auf eine radiale Ebene senkrecht zu der Längsachse (207) angewinkelt ist, und zum Beispiel die Kontaktebene senkrecht zu der radialen Ebene ausgerichtet ist, und/oder
wobei der Nulldosisanschlag (340) des Dosierelements (330) eine Anschlagfläche umfasst, die konfiguriert ist, um an einer entsprechenden Anschlagfläche des Gehäuses (3, 43, 210, 221, 223, 226) anzuliegen,
und/oder
wobei der Nulldosisanschlag (340) an einem proximalen Ende des Dosierelements (330) vorgesehen ist und/oder
wobei das Nullanschlagmerkmal (196) an einem proximalen Ende eines Gehäusehohlraums (189) des Gehäuses (3, 43, 210, 221, 223, 226) vorgesehen ist.

15. Medikamenten-Verabreichungsvorrichtung (10, 200, 220, 222, 225) nach einem der vorhergehenden Ansprüche,
wobei der Dosisdefinitionsmechanismus (232) zwischen dem Dosisauswahlelement (35, 310) und dem Dosiseinstellelement (31, 290) über zusätzliche Elemente wirkt, die sich zwischen dem Dosisauswahlelement (35, 310) und dem Dosiseinstellelement (31, 290) befinden, wie zum Beispiel über das Dosierelement (330).

16. Medikamenten-Verabreichungsvorrichtung (10, 200, 220, 222, 225) nach einem der Ansprüche 1 bis 14,
wobei der Dosisdefinitionsmechanismus (232) durch direkten Eingriff des Dosiseinstellelements (31, 290) oder eines Teils, das dauerhaft an dem Dosiseinstellelement (31, 290) befestigt ist, mit dem Dosisauswahlelement (35, 310) oder einem Teil, das dauerhaft an dem Dosisauswahlelement (35, 310) befestigt ist, realisiert wird.

## Revendications

1. Dispositif de distribution de médicament (10, 200, 220, 222, 225) ayant un boîtier (3, 43, 210, 221, 223, 226) avec un axe longitudinal (207),
un élément de réglage de dose (31, 290) qui peut être actionné par un utilisateur et être mis en rotation autour de l'axe longitudinal (207) pour régler une dose devant être distribuée par le dispositif de distribution de médicament (10, 200, 220, 222, 225),
une tige de piston (42, 240) qui est configurée pour être avancée axialement dans une direction proximale pour distribuer la dose réglée, et
un élément de dosage (330) destiné à définir l'avancement axial de la tige de piston (42, 240) lors de la distribution de la dose réglée,
dans lequel l'élément de dosage (330) peut être déplacé axialement le long de l'axe longitudinal (207) et déplacé en rotation autour de l'axe longitudinal (207) pendant le réglage de dose,
dans lequel l'élément de dosage (330) est fixé en rotation à l'élément de réglage de dose (31, 290) pendant le réglage de dose,
dans lequel l'élément de dosage (330) comprend un arrêt de dose maximum (337),
dans lequel l'arrêt de dose maximum (337) est configuré pour s'engager avec un dispositif d'arrêt maximum (190) pour limiter un déplacement de l'élément de dosage (330) par rapport au boîtier (3, 43, 210, 221, 223, 226) quand une dose maximum a été réglée,
dans lequel le dispositif d'arrêt maximum (190) est prévu au niveau du boîtier (3, 43, 210, 221, 223, 226),
**caractérisé en ce que**
le dispositif de distribution de médicament (10, 200, 220, 222, 225) comprend un mécanisme de définition de dose (232) destiné à définir des positions de dose rotatives de l'élément de réglage de dose (31, 290) par rapport au boîtier (3, 43, 210, 221, 223, 226),
dans lequel l'élément de réglage de dose (31, 290) est connecté au boîtier (3, 43, 210, 221, 223, 226) via un élément de sélection de dose (35, 310),
dans lequel l'élément de sélection de dose (35, 310) est fixé en rotation et peut être déplacé axialement par rapport au boîtier (3, 43, 210, 221, 223, 226),
dans lequel le mécanisme de définition de dose (232) agit entre l'élément de sélection de dose (35, 310) et l'élément de réglage de dose (31, 290) ou dans lequel le mécanisme de définition de dose (232) agit entre l'élément de sélection de dose (35, 310) et l'élément de dosage (330).

2. Dispositif de distribution de médicament (10, 200, 220, 222, 225) selon la revendication 1,
dans lequel l'élément de dosage (330) peut être déplacé en rotation par rapport à l'élément de réglage de dose (31, 290) pendant la distribution de dose, et/ou
dans lequel l'élément de dosage (330) est connecté par vissage au boîtier (3, 43, 210, 221, 223, 226), par exemple via un filetage extérieur (335) prévu sur l'élément de dosage (330) et un filetage intérieur correspondant (185) prévu sur le boîtier (3, 43, 210, 221, 223, 226).

3. Dispositif de distribution de médicament (10, 200, 220, 222, 225) selon l'une des revendications précédentes,
dans lequel l'arrêt de dose maximum (337) comprend une surface d'arrêt (338) qui est configurée pour venir axialement en butée avec le dispositif d'arrêt maximum (190) du boîtier (3, 43, 210, 221, 223, 226) lors du réglage de la dose maximum,
dans lequel, par exemple, la surface d'arrêt (338) est orientée perpendiculairement à l'axe longitudinal (207), et/ou la surface d'arrêt (338) est une surface annulaire entourant l'axe longitudinal (207).

4. Dispositif de distribution de médicament (10, 200, 220, 222, 225) selon l'une des revendications précédentes,
dans lequel l'arrêt de dose maximum (337) se projette depuis une surface extérieure de l'élément de dosage (31, 290), et/ou
dans lequel le dispositif d'arrêt maximum (190) se projette depuis une surface intérieure du boîtier (3, 43, 210, 221, 223, 226), et/ou
dans lequel l'arrêt de dose maximum (337) est espacé d'une extrémité distale de l'élément de dosage (330), et/ou
dans lequel le dispositif d'arrêt maximum (190) du boîtier (3, 43, 210, 221, 223, 226) a une surface de limitation (192) qui est orientée perpendiculairement à l'axe longitudinal (207) et l'arrêt de dose maximum (337) s'engage avec la surface de limitation (192) lors du réglage de la dose maximum.

5. Dispositif de distribution de médicament (10, 200, 220, 222, 225) selon l'une des revendications précédentes,
dans lequel le dispositif d'arrêt maximum (190) du boîtier (3, 43, 210, 221, 223, 226) est prévu sur un élément flexible (191) qui est configuré pour s'encliqueter sur l'arrêt de dose maximum (337) de l'élément de dosage (330) lors de l'assemblage du dispositif de distribution de médicament (10, 200, 220, 222, 225),
dans lequel, par exemple, l'élément flexible (191) repose contre un élément de renfort, de telle sorte qu'un boîtier extérieur (211) qui entoure un boîtier intérieur (1806) a l'élément flexible, après assemblage du dispositif de distribution de médicament (10, 200, 220, 222, 225) pour empêcher un désengagement du dispositif d'arrêt maximum (190) de l'arrêt de dose maximum (337).

6. Dispositif de distribution de médicament (10, 200, 220, 222, 225) selon l'une des revendications précédentes,
dans lequel l'élément de dosage (330) comprend un arrêt de dose à zéro (340),
dans lequel l'arrêt de dose à zéro (340) est configuré pour s'engager avec un dispositif d'arrêt à zéro (196) pour limiter un déplacement de l'élément de dosage (330) par rapport au boîtier (3, 43, 210, 221, 223, 226) lorsque l'élément de dosage (330) atteint une position de dose à zéro,
dans lequel le dispositif d'arrêt à zéro (196) est prévu au niveau du boîtier (3, 43, 210, 221, 223, 226),
dans lequel, par exemple, l'arrêt de dose à zéro (340) s'engage avec le dispositif d'arrêt à zéro (196) dans un plan de contact qui est en angle par rapport à un plan radial perpendiculaire à l'axe longitudinal (207) et, par exemple, le plan de contact est orienté perpendiculairement au plan radial.

7. Dispositif de distribution de médicament (10, 200, 220, 222, 225) selon la revendication 6,
dans lequel l'arrêt de dose à zéro (340) est prévu au niveau d'une extrémité proximale de l'élément de dosage (330) et/ou
dans lequel le dispositif d'arrêt à zéro (196) est prévu au niveau d'une extrémité proximale d'une cavité de boîtier (189) du boîtier (3, 43, 210, 221, 223, 226), et/ou
dans lequel le dispositif d'arrêt à zéro (196) et le dispositif d'arrêt maximum (190) sont prévus au niveau du même élément structurel du boîtier (3, 43, 210, 221, 223, 226), par exemple au niveau d'un boîtier intérieur (180) et, par exemple, l'élément structurel comprend un filetage de dose (185) qui s'engage par vissage avec l'élément de dosage (330).

8. Dispositif de distribution de médicament (10, 200, 220, 222, 225) selon l'une des revendications précédentes,
dans lequel l'élément de sélection de dose (35, 310) est fixé axialement par rapport à l'élément de dosage (330).

9. Dispositif de distribution de médicament (10, 200, 220, 222, 225) selon l'une des revendications précédentes,
dans lequel l'élément de sélection de dose (35, 310) est connecté au boîtier (3, 43, 210, 221, 223, 226) via une connexion (187, 188, 315, 316) qui permet à l'élément de sélection de dose (35, 310) d'être monté sur le boîtier (3, 43, 210, 221, 223, 226) uniquement dans des orientations de rotation qui font en sorte que l'élément de réglage de dose (31, 290) est réglé à une position de dose lors de l'engagement de l'arrêt de dose maximum (337) avec le dispositif d'arrêt maximum (190),
dans lequel, par exemple, la connexion permet une seule orientation de rotation simple uniquement.

10. Dispositif de distribution de médicament (10, 200, 220, 222, 225) selon la revendication 9,
dans lequel la connexion (187, 188, 315, 316) comprend une connexion cannelée qui permet un déplacement axial et empêche un déplacement rotatif de l'élément de sélection de dose (35, 310) par rapport au boîtier (3, 43, 210, 221, 223, 226),
dans lequel la connexion cannelée comprend un ensemble de cannelures de codage, les cannelures de codage ayant des dimensions respectives qui diffèrent les unes des autres, par exemple en largeur et/ou en hauteur,
dans lequel, par exemple, la connexion cannelée comprend une cannelure de codage unique (187, 315) qui diffère des cannelures restantes (188, 316) de la connexion (187, 188, 315, 316).

11. Dispositif de distribution de médicament (10, 200, 220, 222, 225) selon l'une des revendications précédentes,
dans lequel l'élément de dosage (330) est couplé à la tige de piston (42, 240) via un mécanisme d'avancement qui translate un déplacement axial de l'élément de dosage (330) en avancement axial de la tige de piston (42, 240) pendant la distribution de dose de telle sorte que le déplacement axial de l'élément de dosage (330) pendant la distribution de dose amène la tige de piston (42, 240) à avancer axialement dans la direction proximale,
dans lequel, par exemple, le mécanisme d'avancement est configuré comme mécanisme à engrenage qui réduit le déplacement axial de l'élément de dosage (330) jusqu'à un avancement axial plus petit de la tige de piston (42, 240).

12. Dispositif de distribution de médicament (10, 200, 220, 222, 225) selon la revendication 11,
dans lequel la tige de piston (42, 240) est fixée en rotation au boîtier (3, 43, 210, 221, 223, 226),
dans lequel le mécanisme d'avancement comprend un écrou (36, 250) qui est couplé entre la tige de piston (42, 240) et l'élément de dosage (330),
dans lequel l'écrou (36, 250) est connecté par vissage à la tige de piston (42, 240),
dans lequel l'écrou (36, 250) est fixé en rotation par rapport à l'élément de dosage (330) et peut être mis en rotation par rapport au boîtier (3, 43, 210, 221, 223, 226) pendant le réglage de dose,
dans lequel l'écrou (36, 250) peut être mis en rotation par rapport à l'élément de dosage (330) et être fixé en rotation par rapport au boîtier (3, 43, 210, 221, 223, 226) pendant la distribution de dose.

13. Dispositif de distribution de médicament (10, 200, 220, 222, 225) ayant un boîtier (3, 43, 210, 221, 223, 226) avec un axe longitudinal (207),
un élément de réglage de dose (31, 290) qui peut être actionné par un utilisateur et être mis en rotation autour de l'axe longitudinal (207) pour régler une dose devant être distribuée au dispositif de distribution de médicament (10, 200, 220, 222, 225),
une tige de piston (42, 240) qui est configurée pour être avancée axialement dans une direction proximale pour distribuer la dose réglée, et
un élément de dosage (330) destiné à définir l'avancement axial de la tige de piston (42, 240) lors de la distribution de la dose réglée,
dans lequel l'élément de dosage (330) peut être déplacé axialement le long de l'axe longitudinal (207) et peut être déplacé en rotation autour de l'axe longitudinal (207) pendant le réglage de dose,
dans lequel l'élément de dosage (330) est fixé en rotation à l'élément de réglage de dose (31, 290) pendant le réglage de dose,
dans lequel l'élément de dosage (330) comprend un arrêt de dose à zéro (340),
dans lequel l'arrêt de dose à zéro (340) est configuré pour s'engager avec un dispositif d'arrêt à zéro (196) pour limiter un déplacement de l'élément de dosage (330) par rapport au boîtier (3, 43, 210, 221, 223, 226) lors du réglage d'une dose à zéro,
dans lequel le dispositif d'arrêt à zéro (196) est prévu au niveau du boîtier (3, 43, 210, 221, 223, 226),
**caractérisé en ce que**
le dispositif de distribution de médicament (10, 200, 220, 222, 225) comprend un mécanisme de définition de dose (232) destiné à définir des positions de dose rotatives de l'élément de réglage de dose (31, 290) par rapport au boîtier (3, 43, 210, 221, 223, 226),
dans lequel l'élément de réglage de dose (31, 290) est connecté au boîtier (3, 43, 210, 221, 223, 226) via un élément de sélection de dose (35, 310), dans lequel l'élément de sélection de dose (35, 310) est fixé en rotation et peut être déplacé axialement par rapport au boîtier (3, 43, 210, 221, 223, 226),
dans lequel le mécanisme de définition de dose (232) agit entre l'élément de sélection de dose (35, 310) et l'élément de réglage de dose (31, 290) ou dans lequel l'élément de définition de dose (232) agit entre l'élément de sélection de dose (35, 310) et l'élément de dosage (330).

14. Dispositif de distribution de médicament (10, 200, 220, 222, 225) selon la revendication 13,
dans lequel l'élément de dosage (330) peut être déplacé en rotation par rapport à l'élément de réglage de dose (31, 290) pendant la distribution de dose, et/ou
dans lequel l'arrêt de dose à zéro (340) s'engage avec le dispositif d'arrêt à zéro (196) dans un plan de contact qui est en angle par rapport à un plan radial perpendiculaire à l'axe longitudinal (207) et, par exemple, le plan de contact est orienté perpendiculairement au plan radial, et/ou
dans lequel l'arrêt de dose à zéro (340) de l'élément de dosage (330) comprend une surface d'arrêt qui est configurée pour venir en butée contre une surface d'arrêt correspondante du boîtier (3, 43, 210, 221, 223, 226), et/ou
dans lequel l'arrêt de dose à zéro (340) est prévu au niveau d'une extrémité proximale de l'élément de dosage (330) et/ou
dans lequel le dispositif d'arrêt à zéro (196) est prévu au niveau d'une extrémité proximale et d'une cavité de boîtier (189) du boîtier (3, 43, 210, 221, 223, 226).

15. Dispositif de distribution de médicament (10, 200, 220, 222, 225) selon l'une des revendications précédentes,
dans lequel le mécanisme de définition de dose (232) agit entre l'élément de sélection de dose (35, 310) et l'élément de réglage de dose (31, 290) via des éléments additionnels qui sont situés entre l'élément de sélection de dose (35, 310) et l'élément de réglage de dose (31, 290), comme via l'élément de dosage (330).

16. Dispositif de distribution de médicament (10, 200, 220, 222, 225) selon l'une des revendications 1 à 14,
dans lequel le mécanisme de définition de dose (232) est réalisé par engagement direct de l'élément de réglage de dose (31, 290) ou d'une partie fixée en permanence à l'élément de réglage de dose (31, 290)avec l'élément de sélection de dose (35, 310) ou une partie fixée en permanence à l'élément de sélection de dose (35, 310).
